(19) Europäisches Patentamt
European Patent Office
Office européen des brevets

(11) EP 4 495 121 A1

(12) **EUROPEAN PATENT APPLICATION**
published in accordance with Art. 153(4) EPC

(43) Date of publication:
**22.01.2025 Bulletin 2025/04**

(21) Application number: **23792205.9**

(22) Date of filing: **20.04.2023**

(51) International Patent Classification (IPC):
***C07D 491/22*** *(2006.01)* ***A61K 31/4745*** *(2006.01)*
***A61P 35/00*** *(2006.01)* ***A61K 47/65*** *(2017.01)*
***A61K 47/68*** *(2017.01)* ***G01N 33/574*** *(2006.01)*

(52) Cooperative Patent Classification (CPC):
**A61K 31/4745; A61K 47/65; A61K 47/68; A61P 35/00; C07D 491/22; G01N 33/574**

(86) International application number:
**PCT/KR2023/005380**

(87) International publication number:
**WO 2023/204631 (26.10.2023 Gazette 2023/43)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **20.04.2022 KR 20220049158**
**11.07.2022 KR 20220085227**
**18.08.2022 KR 20220103591**
**08.09.2022 KR 20220114079**
**07.12.2022 KR 20220170146**
**13.03.2023 KR 20230032226**

(71) Applicant: **Pinotbio, Inc.**
**Suwon-si, Gyeonggi-do 16506 (KR)**

(72) Inventors:
- **JUNG, Doo Young**
  **Suwon-si Gyeonggi-do 16506 (KR)**
- **LEE, Jin Soo**
  **Suwon-si Gyeonggi-do 16506 (KR)**
- **CHO, Hyun Yong**
  **Suwon-si Gyeonggi-do 16506 (KR)**
- **LEE, Byeong Sung**
  **Suwon-si Gyeonggi-do 16056 (KR)**
- **JUNG, Jin Kyo**
  **Suwon-si Gyeonggi-do 16506 (KR)**
- **SHIN, Seung Gun**
  **Suwon-si Gyeonggi-do 16506 (KR)**
- **KWEON, So Hui**
  **Suwon-si Gyeonggi-do 16505 (KR)**
- **MATHI, Gangadhar Rao**
  **Suwon-si Gyeonggi-do 16506 (KR)**

(74) Representative: **Prüfer & Partner mbB**
**Patentanwälte · Rechtsanwälte**
**Sohnckestraße 12**
**81479 München (DE)**

# (54) CAMPTOTHECIN DERIVATIVES THAT BIND TO DDX5 PROTEIN AND PRODRUGS THEREOF

(57) The present invention relates to (a) an active camptothecin derivative represented by Chemical Formula 1 below, which is designed to bind to the DDX5 protein and an E3 ligase; (b) a prodrug thereof designed to release the active camptothecin derivative (a) at an *in vivo* target site; or (c) a ligand-containing complex targeting the DDX5 protein, in which an topoisomerase-1 inhibitory ability of the active camptothecin derivative (a) or FL118 compound of Chemical Formula 2 is inactivated through linker conjugation.

The active camptothecin derivative designed according to the present invention may bind to the DDX5 protein in cells to induce cell death through DDX5 protein degradation.

EP 4 495 121 A1

[FIG. 1]

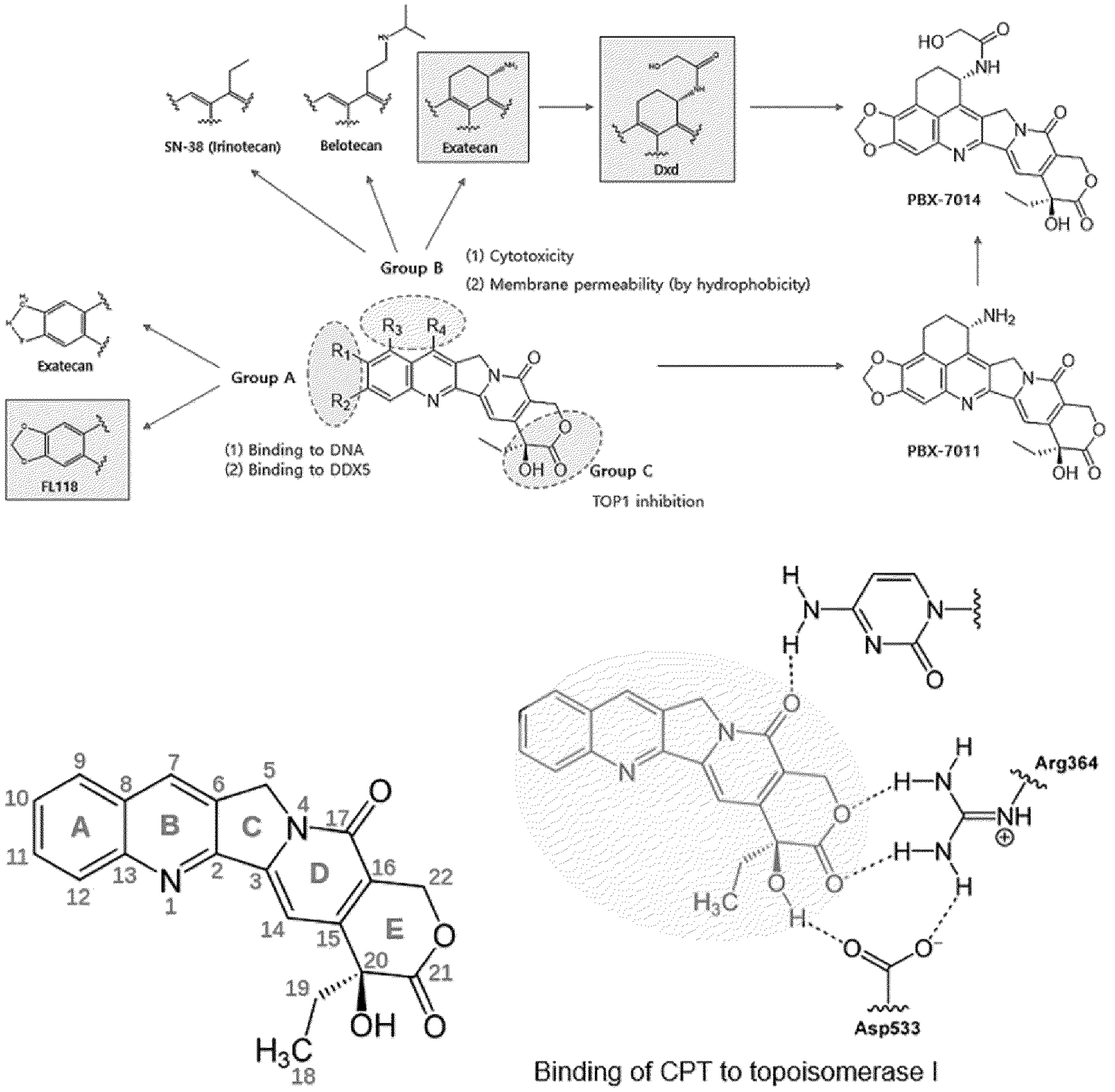
SN-38 (Irinotecan)
Belotecan
Exatecan
Dxd
PBX-7014
Group B
(1) Cytotoxicity
(2) Membrane permeability (by hydrophobicity)
Exatecan
Group A
FL118
(1) Binding to DNA
(2) Binding to DDX5
Group C
TOP1 inhibition
PBX-7011
Arg364
Asp533
Binding of CPT to topoisomerase I

## Description

[Technical Field]

The present invention relates to (a) an active camptothecin derivative represented by Chemical Formula 1 below, which is designed to bind to the DDX5 protein and an E3 ligase; (b) a prodrug thereof designed to release the active camptothecin derivative (a) at an *in vivo* target site; or (c) a ligand-containing complex targeting the DDX5 protein, in which an topoisomerase-1 inhibitory ability of the active camptothecin derivative (a) or the FL118 compound of Chemical Formula 2 is inactivated through linker conjugation.

The active camptothecin derivative designed according to the present invention can bind to the DDX5 protein in cells to induce cell death through the degradation of the DDX5 protein.

[Background Art]

Cancer genes are genes that originally exist to regulate the normal functions of cells, but can cause cancer when the genes are mutated or abnormally regulated. One of the normal cell functions is a cell death function, and in this regard, there are pro-apoptotic factors and apoptotic factors.

For example, in cancer genes, there are (i) oncogenes that actively promote cell division when mutated and (ii) tumor suppressor genes that do not suppress cell division when mutated. Examples of oncogenes include Src, EGFR, HER2, RAS, APC, and BCL-2, and examples of tumor suppressor genes include p53, BRCA, Rb, PTEN, and BAX.

Cancer is a group of diseases in which the cell cycle of growth and division is unregulated. As shown in FIG. 29, a damaged programmed cell death (e.g., apoptosis) mechanism may lead to cancer occurrence.

Cancer is caused by mutations in genes whose protein products are involved in cell cycle regulation. Many types of cancer are associated with the overexpression of a specific gene or the abnormal activity of a mutant protein product (oncogenes).

Proteins encoded by viral oncogenes are similar to cellular proteins with important regulatory functions. These cellular homologs are called proto-oncogenes or normal cellular oncogenes. Mutations in proto-oncogenes actively promote cell proliferation. Mutant c-H-ras proteins have mutations that impair the ability to hydrolyze GTP. This maintains the mutant protein in an active signal mode and stimulates cell division. The mutant versions of *c-ras* have been found in many types of tumors.

Single mutations do not usually cause cancer. Several genes that normally regulate cell growth are mutated before cancer occurs.

HER2-positive breast cancer, in which HER2 is abnormally expressed in breast epithelial cells, accounts for approximately ¼ of all breast cancer patients, and has a higher recurrence rate and worse prognosis when treated with chemotherapy, compared to female hormone-related breast cancer. Herceptin (generic name: trastuzumab), which is a HER2-targeting drug, treats breast cancer by reducing the number of HER2 receptors on a cell surface and helping immune cells kill HER2-positive breast cancer cells.

HER2-positive breast cancer is breast cancer having the amplification or overexpression of the HER2 (ErbB2) oncogene, which is a member of the ErbB receptors. While trastuzumab (Herceptin) has increased the therapeutic effect on HER2-positive breast cancer, HER2-positive breast cancer shows an aggressive pattern compared to other types of breast cancer, and more than half of patients are resistant to existing targeted therapies or develop tolerance during treatment.

Ligands of the ErbB receptor attached to the cell membrane are NRG1 and HB-EGF, and the activated ErbB ligands bind to their receptors (EGFR and HER3) to catalyze phosphorylation (P) by bonds between receptors (EGFR-HER2 and HER2-HER3), inducing intracellular growth and proliferation signals. The overactivation of ErbB receptors leads to resistance to anti-HER2 treatment.

Meanwhile, dysregulated transcription factors may lower the sensitivity of cancer cells to chemotherapy or radiotherapy. In cancer treatment, drug resistance may be intrinsic or acquired resistance. In cancer treatment, intrinsic drug resistance may be caused by an abnormally expressed transcription factor, which is a critical regulator for cell proliferation and cell death. The abnormal overexpression of NF-κB, STAT3, HIF-1, AR or ER and the inactivation of p53 and FOXO3a in tumor tissue protect cancer cells from death by anticancer agents, exhibiting intrinsic drug resistance.

However, chemotherapy or radiotherapy also induces acquired drug resistance by the activation of dysregulated transcription factors. It has been known that ionizing radiation can stimulate the activation and induction of several different transcription factors, including STAT3, NF-κB, and HIF-1. The upregulation of NF-κB and STAT3 induces the transcription of anti-apoptotic genes in cancer cells and promotes the cell cycle entry and proliferation of cancer cells, resulting in radiation resistance. In addition, the radiation-mediated upregulation of transcription factors NF-κB and HIF-1 may induce genes responsible for the maintenance of EMT cells and cancer stem cells, which are resistant to cancer treatment. In addition, the activation of transcription factor STAT3 promotes drug resistance to cancer treatment. The feedback

activation of STAT3 by chemotherapy or radiotherapy has been shown to be a molecular mechanism of cancer treatment resistance. Therefore, co-targeting of the dysregulated transcription factors and their main targets may become a promising approach to overcoming drug resistance in cancer treatment.

The signal transducer and activator of transcription (STAT) transcription factor family includes STAT1, STAT2, STAT3, STAT4, STATS, and STAT6. STAT proteins may be activated by JAK kinases upon stimulation with a growth factor, a cytokine, an interferon, or an oncogene to regulate interferon signaling. The DNA-binding domain of STAT is an immunoglobulin-like structure that mediates binding to a specific DNA target sequence. The phosphorylation of STAT3 by JAK kinase during activation activates dimerization. The dimer then moves to a nuclear compartment and binds to the sequence of a target promoter, inducing the expression of a target gene. STAT3 target genes include survivin, bcl-xl, mcl-1, cyclin D1, MMP2, MMP9, VEGF, Myc, and Sox2. STAT proteins regulate many biological processes, including cell growth, cell death, differentiation, and immunity. Within the STAT family, STAT3 and STATS were involved in cancer development.

Unregulated STAT3 and activated STATS are considered oncogenes, increasing angiogenesis and improving cancer cell survival. Studies using many samples of the cBioPortal database showed that STAT3 was more frequently overexpressed in lung, ovarian, gastric, hematopoietic, and brain cancers compared to normal tissue, and the overexpression of STAT3 was associated with a low overall survival rate. STAT3 phosphorylation by JAK kinase is the first step in STAT3 activation. Another study involving 90 patients with glioblastoma found that a high p-STAT3 level is significantly associated with decreases in progression-free survival and overall survival. Multivariate survival analysis showed that a high p-STAT3 level can serve as an important prognostic indicator for poor progression-free survival and overall survival.

Inhibitors (irinotecan, topotecan, etc.) of type 1 topoisomerase (topoisomerase-1) are anticancer mechanisms whose efficacy/safety has been clinically proven, and have been clinically proven to have excellent anticancer efficacy in various types of refractory solid cancer, such as colorectal cancer, lung cancer, breast cancer, and ovarian cancer.

In this regard, a camptothecin derivative, which is a small molecule compound expressing antitumor activity by inhibiting topoisomerase-1, is known.

Camptothecin (CPT) has low solubility in water, and to prepare clinical trials, the National Cancer Institute (NCI) prepared a water-soluble sodium salt (NSC100880). Phase I and II clinical trials were not completed due to high toxicity exhibited by the compound [hemorrhagic cystitis, gastrointestinal toxicity (e.g., nausea, vomiting, and diarrhea), and bone marrow suppression (particularly, leukopenia and thrombocytopenia)].

Subsequently, a number of CPT analogs were synthesized to obtain compounds with lower toxicity and higher water solubility. There are two drugs, irinotecan (CPT-11) and topotecan.

Irinotecan (CPT-11), jointly developed by Japan's Dallchi and Yakult, was the world's first camptothecin-based anticancer agent in 1994, and was released in Europe and Japan after it was proven effective against lung cancer (small cell and non-small cell lung cancer). In 1995, the efficacy of irinotecan (CPT-11) on colorectal cancer and breast cancer was additionally proven. In addition, the efficacy of topotecan, developed by Glaxo Smith Kline, against metastatic ovarian cancer was approved by the U.S. FDA in April 1995 and released. CKD-602 (belotecan), which is a new camptothecin derivative recently developed in Korea, has a potent topoisomerase-1 inhibitor effect and is water-soluble, resulting in successfully overcoming the toxicity caused by existing poor solubility.

All camptothecin derivatives identified so far contain a parent structure with five rings essential for cytotoxicity (FIG. 1). In terms of molecular structure, the E-ring and A- and B-ring regions were confirmed as important regions. Camptothecin includes alpha-hydroxy lactone structures or 6-membered lactone structures in the E-ring essential for cytotoxicity. Among them, a lactone group and an alpha-hydroxy group, located at position C-20 of the E-ring, are important for the stability of a topoisomerase-1-DNA by-product, and it was proven that modifications of the A- and B-rings can increase water solubility and activity. It was proven that modifications of the first ring increase water solubility and allow greater tolerability, for example, in the case of the above-described drugs.

To increase water solubility and an anticancer effect, CKD-602 also had a substitution at position C-7 of the B-ring region. Lee et al. reported that CKD-602 has superior anticancer effects in a wide range of cancer cell lines, compared to camptothecin and topotecan. In addition, it was confirmed that CKD-602 is a relatively safe drug with a maximum tolerated dose (MTD) of 25 mg/kg in a L1210 leukemia nude mouse model. The generally known side effects of a camptothecin-based drug can be largely classified into hematological side effects and non-hematological side effects. Hematological side effects include neutropenia accompanied by fever, sepsis, and bleeding, whereas non-hematological side effects include side effects such as nausea, vomiting and hair loss, and toxicity to the gastrointestinal tract, kidney, and nervous system. In animal studies of CKD-602, conducted by Kim et al., among the side effects of such a camptothecin-based drug, no adverse drug reactions, except an increase in gastric juice secretion, was observed even by administration at a dose more than 10 times higher than a clinically applicable dose. In addition, recent domestic clinical studies have also proven its relative safety, with only reversible and controllable side effects of neutropenia and leukopenia reported rather than serious systemic toxicity. However, to date, it has been used in a limited manner with the main indication being patients who have failed standard chemotherapy or cannot undergo standard chemotherapy, that is, treatment of refractory or recurrent ovarian cancer and colon cancer, treatment of refractory or recurrent limited disease (LD) small cell lung cancer that had failed first-line chemotherapy, or treatment of extensive disease (ED) small cell lung cancer, which has relapsed or

worsened after standard treatment and is judged as unlikely to respond to further chemotherapy or surgery.

SN-38 is a potent topoisomerase-1 inhibitor with an $IC_{50}$ value in the nanomolar range in several cell lines. It is the active form of irinotecan, which is a prodrug used in the treatment of colorectal cancer, and is also active in lung cancer, breast cancer, and brain cancer. Trop-2-SN-38 ADC is currently being successfully developed for a number of cancers, including TNBC, bladder cancer, and gastric cancer, but tolerance issues caused by the overexpression of a drug efflux transporter, epigenetic silencing of topoisomerase-1, and an increase in anti-apoptotic proteins still remain.

Meanwhile, Daiichi Sankyo used DXD (exatecan), which is a cytotoxic drug that is approximately 10 times more active in cancer cells than SN-38, in the development of Enhertu®. DXD has high solubility, is relatively safe, and has a high killing effect on surrounding cells, making it advantageous for the treatment of heterogeneous tumors. However, the half-life, which can reduce off-target effects, is short. DXD is bioconjugated to a cysteine residue of an anti-HER2 antibody with a maleimide linker and has a homogeneous DAR value of 8. Despite a high DAR value, DXD is highly stable, with only 2.1% released into the plasma over 21 days (Ogitani et al., 2016). Enhertu® was approved by the US FDA in 2019, and applied to adult patients with unresectable metastatic Her2-positive breast cancer, who have received HER2-targeted therapy at least twice in the past.

Camptothecin-based derivatives, such as SN-38 and Dxd (the derivative of topoisomerase-1, exatecan, in the clinical stage), have recently been successfully developed as novel payloads for an antibody-drug conjugate (ADC). As a result, two ADCs using a camptothecin-based payload (Trodelvy with an SN-38 payload (sacituzumab govitecan) and Enhertu with a Dxd payload (trastuzumab deruxtecan)) were recently approved by the FDA. These camptothecin-based payloads have moderate cytotoxicity compared to conventional ADCs with ultra-toxic payloads (DAR = 2 or 4), such as MMAE and MMAF, but a higher drug-to-antibody ratio (DAR) of 8 can be used due to their excellent safety profiles. In addition, such camptothecin-based payloads may allow a linker system with a faster drug release profile such as a CL-2A or GGFG linker system to be used. The combination of these components has led to the development of novel ADCs with a broader therapeutic window.

In short, since camptothecin-based compounds were confirmed to act as topoisomerase-1 inhibitors and have the potential to be used anticancer agents, various compounds with similar structures were synthesized and studied. Among them, as topotecan and irinotecan were approved by the FDA in 1996 and, 2002, respectively, various compounds were synthesized but did not exhibit outstanding effects, thereby losing theirdevelopment momentum. However, an ADC (Enhertu) using the novel camptothecin derivative, Dxd as a payload, and an ADC (Trodelvy) using SN-38, which is an *in vivo* active metabolite of irinotecan, as a payload, were approved by the FDA in 2019 and 2020, respectively, revitalizing research.

Antibody-drug conjugates (ADCs) using camptothecin derivatives as payloads, such as Trodelvy and Enhertu, have been successful in delivering camptothecin compounds that exhibit strong anticancer effects selectively to cancer cells, thereby achieving excellent anticancer effects without severe systemic side effects, but they still have several shortcomings. The most representative shortcoming may be resistance by overexpression of an ABCG2 drug efflux pump. Many camptothecin derivatives are rapidly excreted from cells by ABCG2, which results in severely lowering therapeutic efficacy in ABCG2-overexpressing cancer cells, compared to cancer cells that do not express ABCG2 or less express it at a normal level. Such ABCG2 overexpression becomes the main cause of reducing therapeutic efficacy even in ADCs such as Trodelvy and Enhertu, which use small molecule camptothecin compounds, e.g., irinotecan and topotecan, as well as camptothecin compounds as payloads.

Meanwhile, it was also reported that, in contrast to DM1 from Kadcyla, Enhertu® DXd was highly membrane permeable, resulting in the delivery of a payload released in cells to cells that do not express HER2 adjacent to a target cell. This suggests that Enhertu® may have a potential bystander effect due to the nature of the payload and provide clinical benefits to patients refractory to Herceptin or Kadcyla.

[Disclosure]

[Technical Problem]

The present invention is directed to providing a camptothecin derivative designed to bind to the DDX5 protein and an E3 ligase as a drug candidate that kills target cells.

The present inventors synthesized FL118 compound (FIG. 2), which is a camptothecin-based drug that has a dual mechanism of action (MoA) that degrades the oncoprotein DDX5 (p68) along with an excellent ability to inhibit topoisomerase-1, and continued to conduct various studies on its physicochemical properties. However, the FL118 compound had limitations in formulation development due to limitations in physicochemical properties.

Therefore, based on the structure of FL118 that is differentiated from the SN38 drug, the present inventors attempted to design and synthesize a camptothecin derivative with a novel structure, which exhibits a dual MoA in terms of topoisomerase-1 inhibition and DDX5 degradation, which are the advantages of FL118.

Accordingly, the present invention is directed to newly designing and providing an active camptothecin derivative

having a dual MoA that inhibits topoisomerase-1 and degrades the oncoprotein DDX5 and/or its prodrug, which is an antibody-drug conjugate (ADC) that is designed to release the active camptothecin derivative *in vivo.*

In addition, the present invention is directed to providing an anticancer mechanism as a new modality in which an active camptothecin derivative kills cancer cells and/or surrounding cells prior to intrinsic or acquired drug tolerance by inducing cell death through the degradation of the DDX5 protein by binding to the DDX5 protein in cells, as well as a bystander effect.

[Technical Solution]

A first aspect of the present invention provides (a) an active camptothecin derivative represented by Chemical Formula 1 below, which is designed to bind to the DDX5 protein and an E3 ligase; (b) a prodrug thereof designed to release the active camptothecin derivative (a) at an *in vivo* target site; or (c) a ligand-containing complex targeting the DDX5 protein, in which an topoisomerase-1 inhibitory ability of the active camptothecin derivative (a) or the FL118 compound of Chemical Formula 2 is inactivated through linker conjugation.

[Chemical Formula 1]

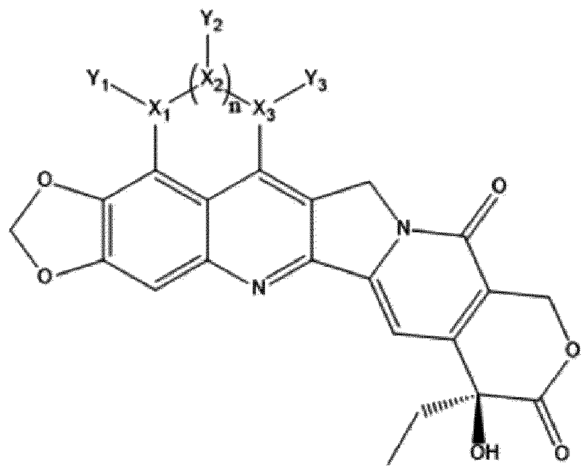

$X_1$ and $X_3$ may each be independently carbon, oxygen, nitrogen, or sulfur, and may be the same or different,
$X_2$ may be carbon, oxygen, nitrogen, sulfur, a single bond, or a double bond,
$X_1$, $(X_2)_n$ and $X_3$ may form a 6- or 7-membered ring (n= 1~2), and
$Y_1$, $Y_2$ and $Y_3$ may each be independently hydrogen, or a functional group containing oxygen, nitrogen, phosphorus or sulfur.

In the ligand-containing complex targeting the DDX5 protein, the ligand targeting the DDX5 protein may be a ligand binding to the DDX5 protein.

The active camptothecin derivative may be designed to exhibit a bystander effect in cancer tissue or control the degree of the effect by adjusting cell membrane permeability as desired through $X_1$, $(X_2)_n$, $X_3$, $Y_1$, $Y_2$ and/or $Y_3$ modifications in Chemical Formula 1.

Preferably, the active camptothecin derivative (a) designed to bind to the DDX5 protein and an E3 ligase may act as a ligand (binder) that binds to the E3 ligase as a molecular glue; may kill target cells expressing the DDX5 protein through a molecular glue mechanism of action (MoA); and/or may have an MoA that degrades the oncoprotein DDX5 as well as the ability to inhibit topoisomerase-1.

A second aspect of the present invention provides a pharmaceutical composition for preventing or treating cancer or cancer diagnosis composition, which includes (a) the active camptothecin derivative of the first aspect, (b) a prodrug thereof, or (c) a ligand-containing complex targeting the DDX5 protein.

For example, the above composition may be administered to treat HER2 positive cancer, breast cancer, lung cancer, or colorectal cancer.

Preferably, the pharmaceutical composition for preventing or treating cancer may be used as a first-line treatment after cancer diagnosis or administered to a subject (over)expressing DDX5 in cancer tissue.

A third aspect of the present invention provides a method of preparing (a) an active camptothecin derivative designed to bind to the DDX5 protein and an E3 ligase, (b) a prodrug thereof, which is designed to release the active

camptothecin derivative (a) at a target site *in vivo,* or (c) a ligand-containing complex targeting the DDX5 protein, in which an topoisomerase-1 inhibitory ability of the active camptothecin derivative (a) or the FL118 compound of Chemical Formula 2 is inactivated through linker conjugation. Here, the method includes designing the active camptothecin derivative (a) to include the camptothecin-based backbone represented by Chemical Formula 1 as a parent nucleus, selecting a compound designed in this way, or synthesizing a compound designed in this way.

For example, the step of designing the active camptothecin derivative (a) to include a camptothecin-based backbone represented by Chemical Formula 1 as a parent nucleus, or selecting the compound designed in this way may include

(1) selecting an active camptothecin derivative designed to have an MoA that inhibits topoisomerase-1 and/or an MoA that degrades the oncoprotein DDX5 from the library of compounds including a camptothecin-based backbone represented by Chemical Formula 1 as a parent nucleus, and/or
(2) confirming whether the compounds including the camptothecin-based backbone represented by Chemical Formula 1 as a parent nucleus inhibit topoisomerase-1 and/or degrade the oncoprotein DDX5 through an *in vitro* and/or *in vivo* experiment(s).

A fourth aspect of the present invention provides a method of preparing (a) an active camptothecin derivative, which binds to DDX5 acting as an oncoprotein in cells to induce cell death through DDX5 protein degradation, (b) a prodrug thereof, which is designed to release the active camptothecin derivative (a) *in vivo,* or (c) a ligand-containing complex targeting the DDX5 protein, in which topoisomerase-1 inhibitory ability of the active camptothecin derivative (a) is inactivated through linker conjugation. Here, the method includes a first step of selecting the active camptothecin derivative (a) from the group consisting of a compound of Chemical Formula 3, a compound of Chemical Formula 4, a compound of Chemical Formula 5, a compound of Chemical Formula 6, a compound of Chemical Formula 7, a compound of Chemical Formula 8, a compound of Chemical Formula 9, and isomers thereof, which are shown below; and a second step of optionally selecting a prodrug (b) thereof, which is designed to release the active camptothecin derivative (a) selected in Step 1 *in vivo.*

[Chemical Formula 3]

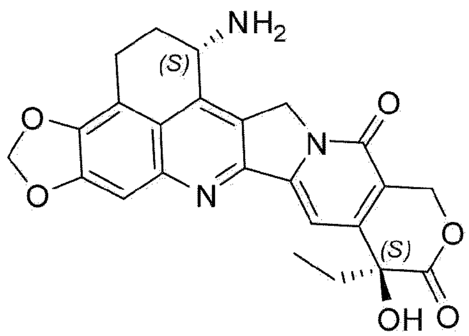

[Chemical Formula 4]

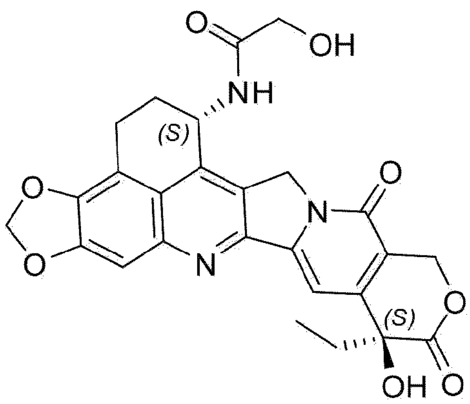

[Chemical Formula 5]

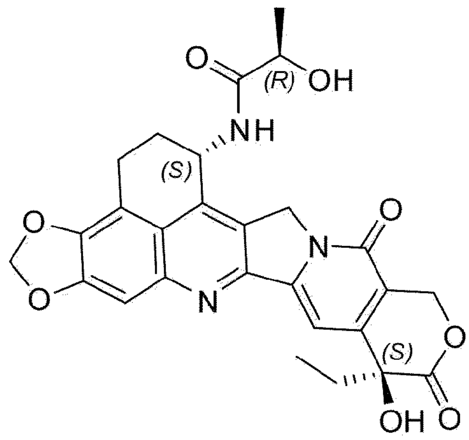

[Chemical Formula 6]

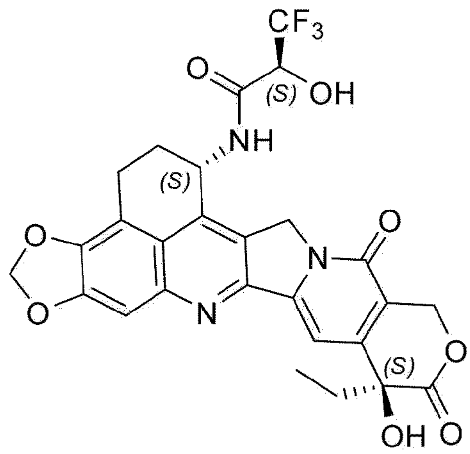

[Chemical Formula 7]

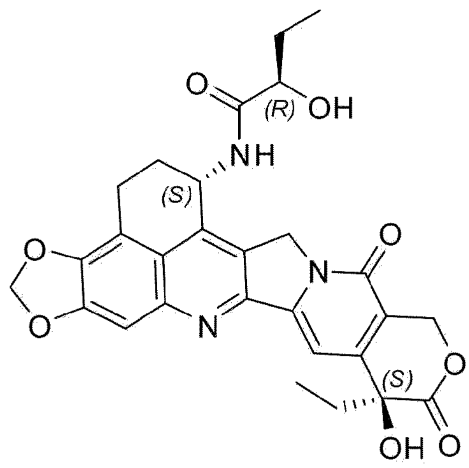

[Chemical Formula 8]

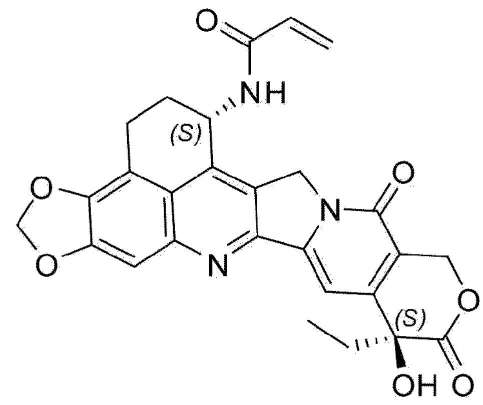

[Chemical Formula 9]

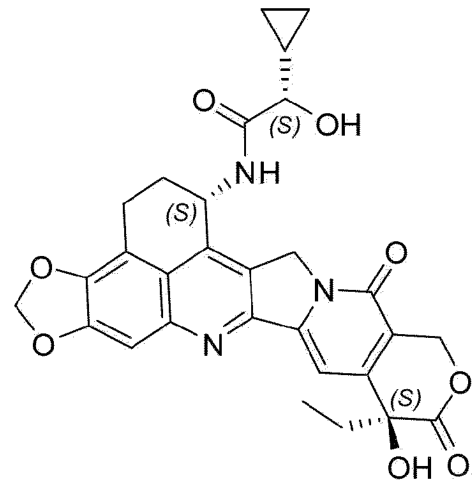

The preparation method of the third or fourth aspect may further include confirming a patient group to be administered the active camptothecin derivative (a) or a prodrug thereof (b) by quantitative or qualitative analysis of the DDX5 protein in cells using the ligand-containing complex (c) targeting the DDX5 protein, in which the topoisomerase-1 inhibitory ability of the FL118 compound of Chemical Formula 2 or the active camptothecin derivative (a) is inactivated through non-cleavable linker conjugation, in tissue biopsy or liquid biopsy.

A fifth aspect of the present invention provides a compound represented by Chemical Formula 3, Chemical Formula 3-1, Chemical Formula 4, Chemical Formula 4-1, Chemical Formula 5, Chemical Formula 5-1, Chemical Formula 6, Chemical Formula 7, Chemical Formula 8, or Chemical Formula 9 below or a pharmaceutically acceptable salt thereof.

A sixth aspect of the present invention provides a pharmaceutical composition for preventing or treating cancer, which includes a compound represented by Chemical Formula 3, Chemical Formula 3-1, Chemical Formula 4, Chemical Formula 4-1, Chemical Formula 5, Chemical Formula 5-1, Chemical Formula 6, Chemical Formula 7, Chemical Formula 8, or Chemical Formula 9 below, a pharmaceutically acceptable salt thereof, a solvate thereof, or a prodrug thereof.

[Chemical Formula 3]

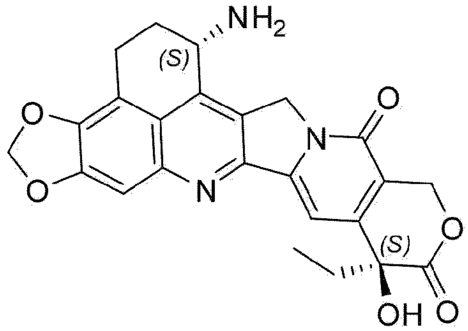

[Chemical Formula 3-1]

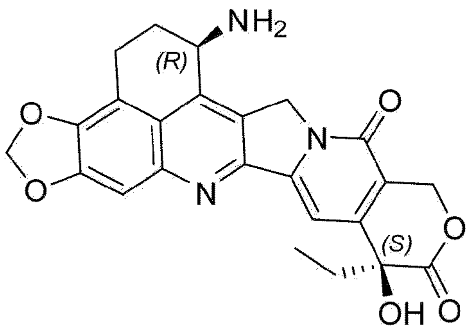

[Chemical Formula 4]

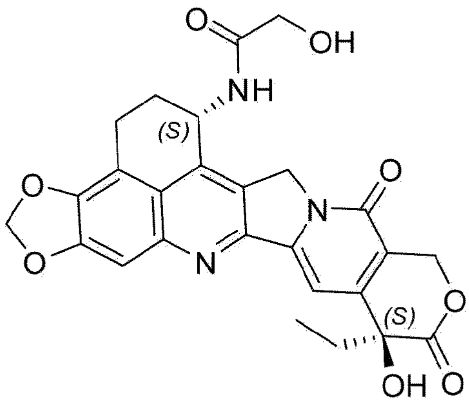

[Chemical Formula 4-1]

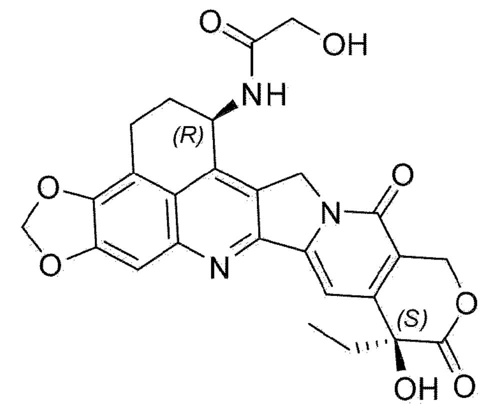

[Chemical Formula 5]

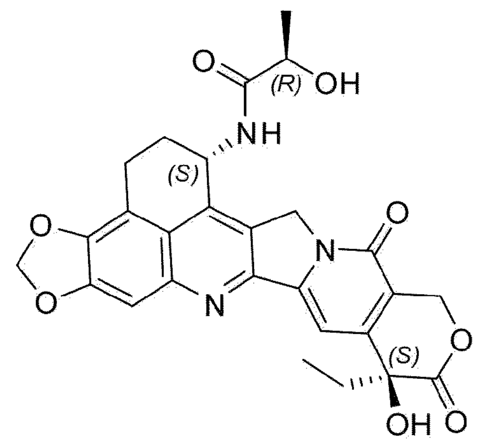

[Chemical Formula 5-1]

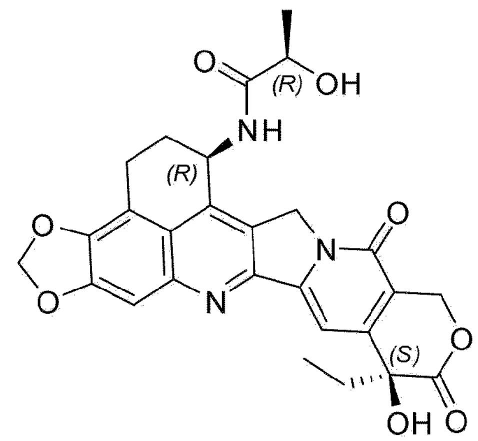

[Chemical Formula 6]

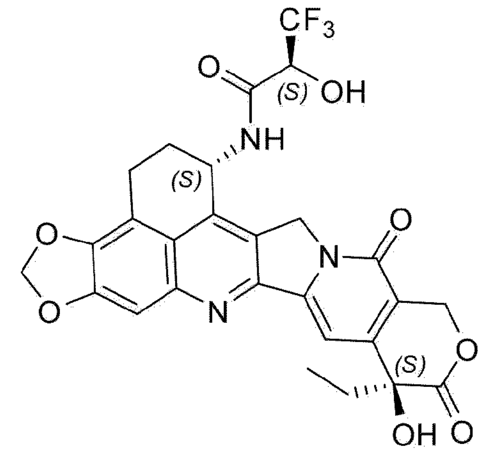

[Chemical Formula 7]

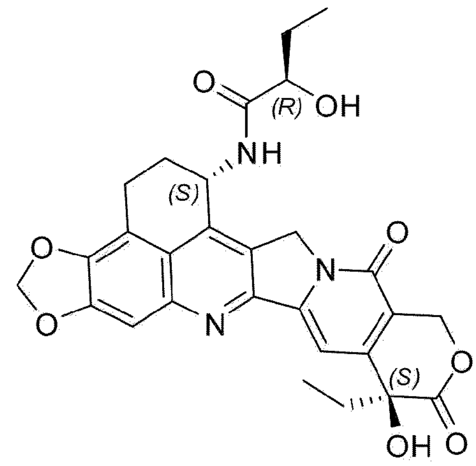

[Chemical Formula 8]

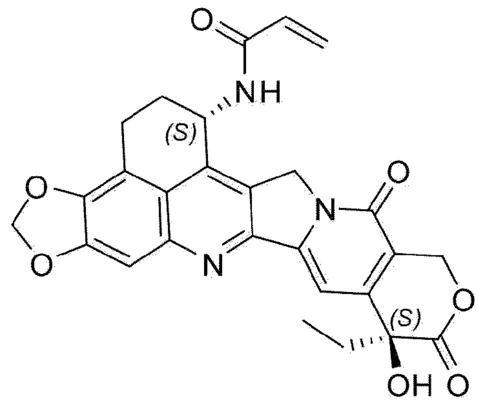

[Chemical Formula 9]

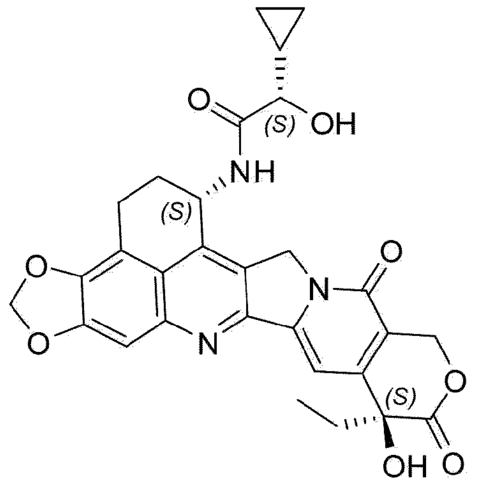

A seventh aspect of the present invention provides a pharmaceutical composition for killing cells, which includes (a) the active camptothecin derivative represented by Chemical Formula 1, which is designed to bind to the DDX5 protein and an E3 ligase; or (b) a prodrug thereof, which is designed to release the active camptothecin derivative (a) at a target site *in vivo.*

An eighth aspect of the present invention provides an anticancer formulation, which may be administered in a dose that (a) an active camptothecin derivative represented by Chemical Formula 1, which is designed to bind to the DDX5 protein and an E3 ligase; or (b) a prodrug thereof, which is designed to release the active camptothecin derivative (a) at a target site *in vivo,* stimulates antigen-presenting cells (APCs) through the cell death of cancer cells to induce an antitumor immune response.

A ninth aspect of the present invention provides a method of preparing an active camptothecin derivative (a)-based anticancer agent, in which the bystander effect and/or ADME profiles of the active camptothecin derivative (a) are/is controlled to avoid T cell exhaustion due to chronic antigen exposure. The active camptothecin derivative (a)-based anticancer agent is an anticancer formulation, which contains (a) an active camptothecin derivative designed to bind to the DDX5 protein and an E3 ligase as a molecular glue; or (b) a prodrug thereof designed to release the active camptothecin derivative (a) at a target site *in vivo.* This method includes designing the active camptothecin derivative (a) or the prodrug thereof (b) to contain the camptothecin-based backbone represented by Chemical Formula 1 as a parent nucleus, selecting a compound designed in this way, or synthesizing a compound designed in this way.

Hereinafter, the present invention will be described.

In this specification, "drug" is any material that is used to diagnose, cure, alleviate, treat, or prevent a disease (excluding food or a device) or used to affect a body structure or function. For example, a drug is any chemical or biological material that affects the body and its metabolism. Preferably, any material belongs to the category of drugs as long as it binds to the DDX5 protein.

For example, the drug of the present invention may be an active camptothecin derivative itself or a prodrug thereof, an active camptothecin derivative as a prodrug payload such as targeted drug delivery (e.g., a carrier-drug conjugate), or a camptothecin derivative or a prodrug thereof as a diagnostic agent.

For a drug to effectively act *in vivo,* the concentration of the drug at the target site in the body must be maintained within a therapeutic range for a certain period of time (Example 8, Table 6, and FIG. 23). When the drug is present in an excessive amount in the body, it becomes toxic, and when the concentration of the drug at the target site is too small, a therapeutic effect is not shown.

Drug efficacy means that a drug remains in the body without being decomposed for the expected period of time to exert an effect on target indications (Example 8, Table 6, and FIG. 23). The lower the metabolic rate, the longer the blood concentration maintenance time, and thus the duration of efficacy becomes longer.

Meanwhile, the *in vivo* efficacy and *in vivo* side effects of an anticancer agent are closely related to the absorption, distribution, metabolism, and excretion (ADME) properties of the anticancer agent. The ADME of a drug determines pharmacokinetics, which describes how the drug moves through the body and interacts with tissue and organs.

The absorption of a drug can affect its efficacy and side effects. A drug that is not well absorbed may not reach a therapeutic concentration at target tissue, resulting in reduced efficacy. On the other hand, a well-absorbed drug may increase systemic exposure and cause non-target side effects.

The distribution of a drug may also affect its efficacy and side effects. Drugs that are poorly distributed to target tissue may be ineffective, and drugs that are widely distributed to non-target tissues may cause toxicity in the corresponding tissue. In addition, a drug with high binding affinity to a plasma protein may have reduced efficacy due to decreased distribution to target tissue.

Drug distribution in the body may be influenced by physicochemical properties, including size, charge, and lipophilicity. The ability of a drug to penetrate tumor tissue may be influenced by factors such as the tumor microenvironment, including blood flow and cell density. Some anticancer agents may be accumulated in certain tissue and cause a toxic effect.

Drug metabolism may also affect both efficacy and side effects. A drug that is rapidly metabolized may have reduced efficacy due to its short half-life, and a drug that is slowly metabolized may have increased toxicity due to accumulation. In addition, some metabolites may have side effects due to being more toxic than the parent drug.

The rate and route of excretion may also affect both the efficacy and side effects of a drug. A drug that is rapidly metabolized may have reduced efficacy due to its short half-life, and a drug that is slowly metabolized may cause toxicity due to accumulation. In addition, some drugs may be excreted unchanged or as active metabolites, which may result in long-term exposure or increased exposure and an increased risk of side effects.

Therefore, it is necessary to optimize the efficacy of an anticancer agent and minimize side effects *in vivo* by analyzing its ADME properties. That is, the evaluation and optimization of ADME profiles may improve the therapeutic index of such a drug, leading to better outcomes in cancer patients.

An anticancer formulation or anticancer composition means a medication or therapy designed to treat cancer or prevent the growth and spread of cancer cells. Anticancer formulations may include combinations of a drug, a chemical compound, and/or a biological agent, which target cancer agents by interfering with their ability to grow and divide.

The formulation of an anticancer drug may reflect a complex process including various steps of drug discovery, pre-clinical development, clinical trials, and regulatory approval. The goal is to develop safe and effective treatments that can be administered to patients with minimal side effects.

The development of anticancer formulations involves identifying target cancer cells and designing a drug or drug combination selectively targeting these cells while sparing healthy cells. This may be achieved by targeting a specific protein or enzyme overexpressed in cancer cells or interfering with the cell cycle, DNA replication, or other cell processes, essential for cancer cell growth and survival.

Once a promising drug candidate is identified, it undergoes preclinical development that evaluates safety and efficacy by testing the drug in laboratory and animal models. When the drug shows promise in preclinical studies, it may undergo clinical trials which are conducted in humans to determine safety, dosage, and effectiveness.

During clinical trials, drug formulations are optimized to be safely administered to patients and achieve a desired therapeutic effect. These trials may involve regulating the dose, administration route, or formulation of the drug to improve efficacy and minimize side effects.

As a drug candidate that kills target cells, (a) an active camptothecin derivative represented by Chemical Formula 1, which is designed to bind to the DDX5 protein and an E3 ligase; or (b) a prodrug thereof, which is designed to release the active camptothecin derivative (a) at a target site *in vivo* is provided.

[Chemical Formula 1]

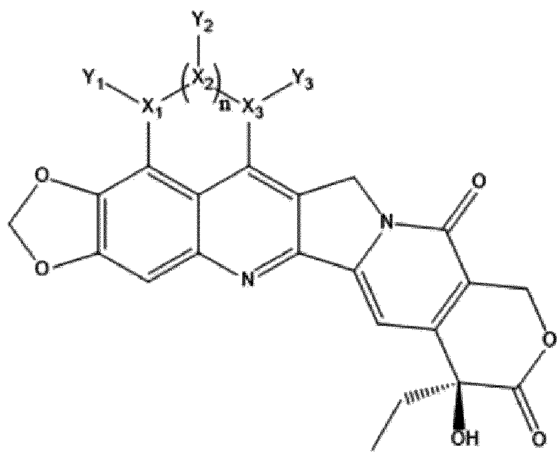

$X_1$ and $X_3$ may each be independently carbon, oxygen, nitrogen, or sulfur, and may be the same or different,
$X_2$ may be carbon, oxygen, nitrogen, sulfur, a single bond, or a double bond,
$X_1$, $(X_2)_n$ and $X_3$ may form a 6- or 7-membered ring (n= 1~2), and
$Y_1$, $Y_2$ and $Y_3$ may each be independently hydrogen, or a functional group containing oxygen, nitrogen, phosphorus or sulfur.

Here, non-limiting examples of the functional groups including oxygen, nitrogen, phosphorous, or sulfur may include functional groups selected from the group consisting of -CHO, -COOH, $-NH_2$, -SH, $-CONH_2$, $-PO_3H$, $-PO_4H_2$, $-OPO_4H$, $-PO_2(OR^1)(OR^2)$($R^1$, $R^2=C_sH_tN_uO_wS_xP_yX_z$, X = -F, -Cl, -Br or -I, $0\leq s\leq 20$, $0\leq t\leq 2(s+u)+1$, $0\leq u\leq 2s$, $0\leq w\leq 2s$, $0\leq x\leq 2s$, $0\leq y\leq 2s$, $0\leq z\leq 2s$), $-SO_3H$, $-OSO_3H$, $-NO_2$, $-N_3$, $-NR_3OH$($R=C_nH_{2n+1}$, $0\leq n\leq 16$), $-NR_3^+X^-$($R= C_nH_m$, $0\leq n\leq 16$, $0\leq m\leq 34$, X = OH, Cl or Br), $NR_4^+X^-$($R= C_nH_m$, $0\leq n\leq 16$, $0\leq m\leq 34$, X = OH, Cl or Br), - COSH, -COOCO-, -CORCO- ($R = C_lH_m$, $0\leq 1\leq 3$, $0\leq m\leq 2l+1$), -COOR, -CN, $-N_3$, $-N_2$, -NROH($R = C_sH_tN_uO_wS_xP_yX_z$, X = -F, -Cl, -Br or -I, $0\leq s\leq 20$, $0\leq t\leq 2(s+u)+1$, $0\leq u\leq 2s$, $0\leq w\leq 2s$, $0\leq x\leq 2s$, $0\leq y\leq 2s$, $0\leq z\leq 2s$), $-NR^1NR^2R^3$($R^1$, $R^2$, $R^3 = C_sH_tN_uO_wS_xP_yX_z$, X = -F, -Cl, -Br or -I, $0\leq s\leq 20$, $0\leq t\leq 2(s+u)+1$, $0\leq u\leq 2s$, $0\leq w\leq 2s$, $0\leq x\leq 2s$, $0\leq y\leq 2s$, $0\leq z\leq 2s$), $-CONHNR^1R^2$($R^1$, $R^2 = C_sH_tN_uO_wS_xP_yX_z$, X = -F, -Cl, -Br or -I, $0\leq s\leq 20$, $0\leq t\leq 2(s+u)+1$, $0\leq u\leq 2s$, $0\leq w\leq 2s$, $0\leq x\leq 2s$, $0\leq y\leq 2s$, $0\leq z\leq 2s$), $-NR^1R^2R^3X'$($R^1$, $R^2$, $R^3 = C_sH_tN_uO_wS_xP_yX_z$, X = -F, -Cl, -Br or -I, X'= $F^-$,$Cl^-$,$Br^-$, or $I^-$, $0\leq s\leq 20$, $0\leq t\leq 2(s+u)+1$, $0\leq u\leq 2s$, $0\leq w\leq 2s$, $0\leq x\leq 2s$, $0\leq y\leq 2s$, $0\leq z\leq 2s$), -OH, -O-, >C=O, -SS-, -SO-, $-NO_2$, -COX(X = F, Cl, Br or I), -COOCO-, -CONH-, -CN, $-SCOCH_3$, -SCN, -NCS, -NCO, -OCN, -CN, -F, -Cl, -I, -Br, epoxy, -hydrazone, $-ONO_2$, $-PO(OH)_2$, $-C=NNH_2$, -HC=CH-, -C=C-, -C≡C-, and a hydrocarbon with two or more carbon atoms:

The active camptothecin derivative (a) designed to bind to the DDX5 protein and the E3 ligase according to the present invention may kill target cells expressing the DDX5 protein through a molecular glue MOA.

The target cells may be cancer cells or senescent cells. Senescent cells also include cells that no longer perform an organ's specific functions.

Preferably, the active camptothecin derivative (a) designed to bind to the DDX5 protein and the E3 ligase according to the present invention may have multiple mechanisms of action (MoAs) that degrade the oncoprotein DDX5 as well as the ability to inhibit topoisomerase-1.

For example, the present invention exposes tumor antigens through cancer cell death in tumor tissue, thereby inducing antitumor immune responses by the stimulation of an antigen-presenting cells (APCs) such as dendritic cells and macrophages.

In addition, by providing (a) an active camptothecin derivative represented by Chemical Formula 1, which is designed to bind to the DDX5 protein and E3 ligase; or (b) a prodrug thereof, which is designed to release the active camptothecin derivative (a) at a target site *in vivo* as drug candidates killing cancer cells in various ways, the present invention may design a variety of active camptothecin derivative (a)-based anticancer formulations, and their doses and dosages to control the bystander effect and/or ADME profiles of the active camptothecin derivatives (a) to avoid T cell exhaustion caused by chronic antigen exposure.

Regarding the modification of Ri and $R_2$ on ring A (Group A) proven to allow greater permissibility in the design of camptothecin-based drugs as shown in General Formula 1 by applying camptothecin derivatives exerting a topoisomerase-1 inhibitor effect, the active camptothecin derivatives of the present invention are designed identically to the FL 11 8 compound of Chemical Formula 2 (Example 1), exhibiting an anticancer mechanism that degrades the oncoprotein DDX5 (Example 2).

[General Formula 1]

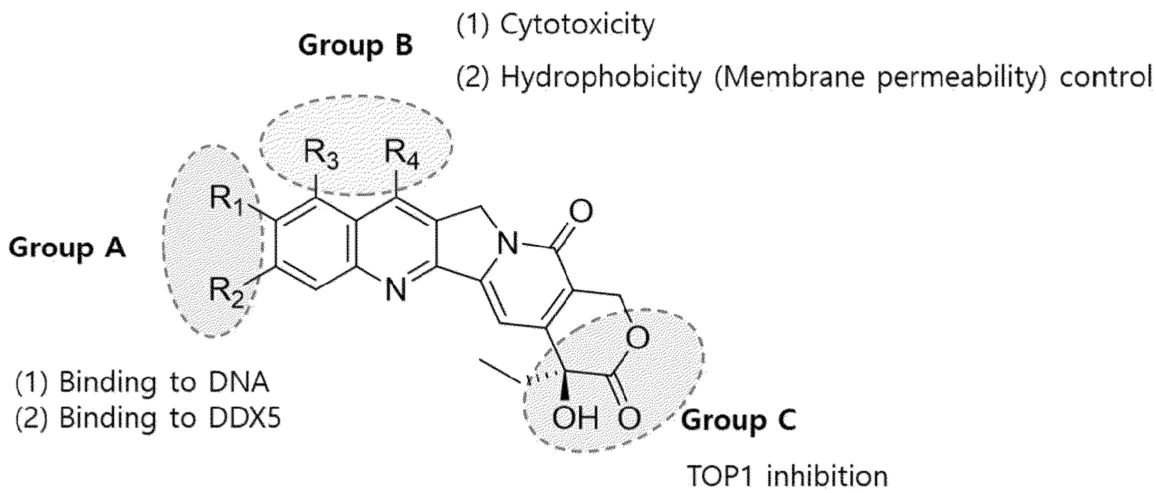


[Chemical Formula 2]

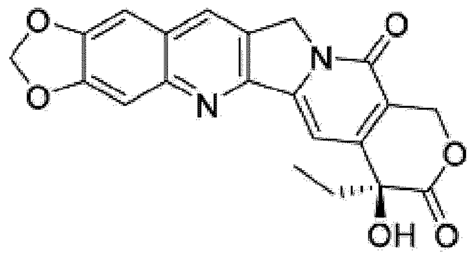

FL118

This is based on the finding that, in General Formula 1, the active camptothecin derivatives of Chemical Formula 1 with Ri and $R_2$ designed identically to the FL118 compound, for example, a compound of Chemical Formula 3, 4, or 5 below also degrades DDX5, which is a transcription cofactor and an oncoprotein, thereby strongly inhibiting the expression of anti-apoptotic proteins (survivin, cIAP2, XIAP, etc.) in a concentration-dependent manner (FIGS. 11 to 14) and killing cells into which the compound is introduced, observed through a cell viability test (FIGS. 15 and 16).

[Chemical Formula 3]

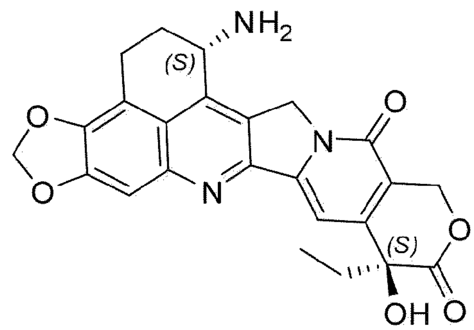

[Chemical Formula 4]

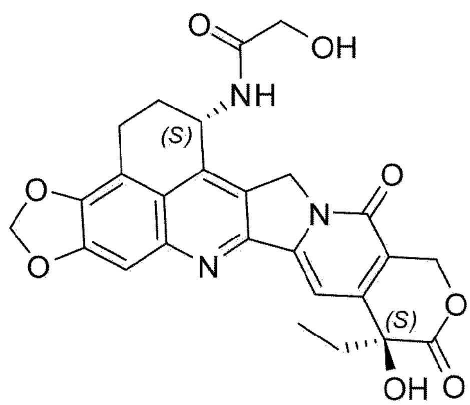

[Chemical Formula 5]

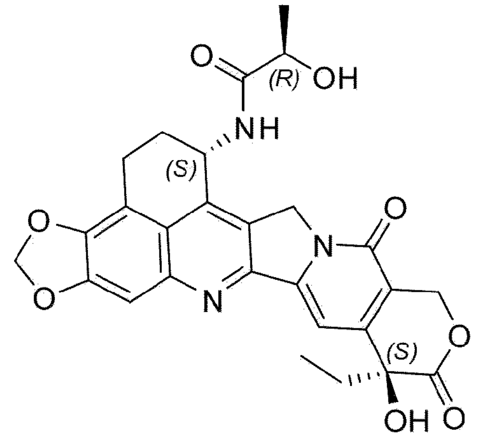

In General Formula 1 or Chemical Formula 2, Group C may bind to topoisomerase-1 and Group A may bind to DNA, such that the covalent bonding of the topoisomerase-DNA complex may be stabilized, preventing the re-ligation of cleaved DNA fragments (Example 1). Alternatively, in General Formula 1 or Chemical Formula 2, Group A may bind to DDX5 and Group C may bind to an E3 ligase, thereby inducing DDX5 degradation (FIGS. 11 to 14).

Topoisomerase-1 inhibitors are anticancer mechanisms with clinically proven efficacy/safety, and have been developed as the camptothecin-based drug, such as exatecan, and exatecan derivatives, such as Dxd and SN-38, have been developed as payloads for ADCs.

The drug exatecan is a material with cytotoxicity that is 5 to 10 times more potent than the SN-38 drug. Exatecan differs from irinotecan and does not require enzymatic activation. In addition, it has stronger topoisomerase-1 inhibitory activity than SN-38, which is the active ingredient of irinotecan, or topotecan used in the same clinical trial, and stronger cytotoxic activity against various cancer cells *in vitro.* Particularly, it also showed effectiveness against cancer cells that exhibit a tolerance to SN-38 or the like by the expression of P-glycoprotein. In addition, it showed a strong anti-tumor effect in a human tumor subcutaneously transplanted mouse model, and clinical trials were conducted.

The FL118 drug in Chemical Formula 2 is a hydrophobic small molecule that can penetrate the cell membrane. The FL118 drug is a potent topoisomerase-1 inhibitor that has the same camptothecin parent core structure as the existing commercialized Top I inhibitors such as SN-38, topotecan, and exatecan (FIGS. 1 and 2), and also is an anticancer agent that has a wide therapeutic window due to having differentiated anticancer efficacy and excellent safety compared to SN-38 in various cancer cells and animal models when administered alone.

The present inventors synthesized the FL118 drug, which is a camptothecin-based compound, and continued to conduct various studies on its physiochemical properties, but FL 118 had limitations in formulation development due to its physicochemical properties.

The present inventors confirmed that the FL118 drug, when used as an antibody-drug conjugate (ADC) payload, has stronger cytotoxicity than an ADC using SN-38 as a payload, and has the same level of cytotoxicity as the exatecan drug.

Meanwhile, the FL118 drug is a camptothecin-based compound which has a dual MoA that has an excellent ability to inhibit topoisomerase-1 and degrades the oncoprotein DDX5 (p68).

Surprisingly, it was found that the exatecan drug, like the FL 118 drug, can strongly inhibit the expression of an anti-apoptotic protein (e.g., survivin, cIAP2, and XIAP), which is another main cause of tolerance to anticancer agents, at a low concentration, and therefore can block a drug tolerance mechanism (FIG. 10).

Accordingly, based on the above finding, as shown in FIG. 1, the present invention is characterized by designing, from the FL118 compound, (a) a novel active camptothecin derivative that has a dual MoA that has an excellent ability to inhibit topoisomerase-1 and degrades the oncoprotein DDX5 (p68) , (b) a prodrug thereof, which is designed to release the active camptothecin derivative (a) *in vivo,* and (c) a ligand-containing complex which targets the DDX5 protein, in which the topoisomerase-1 inhibitory ability of the active camptothecin derivative (a) is inactivated by a linker conjugation.

Specifically, the synthetic design concept of the active camptothecin derivative (a) having a dual MoA that inhibits topoisomerase-1 and degrades the oncoprotein DDX5 may be to design a structure that, as shown in FIG. 1, can maintain the structure of Group C, which is a topoisomerase-1 inhibition region, and the same structure of Group A, which is a binding site for DDX5 degradation, as FL118, improve the physicochemical properties of the drug by modifying the structures ($R_3$ and $R_4$) of Group B in General Formula 1 as shown in Chemical Formula 1, and adjust a bystander effect by adjusting the cytotoxicity of the drug and/or the cell membrane permeability of the drug, based on the structure-activity relationship (SAR) (Example 9, and FIGS. 25 and 26).

When simply comparing cell viability, PBX-7016 (Chemical Formula 5) which is newly designed and synthesized according to the present invention showed an $IC_{50}$ value 1.5 to 2 times higher than that of the Dxd drug, but surprisingly, when used as an ADC payload, the PBX-7016-containing ADC exhibited a cell viability-related cytotoxic effect that was approximately 5 times better than the Dxd-containing ADC (Enhertu) (Example 7, and FIG. 22). The compound (PBX-7016) represented by Chemical Formula 5 and its isomer, such as a compound (PBX-7017) represented by Chemical Formula 5-1, are diastereomers.

Accordingly, in the present invention, it is preferable to design an active camptothecin derivative so that an appropriate bystander effect is exhibited in cancer tissue by adjusting cell membrane permeability as desired through $R_3$ and/or $R_4$ modifications (e.g., PBX-7014, PBX-7016, PBX-7018, PBX-7020, PBX-7022, and PBX-7024) in General Formula 1.

As shown in FIG. 1, in the present invention, a compound with a novel structure such as PBX-7011, similar to the structure of exatecan, was designed and synthesized by modifying $R_3$ and $R_4$ of Group B in General Formula 1 (Preparation Example 1). As shown in FIG. 2, it was confirmed through calculated LogP (or cLogP) and tPSA values that PBX-7011 of Chemical Formula 3 newly designed according to the present invention has relatively high hydrophilicity compared to FLU 8 of Chemical Formula 2.

Further, compounds with new structures, PBX-7014 and PBX-7016, were designed and synthesized by modifying Group B of General Formula 1 in the newly-designed PBX-7011 structure (Preparation Examples 2 and 3). It was calculated that the PBX-7014 and PBX-7016 show similar hydrophilicity values to that of Dxd derived from exatecan (FIGS. 1, 2, and 27).

FIG. 27 shows the comparison of the solubility and topological polar surface areas (tPSAs) of FL118, exatecan, SN-38, Dxd, exatecan-lactic acid, PBX-7011/PBX-7012, PBX-7014/PBX-7015, and PBX-7016/PBX-7017.

The relative hydrophilic/hydrophobic properties of a drug have important effects on its solubility, and absorption, distribution, metabolism, and excretion (ADME). How easily the drug passes through the cell membrane is important in receptor interactions.

The hydrophobicity of the drug is expressed by the partition coefficient (*p*).

When the drug is hydrophobic, the p value is large, and when the drug is hydrophilic (polar), the p value is small. In fact, the log P value is used as a measure to evaluate hydrophobicity. Calculated log P (cLog P) refers to a log P value of the given compound obtained through appropriate software. The smaller the cLog P value, the higher the polarity.

The polar surface area (PSA) or topological polar surface area (tPSA) of a molecule is defined as the sum of the surfaces of all polar atoms or molecules, mainly, oxygen and nitrogen, including attached hydrogen atoms. tPSAis a commonly used medicinal chemistry measurement method generally used to optimize the cell penetration ability of a drug. Molecules with a polar surface area greater than 140 $Å^2$ tend not to penetrate cell membranes. Molecules generally need a tPSA of less than $90Å^2$ to cross the blood-brain barrier and act on a receptor of the central nervous system.

PBX-7016/PBX-7017 (Chemical Formulas 5 and 5-1) of the present invention have a methyl group, which is a metabolically unstable functional group, introduced to the PBX-7014/PBX-7015 (Chemical Formulas 4 and 4-1), to reduce the drug lifespan. Drugs that are extremely stable in metabolism and metabolized very slowly require an appropriate retention time because they may accumulate and likely cause toxicity and serious side effects.

The active camptothecin derivative (a) newly designed according to the present invention is a hydrophobic small molecule designed by modifying $R_3$ and $R_4$ of General Formula 1 to penetrate cell membranes. When delivered to cancer tissue, the active camptothecin derivative (a) can accumulate at a high concentration while penetrating deep into the cancer tissue, and after an ADC, such as the active camptothecin derivative (a) or a prodrug thereof (b), penetrates the cell membrane, the active camptothecin derivative (a) exerts cytotoxicity inside the cell, causing cell death, is released into the extracellular fluid, and can continuously penetrate cell membranes in surrounding cells and migrate into the cells to exert a cell death mechanism (Example 9, and FIGS. 25 and 26). Therefore, the active camptothecin derivative of the present invention can transform a solid tumor agglomerated due to high cell density into scattered cancer with a low cell density, and/or a cold tumor with low immune activity into a hot tumor with high immune activity. In addition, the active camptothecin derivative (a) has a high effect of killing surrounding cells, so it is advantageous in the treatment of heterogeneous tumors.

In summary, as shown in FIGS. 1, 2, 27, and 28, the present invention cannot only design various camptothecin derivatives with new structures and prodrugs thereof (FIG. 3), exerting a dual MoA for topoisomerase-1 inhibition and DDX5 protein degradation, but can also control the cell membrane permeability related to a bystander effect by controlling hydrophilicity in various ways when designing the camptothecin derivatives.

**[FL118 drug]**

FL 11 8 of Chemical Formula 2 may serve as a molecular glue degrader that degrades DDX5 by directly binding DDX5 and a ubiquitination regulator.

FL118, acting as a molecular glue, directly binds to a multifunctional master regulator, the oncoprotein DDX5, and has a function of dephosphorylating and degrading DDX5 through a proteasome degradation pathway without reducing

DDX5 mRNA, and DDX5 silencing indicates that DDX5 is a master regulator that regulates the expression of several oncogenic proteins, including survivin, Mcl-1, XIAP, cIAP2, c-Myc, and mutant Kras.

FL118 directly controls DDX5 downstream targets to suppress cancer initiation, development, metastasis, recurrence and treatment resistance with high efficacy as proven in studies using human colorectal cancer/pancreatic ductal adenocarcinoma cells, and tumor models.

The genetic manipulation of DDX5 in PDAC cells affects tumor growth. PDAC cells with DDX5 KO are resistant to FL118 treatment. In studies with human tumor animal models, FL118 exerts high efficacy to remove human PDAC and CRC tumors with high DDX5 expression, but FL118 is proven to be less effective in PDAC and CRC tumors with low DDX5 expression.

The DDX5 protein is a direct target of the FL118 drug and may serve as a biomarker for predicting PDAC and CRC tumor sensitivity to FL118.

Meanwhile, the FL118 drug has Top1 inhibitory efficacy equivalent to or higher than SN-38 in cancer cells, and exhibits cytotoxicity in various cancer lines with a low $IC_{50}$ value, which is 5 to 20 times higher than Sn-38, and the results of evaluation on 140 cell lines originating from various cancer types also showed very strong anticancer efficacy, with $IC_{50}$ of < 100 nM against the majority of cancer cells. The FL 118 drug exhibited excellent safety through GLP-toxicity tests in mice and beagle dogs, and showed superior efficacy compared to SN-38 in various cancer cell line xenograft models. Meanwhile, when used for patients, the camptothecin-based anticancer agent initially showed excellent anticancer responses, but showed strong tolerance to the drugs through the epigenetic silencing of the Top1 gene and the Top2 dependence of cancer cells. In contrast, the FL118 drug showed strong efficacy even in a xenograft model of a cancer cell line in which Top1 is not expressed through epigenetic silencing or knock-out.

In addition, the FL118 drug is a triple-target anticancer agent that inhibits resistance proteins involved in the resistance mechanism, such as the Bcl family including survivin, and simultaneously inhibits the action of an efflux pump, while directly targeting topoisomerase-1, which is a well-established anticancer target.

Specifically, while camptothecin-based anticancer agents such as SN-38 show resistance in the form of drug excretion from cells by the overexpression of the ABCG2 transporter, the FL118 drug is not affected by the ABCG2 transporter, so it is possible to overcome the resistance caused thereby. The FL118 drug strongly suppresses the expression of an anti-apoptotic protein (e.g., survivin, cIAP2, XIAP, etc.), which is another main cause of resistance to anticancer agents, at a low concentration, thereby blocking the expression of resistance (FIG. 10).

Accordingly, the FLU 8 drug is not released out of cells by the efflux pump ABCG2, and is able to block resistance caused by various anti-apoptotic proteins. As a result, the FL118 drug may overcome various resistance action mechanisms of SN-38/exatecan.

The FL118 drug showed strong tumor regression efficacy compared to SN-38 in colorectal cancer/head and neck cancer/pancreatic cancer when administered *in vivo* at the same amount as SN-38.

The FL118 drug exhibited potent anticancer efficacy when FL118 was administered after inducing SN-38 resistance in tumor xenografts. The potential of the FL118 drug to overcome various resistance mechanisms of SN-38/exatecan was confirmed *in vivo.*

Moreover, the FL118 drug has PK/safety profiles, which are optimal for applying targeted drug delivery (e.g., carrier-drug conjugate). When the FL118 drug is systemically administered alone, it is rapidly metabolized/excreted from the blood and only shows a low concentration, but it accumulates rapidly in cancer tissue immediately after administration and maintains a high concentration for a long time. For example, the maximum selectivity between tumor tissue and normal tissue is ensured when applying ADCs. These results are the same as those for the active camptothecin derivative (a) of Chemical Formula 1 (Examples 6 and 8).

For example, the FL118 drug (i) selectively inhibits/degrades anticancer targets, such as DDX5, UBE2T, and USP2a, other than the well-validated anticancer target Top 1, to create a strong anticancer effect;

(ii) exhibits cellular cytotoxicity 5 to 10 times stronger than SN-38 and equivalent or higher than exatecan;
(iii) can avoid the resistance mechanisms by the overexpression of transporter proteins such as ABCG2 and P-GP because it is not a substrate of these proteins, unlike SN-38 and exatecan;
(iv) exhibits an excellent effect in cancer animal models in which the epigenetic silencing of Top 1/the overexpression of Top 2, which is the main mechanism of development of Top 1 inhibitor resistance, occurs;
(v) can avoid resistance by fundamentally blocking the overexpression of an anti-apoptotic protein, which is a common mechanism of resistance development in most types of cancer; and
(vi) has biomarkers (DDX5, K-ras, and p53) capable of predicting anticancer responses in patients, and companion diagnostic techniques, which have been already established, enables personalized treatment through companion diagnostics by ensuring customized biomarkers, and exhibits strong efficacy, particularly, in p53/K-ras mutant cancer cells with poor prognosis.

KRAS is one of three types of genes involved in the production of RAS proteins involved in cell proliferation

signaling. It is known that mutations occur in approximately 20% or more of overall cases, although it varies depending on cancer type. However, despite the scientific expectation of anticancer efficacy, KRAS was treated as an undruggable target. This is because no pocket suitable for compound binding was found due to the smooth surface of the RAS protein.

**[Anticancer mechanism 1 as selective inhibitor of topoisomerase-1]**

Camptothecin is a selective inhibitor of topoisomerase-1, which is an isomerase involved in DNA replication and re-ligation (FIG. 2). Since it was proven to exhibit strong cytotoxicity *in vitro,* clinical trials were initiated at the National Cancer Institute (NCI) in the United States and other institutions, but development has halted due to its extremely poor solubility, causing various side effects such as bone marrow suppression and hemorrhagic cystitis. However, since 1990, the unique mechanism of action (MoA) of camptothecin in which DNA topoisomerase-1 is selectively inhibited, unlike the DNA topoisomerase-2 inhibition mechanism, which results in an anticancer effect, has been confirmed.

DNA topoisomerases are enzymes in the nucleus, which temporarily cleave DNA or unwind the double helix when a cell requires access to a genetic material for replication or transcription. They are also involved in various intracellular activities, including chromosome condensation and recombination, and DNA repair. The genetic code for topoisomerase-1 is considerably conserved across species.

Topoisomerase-1, which is a drug target of camptothecin, has been observed to be increased in various malignant tumors. This drug does not inhibit a free enzyme, but stabilizes the covalent bond of a topo-DNA complex, preventing the re-ligation of cleaved DNA fragments. Therefore, the sensitivity of cells to such a topoisomerase-targeting drug is related to the level of the enzyme present in the nucleus. This drug prevents transcription from proceeding by interfering with DNA recombination. The larger the amount of topoisomerase-1, the more cleavable complexes are formed, indicating higher drug sensitivity. This has significant clinical relevance, as a topoisomerase-1 inhibitor is used to increase the expression of topoisomerase-2, making it more sensitive to a topoisomerase-2 inhibitor. These results are supported by the antagonistic relationship between topoisomerase-1 and topoisomerase-2. Unlike topoisomerase-2, topoisomerase-1 is not closely related to proliferation in normal tissue, is present in large quantities in solid tumors in the "S" phase with active cell division, such as colorectal cancer, ovarian cancer, and esophageal cancer included as lymphoma, compared to surrounding normal tissue, and is known to cause cell death by blocking the replication and transcription of genes in tumor cells in the "S" phase of the cell cycle.

**[Anticancer mechanism 2 as molecular glue degrader binding to DDX5]**

The ubiquitin-proteasome system (UPS) is a crucial pathway for the degradation of intracellular proteins that regulate a wide range of cellular processes. Ubiquitin is a small protein that covalently binds to a lysine residue of a substrate protein by a series of enzymatic reactions involving ubiquitin-activating enzymes (E1s), ubiquitin-conjugating enzymes (E2s), and ubiquitin ligases (E3s). This process is called ubiquitination, which is a significant mechanism for regulating protein degradation, signaling, and trafficking.

Ubiquitin ligases are responsible for substrate specificity in the ubiquitination pathway.

In this regard, the active camptothecin derivative represented by Chemical Formula 1 according to the present invention is designed to bind to the DDX5 protein and an E3 ligase.

DDX5 (also called p68) is a multifunctional master regulator acting in the following mechanisms: (1) a biological process for co-activating the transcription of many oncogenes through direct interactions with various transcription factors (e.g., c-Myc) at oncogene promoters, and (2) a biological process for regulating miRNA and pre-RNA splicing (e.g., U1, U2, U3, ... snRNP), and (3) ribosome biogenesis (e.g., 32S rRNA, pre-ribosome).

The active camptothecin derivative represented by Chemical Formula 1 according to the present invention binds to the DDX5 protein without reducing DDX5 mRNA to functionally degrade the DDX5 protein through dephosphorylation and proteasomal degradation pathways, suggesting that the active camptothecin derivative of Chemical Formula 1 can bind to both DDX5 and ubiquitin-involved protein stability/degradation regulators, acting as a "molecular glue degrader."

DDX5 downstream protein targets are known to be involved in cancer initiation, development, metastasis, recurrence and treatment resistance. Accordingly, when DDX5 downstream targets are indirectly blocked through the degradation of the DDX5 protein by the active camptothecin derivative represented by Chemical Formula 1 according to the present invention, the active camptothecin derivative represented by Chemical Formula 1 may exhibit high antitumor efficacy.

**[DDX5 as drug target and/or biomarker]**

Transcription factors are a group of proteins that bind to DNA fragments called promoters or enhancers through DNA-binding domains, and initiate the transcription of a gene with the cooperation of RNA polymerase and other cofactors. They play a key role in regulating transcription during embryogenesis and development. The physiological state of

transcription factors in other cell types is also important for maintaining cellular homeostasis. Therefore, when a transcription factor is unregulated, it will lead to the development of cancer cells and tumor progression. A transcription factor may be oncogenic or tumor suppressive depending on its function in cancer cells. In addition, transcription factors regulate cancer stem cells, the epithelial-mesenchymal transition (EMT), and drug responses. Therefore, measuring a dysregulated transcription factor is considered to be predictive of the treatment outcome of cancer patients, and targeting dysregulated transcription factors can be an encouraging strategy for cancer treatment. Dysregulated key transcription factors may lead to poor clinical results in cancer patients. Accordingly, it will help predict prognosis and drug responses and design new drugs and therapeutic strategies for cancer treatment by targeting dysregulated transcription factors.

It is well known that transcription is the first step of gene expression in cells during embryogenesis, development, and homeostasis. During the transcription process, an RNA transcript having specific coding information is produced from DNA. To ensure the specificity of gene expression in different cells and tissue, transcription factors, which are a group of specific proteins, control transcription by selecting genes to be transcribed into RNA transcripts. A transcription factor has a DNA-binding domain, may bind to a specific DNA sequence, such as a promoter or enhancer of a gene, and may initiate the transcription of the gene with the cooperation of an activator, a mediator, a cofactor, and RNA polymerase.

Transcription factors also control the rate of transcription from DNA to mRNA. Consequently, the physiological state of transcription factors in different types of cells is important for maintaining cellular homeostasis. Changes in the physiological state of transcription factors in cells cause transcriptional disorder and the translation of a specific gene, resulting in pathological changes in cells. Therefore, dysregulated transcription factors may cause many human diseases. One of the common diseases with dysregulated transcription factors is cancer.

Homeostatic control in human cells generally prevents inappropriate proliferation and suppresses the growth of cells that proliferate abnormally outside their normal niche. Since transcriptional control is a key process during homeostasis, when the level of activity of a transcription factor is not regulated, cells may escape homeostatic control, leading to the development and progression of human cancer.

In fact, many chemotherapeutics can activate tumor suppressor transcription factors.

Dysregulated transcription factors play an important role in tumorigenesis and tumor progression. They control central tumor signaling pathways, such as NF-κB, Notch, Wnt/β-catenin, and JAK/STAT. Unregulated oncogenic and tumor suppressor transcription factors favor the survival of cancer cells, cancer stem cells, and EMTcells, inducing intrinsic or acquired drug resistance in cancer treatment and tumor progression.

DDX5 (p68) is a well-known multifunctional DEAD-box RNA helicase and transcription cofactor. Therefore, when cancer occurs due to the deregulation of a transcription factor by the physiological state of DDX5, cancer may be treated by selectively degrading the transcription cofactor, DDX5 (p68). Similarly, cancer may be prevented by selectively degrading the transcription cofactor, DDX5 (p68).

DDX5 is recognized as a potential biomarker and a target for the treatment of various cancer types. Studies over the years have greatly expanded the understanding of the functional diversity of DDX5 in various cancer types and expanded the knowledge of its MoA. This provides a rationale for the development of novel cancer therapeutics using DDX5 as a biomarker and a therapeutic marker. However, while most published studies have found DDX5 to be an oncogenic target and a cancer treatment resistance biomarker, some studies have reported that DDX5 may act as a tumor suppressor in certain scenarios. The multiple functions of DDX5 make it an excellent independent oncogenic biomarker as well as a target for targeted cancer therapy.

The transformation of normal cells into cancerous cells occurs through the deregulation of different metabolic pathways involving a complex network of protein-protein interactions. Cellular enzymes and DDX5 play a critical role in maintaining normal cellular metabolism, but when they are deregulated, tumor transformation may be accelerated. DDX5 interacts with hundreds of different cellular proteins, and depending on the specific pathway involved, both proteins may act as tumor suppressor genes or oncogenes.

RNA helicases of the DEAD-box family are involved in several metabolic pathways, including transcription and translation as well as cell proliferation, innate immunity, and stress responses. Given their diverse roles, deregulation of the RNA helicases or their mutations have been associated with various pathological conditions, including cancer. However, in some cases, as the loss of function of a given DEAD-box helicase accelerates tumor transformation, it can act as a tumor suppressor, but in other circumstances, as the overexpression of the corresponding enzyme favors the progression of cancer, it can act as a typical oncogene. The role of DDX5 as an oncogene and a tumor suppressor gene has been documented in several cancer types. Understanding the interplay of DDX5 in different cellular environments defined by the molecular interaction networks of DDX5 in different tumors can help explain the apparently conflicting, cancer-specific roles of the protein as both an oncogene and a tumor suppressor.

Although DDX5 acts much more often as an oncogene, it may also have tumor suppressor functions in certain cancer types (Table 1).

Refer to: Secchi, M.; Lodola, C.; Garbelli, A.; Bione, S.; Maga, G. DEAD-Box RNA Helicases DDX3X and DDX5 as Oncogenes or Oncosuppressors: A Network Perspective. Cancers 2022, 14, 3820. https://doi.org/10.3390/cancers14153820

[Table 1]

| Cancer | Role [1] | Mechanism | Ref. |
|---|---|---|---|
| Colorectal | + | DDX5 overexpression promotes cancer by AKT/mTOR signaling or association with AldoA | [62-66] |
| Breast | + | upregulates a subset of miRNAs; highly correlated with Ki67; involved in β-catenin/Wnt pathway | [67-69] |
| Leukemia | + | DDX5 depletion selectively induces stress in AML cells; positive regulator of NOTCH1 signaling; DDX5 inhibition reduces tumor proliferation | [70-72] |
| Non-small-cell lung/small cell lung | + | induces β-catenin to promote cell proliferation | [73,74] |
| Osteosarcoma | + | lncRNA DLEU1/miR-671-5p/DDX5 interaction to promote cancer progression | [75] |
| Prostate | + | lncRNA CCAT1/DDX5/miR-28-5p interaction to promote cancer progression; DDX5-ETV4 fusion protein | [76,77] |
| Gastric | + | high DDX5 expression activates mTOR/SK61 pathway to induce cancer progression; lnc MIAT interaction | [78,79] |
| Glioblastoma | + | NF-κB p50 subunit activation; lncRNA LINC01116 interaction; hyperactivation of ERK and downregulation of DUSP5 | [80-82] |
| Cervical | + | stimulation of the expression of TGF-β1 in CaSki cells | [83] |
| Endometrial | + | HDGF/DDX5 interaction to induce β-catenin | [84] |
| Squamous cell carcinoma (HNSCC) | + | high DDX5 expression is associated with cancer progression | [85] |
| Squamous cell carcinoma (FSCC) | + | decreased DDX5 expression is associated with inhibition of cancer progression | [86] |
| Human hepatocellular carcinoma (HCC) associated with HBV | - | inhibits cancer progression by interacting with p62/SQSTM1, by regulation of miRNAs and by associating with lncRNA HOTAIR | [87-91] |

[1] Oncogene (+), oncosuppressor (-).

Since the DDX5 protein is a drug target, the active camptothecin derivative represented by Chemical Formula 1 according to the present invention may avoid drug resistance, resistance to targeted therapy, and/or resistance during treatment. In addition, the transcriptional induction of anti-apoptotic genes may be blocked by the active camptothecin derivative. Further, the transcription cofactor DDX5 protein may be degraded by the active camptothecin derivative, thereby maintaining or improving the sensitivity of cancer cells to chemotherapy or radiotherapy.

**[Camptothecin derivatives represented by Chemical Formulas 1, and 3 to 9 and isomers thereof]**

The active camptothecin derivatives represented by Chemical Formula 1 according to the present invention, in which $R_1$ and $R_2$ in General Formula 1 are designed identically to the FL118 compound, for example, a compound of Chemical Formula 3, a compound of Chemical Formula 4, a compound of Chemical Formula 5, a compound of Chemical Formula 6, a compound of Chemical Formula 7, a compound of Chemical Formula 8, a compound of Chemical Formula 9, and isomers thereof are also transcription cofactors, and serve to degrade the oncoprotein DDX5, strongly suppress the expression of anti-apoptotic proteins (e.g., survivin, cIAP2, XIAP, etc.) in a concentration-dependent manner (FIGS. 11 to 14), and kill cells into which the compounds are introduced through a cell viability experiment (FIGS. 15 and 16).

The camptothecin derivatives represented by Chemical Formula 1, for example, Chemical Formulas 3 to 9 according to the present invention, are designed to have a pentacyclic structure with lactone in an E-ring essential for cytotoxicity, like the camptothecin shown in FIG. 1, and maintain a lactone group and an alpha-hydroxide group located at C-20 on the E-ring, which are critical for the stability of a topoisomerase-1-DNA byproduct. While maintaining $R_1$ and $R_2$ ($-OCH_2O-$ (methylenedioxo) pentagonal ring) of General Formula 1, which is the structure of the FLU 8 drug maintaining

the advantages of the above-described FLU 8 drug, the structural feature of the exatecan drug ($R_3$ and $R_4$ of General Formula 1) is that stacking of aromatic rings formed by rings A and B can be weakened or suppressed due to the dynamic equilibrium of various orientations of consecutive carbon-carbon single bonds of a 6-membered ring expanded from rings A and B, compared to SN-38 in which aggregation is induced by the $\pi$-$\pi$ stacking of aromatic rings forming rings A and B.

In addition, the compound represented by Chemical Formula 3 or 3-1 is designed such that $-NH_2$, which has a large degree of freedom due to the rotation of molecular bonds about the carbon-carbon single bond in a hexagonal ring extending from rings A and B, is exposed to water ($H_2O$) to carry a (+) charge or forms a hydrogen bond with water to increase water dispersibility.

Further, the compound represented by Chemical Formula 4 or 4-1 is designed such that the functional moiety $CH_2(OH)CONH$- of $-NH_2$, which has a large degree of freedom due to the rotation of molecular bonds about the carbon-carbon single bond in a hexagonal ring extending from rings A and B, is exposed to water ($H_2O$) and rotates like a propeller to form a hydrogen bond with water, resulting in increased water dispersibility.

Moreover, the compound represented by Chemical Formula 5 or 5-1 is designed such that the lactic acid-like functional moiety $CH_3CH(OH)CONH$- of $-NH_2$, which has a large degree of freedom due to the rotation of molecular bonds about the carbon-carbon single bond in a hexagonal ring extending from rings A and B, is exposed to water ($H_2O$) and rotates like a propeller to form a hydrogen bond with water, resulting in increased water dispersibility.

The compound represented by Chemical Formula 5 or 5-1 of the present invention has a methyl group, which is a metabolically unstable functional group unstable, introduced to the compound represented by Chemical Formula 4 or 4-1 to shorten the drug lifespan. Drugs that are extremely stable in metabolism and metabolized very slowly require an appropriate retention time because they may accumulate and likely cause toxicity and serious side effects.

Meanwhile, the DXd payload used in Enhertu was originally made from the exatecan compound, which is hardly affected by ABCG2, but was strongly affected by ABCG2 due to the presence of the glycolic acid (alpha-hydroxy acetic acid) functional group used to convert exatecan to DXd. However, the glycolic acid functional group plays a very important role in the excellent safety/efficacy profiles of Enhertu, and removing this causes difficulties in manufacturing ADCs and also deteriorates (leading to a safety problem or reduced efficacy) the performance of ADCs in animal models and clinical trials. There is a great need for a new camptothecin derivative that can be easily used in ADC preparation and is not affected by ABCG2. A compound represented by Chemical Formula 9 (PBX-7024) is a compound derived from the compound represented by Chemical Formula 3 (PBX-7011), and a novel camptothecin compound that can be easily used in ADC preparation without being affected by ABCG2.

To confirm the anticancer efficacy of PBX-7024, efficacy was evaluated in FaDu, which is a cancer cell that does not express ABCG2, and A549, which is a cancer cell overexpressing ABCG2. It was confirmed that, in A549, which is a cancer cell line that overexpresses ABCG2, as shown in FIG. 15, PBX-7016 and PBX-7024 still maintain potent efficacy compared to camptothecin compounds including DXd, which show increased $IC_{50}$ values.

That is, when the novel camptothecin compounds, PBX-7016 and PBX-7024, according to the present invention are used, unlike ADCs using the existing camptothecin compounds, SN-38 and DXd, they have the effect of overcoming the resistance mechanism caused by the overexpression of ABCG2.

FIGS. 27 and 28 show the comparison of the Log Ps, cLog Ps and topological polar surface areas (tPSAs) of FL118, exatecan, SN-38, Dxd, compound A, compound B, compound C, compound D, compound E, compound F, compound G, compound H, compound I, compound J, compound G', compound H', compound I', and compound J'.

The compound of Chemical Formula 3 and the compound of Chemical Formula 3-1 are diastereomers. Likewise, the compound of Chemical Formula 4 and the compound of Chemical Formula 4-1; and the compound of Chemical Formula 5 and the compound of Chemical Formula 5-1 are diastereomers. Accordingly, compounds that are in diastereomeric relationships with the compound of Chemical Formula 6, the compound of Chemical Formula 7, the compound of Chemical Formula 8, or the compound of Chemical Formula 9 also fall within the scope of the present invention.

As shown in Preparation Example 2, PBX-7014 of Chemical Formula 4 can be synthesized from PBX-7011 of Chemical Formula 3, and PBX-7015 of Chemical Formula 4-1 can be synthesized from PBX-7012 of Chemical Formula 3-1.

As shown in Preparation Example 3, the compound of Chemical Formula 5 (PBX-7016, Compound E) can be synthesized from the compound of Chemical Formula 3 (PBX-7011, Compound A), and the compound of Chemical Formula 5-1 (PBX-7017, Compound F) can be synthesized from the compound of Chemical Formula 3-1 (PBX-7012, Compound B) in the same manner.

In this specification, the camptothecin-based drug of Chemical Formula 3, Chemical Formula 3-1, Chemical Formula 4, Chemical Formula 4-1, Chemical Formula 5, Chemical Formula 5-1, Chemical Formula 6, Chemical Formula 7, Chemical Formula 8, or Chemical Formula 9 according to the present invention includes not only the compound of Chemical Formula 3, Chemical Formula 3-1, Chemical Formula 4, Chemical Formula 4-1, Chemical Formula 5, Chemical Formula 5-1, Chemical Formula 6, Chemical Formula 7, Chemical Formula 8, or Chemical Formula 9 but also a pharmaceutically acceptable salt thereof, a solvate thereof, and a prodrug thereof.

In this specification, a pharmaceutically acceptable salt refers to a salt commonly used in the pharmaceutical industry, and includes, for example, salts of inorganic ions such as sodium, potassium, calcium, magnesium, lithium, copper, manganese, zinc, and iron ions, salts of inorganic acids such as hydrochloric acid, phosphoric acid, and sulfuric acid, salts of organic acids such as ascorbic acid, citric acid, tartaric acid, lactic acid, maleic acid, malonic acid, fumaric acid, glycolic acid, succinic acid, propionic acid, acetic acid, orotate acid, and acetylsalicylic acid, and salts of amino acids such as lysine, arginine, and guanidine. In addition, a pharmaceutically acceptable salt includes salts of organic ions such as tetramethyl ammonium, tetraethyl ammonium, tetrapropyl ammonium, tetrabutyl ammonium, benzyl trimethyl ammonium, and benzethonium, which can be used in pharmaceutical reactions, purification, and separation processes. However, the type of salt referred to in the present invention is not limited by the salts listed above.

A"solvate" includes the compound represented by Chemical Formula 3, Chemical Formula 3-1, Chemical Formula 4, Chemical Formula 4-1, Chemical Formula 5, Chemical Formula 5-1, Chemical Formula 6, Chemical Formula 7, Chemical Formula 8, or Chemical Formula 9, and a pharmaceutically acceptable salt thereof, further including a stoichiometric or non-stoichiometric amount of solvent bound by a non-covalent intermolecular force. When the solvent is water, the solvate is a hydrate.

**[Advantages of drug modality as molecular glue degrader]**

Proteins in cells of the body are naturally degraded within hours to days after performing their functions. All cells in the body have a purification system called the ubiquitin proteasome system (UPS) that degrades proteins, and in this process, ubiquitin acts as a marker that indicates a protein to be degraded, and the proteasome acts as a grinder that recognizes a ubiquitin marker and destroys the relevant protein. That is, several substances called ubiquitin are attached like markers to proteins that have completed their function, and the substance called proteasome selects only proteins with these markers and degrades them like a grinder. An E3 ligase is an enzyme that initiates the protein degradation system in the body, and is responsible for substrate specificity in the ubiquitination pathway.

A molecular glue degrader or molecular glue is a compound that acts as an adhesive for bonding a target protein with a certain enzyme (E3 ligase) in the body. One of the advantages of molecular glue is that it acts as a catalyst, which can degrade a target and then be dissociated to degrade another target protein.

When the E3 ligase enzyme binds to an oncoprotein through a molecular glue, the oncoprotein is degraded, and other oncoproteins are degraded sequentially until the target oncoprotein disappears, thereby preventing cancer cell proliferation. Therefore, the molecular glue for the oncoprotein overcomes drug resistance, which is a problem of targeted anticancer agents, and has a high therapeutic effect even with a low dose.

In addition to the FL118 drug, the active camptothecin derivative designed in Chemical Formula 1 according to the present invention is a molecular glue degrader binding to the DDX5 protein and an E3 ligase, that is, a molecular glue activating the degradation of the oncoprotein DDX5 (FIGS. 11 to 14).

The molecular glue may act as not only a target protein-binding ligand (warhead), but also an E3 ligase ligand (binder).

Accordingly, in addition to FL118, since the active camptothecin derivative designed in Chemical Formula 1 according to the present invention is a molecular glue activating the oncoprotein DDX5 degradation, it can be used as a ligand targeting the DDX5 protein or a ligand binding to the DDX5 protein.

Unlike kinase inhibitors, molecular glues are 'proximity-driven' and their ability to induce degradation is 'event-driven' depending on the formation of a temporary 'target protein-molecular glue-E3 ligase' triple complex. After degradation occurs, dissociated molecular glues form an additional triple complex with a target protein, allowing multiple degradations until the target protein is eliminated.

Most drug-targeted proteins acquire resistance by adapting to a drug. However, a molecular glue, which is a small molecule compound acting as an adhesive for bonding an oncoprotein with an E3 ligase, is a modality suitable for cancer treatment because it is resistant to resistance.

Each time a genetic mutation occurs, the shape of a drug target protein slightly changes. It would be preferable to block the activity of all mutants with one drug, but this is not possible due to selectivity, so in order to block the activity of all mutants with one drug, the drug target protein is degraded and completely removed.

Therefore, the active camptothecin derivative represented by Chemical Formula 1 according to the present invention is a drug that can precisely bind to the DDX5 oncoprotein, and can avoid the resistance of targeted therapeutics through a molecular glue approach that selectively degrades the protein.

The active camptothecin derivative represented by Chemical Formula 1 according to the present invention is preferably an irreversible drug that binds so strongly that it cannot return to its previous state, and the binding strength of the derivative to the target protein, the DDX5 oncoprotein, is determined by its physicochemical properties.

Unlike the existing SN38 drug, the active camptothecin derivative including FL118, designed from Chemical Formula 1 according to the present invention, may not be easily dissociated due to high affinity with DDX5, degrade DDX5 by the function of a molecular glue not only to irreversibly inhibit the signaling of cancer cells associated with DDX5 but also

to regulate the cell membrane permeability of the drug according to the physicochemical properties thereof as desired, may be beneficial for treatment of heterogeneous tumors due to a high effect of killing surrounding cells by exhibiting a bystander effect or controlling the degree of its effect, may suppress the long-term progression of cancer, and may increase the treatment response rate by reducing the risk of developing resistance.

**[Cell death and drug resistance to anticancer agents]**

The active camptothecin derivative represented by Chemical Formula 1 according to the present invention may bind to DDX5 acting as an oncoprotein in cells to induce cell death through DDX5 protein degradation (FIG. 29).

There are two factors that determine tumor growth: cell proliferation and cell death. When the cell cycle of tumor cells is stopped by a cytotoxic material, the tumor cells die due to apoptosis.

Cell death plays a very important role in maintaining tissue homeostasis and morphology, regulating the cell number, and removing damaged or abnormal cells, and as a defense mechanism against infection, and living organisms maintain their lives normally by precisely regulating cell growth, differentiation, and death.

Apoptosis is described as a complex process that induces cell death through the expression of various genes and the activation of proteins, which are their expression products. Such apoptosis is an intracellular physiological, active suicidal mechanism, which plays an essential role in the development, differentiation, and homeostasis of multicellular organisms. Apoptosis occurs in a variety of cell types by many factors. Unlike necroptosis, in apoptosis, a lysosome enzyme is not released, apoptotic bodies are found, and cell reduction, nuclear condensation, and characteristic ladder-shaped DNA fragmentation are found.

Apoptosis is part of the normal developmental program in animals and is important in cancer prevention. Caspases, a family of proteolytic enzymes, are involved in cell death and cleave many target proteins. When apoptosis is impaired, cells that should be killed may survive and proliferate, forming clones that can potentially be cancerous.

In cancer treatment, intrinsic drug resistance may be caused by abnormally expressed transcription factors, which are critical regulators of cell proliferation and cell death. Therefore, the active camptothecin derivative of the present invention can induce DDX5 protein degradation and resulting cell death through the MoA as a molecular glue degrader binding to DDX5, which is a transcription cofactor and an oncoprotein. Here, DDX5 protein degradation may downregulate the transcription of anti-apoptotic genes. Therefore, unlike other targeted therapeutics, drug resistance caused by abnormally expressed cell proliferation and/or cell death-related transcription factors may not develop, and/or acquired drug resistance induced through the activation of dysregulated transcription factors during chemotherapy and/or radiotherapy may be inhibited.

That is, since the anticancer mechanism of the active camptothecin derivative according to the present invention may bypass the molecular mechanism of cancer treatment resistance, the active camptothecin derivative can be free of drug resistance issues. Since the therapeutic effect is lower than before resistance occurs, the active camptothecin derivative of the present invention may be preferred as a standard treatment or first-line treatment after cancer diagnosis.

For example, the active camptothecin derivative represented by Chemical Formula 1 according to the present invention may be a targeted anticancer agent that acts on the DDX5 protein, which plays an important role in the growth, survival, proliferation, metastasis, and/or metabolism of cancer cells.

**[ADME profile of antibody-drug conjugate (ADC)]**

The *in vivo* efficacy of an anticancer agent may be affected by ADME profiles. The ADME profile of a drug may affect its ability to reach and act on cancer cells. That is, the ADME profiles of an anticancer agent may significantly affect its *in vivo* efficacy.

The ability of a drug to penetrate tumor tissue may be influenced by factors such as the microenvironment of a tumor, including blood flow and cell density.

Therefore, understanding the ADME profile of an anticancer agent, selecting a drug/drug modality, and optimizing a dose/dosage may improve the therapeutic index of such an anticancer agent, leading to better results for cancer patients.

Antibodies (Abs) and antibody-drug conjugates (ADCs) have different lifetimes and ADME profiles due to different structures and MoAs.

Antibodies are large proteins that are naturally produced by the immune system in response to foreign materials (antigens). Antibodies may have a long circulatory half-life (several weeks to months) and may be protected from degradation and removal due to their size and complex structure. Antibodies are distributed throughout the body, including tissue, and may interact with target antigens with high specificity and affinity. Antibodies are removed primarily by catabolism in the reticuloendothelial system (RES), including the liver and the spleen, and the kidney and other organs.

ADCs consist of antibodies (usually monoclonal antibodies) conjugated to a cytotoxic drug molecule. The antibody component provides specificity and targeting for a tumor or diseased tissue, while the drug component provides

cytotoxic activity that kills target cells. ADCs have a shorter half-life than antibodies, typically ranging from a few days to a week. This is because ADCs are internalized into target cells, resulting in lysosomal degradation of ADCs and the release of drug payloads. ADCs are primarily eliminated by the RES, but the drug payloads may also undergo metabolism and excretion via the liver and kidneys (Example 4).

Antibodies are generally administered subcutaneously or intravenously and are absorbed into the bloodstream. While they can be distributed throughout the body, including tissue, they are generally restricted to the extracellular space due to their size. ADCs are also administrated by injection and absorbed into the bloodstream, but due to the antibody component, drug payloads released from the ADCs may penetrate into cells and tissue after being targeted to tumor or diseased tissue, and in some cases, through receptor-mediated endocytosis. Furthermore, the metabolism and excretion of the ADCs vary depending on the specific structure and specific drugs or antibodies used.

Antibody-drug conjugates (ADCs) using camptothecin-based payloads are receiving greater attention as a novel approach for treating various types of cancer, particularly, solid tumors. Among these ADCs, particularly, novel ADCs (e.g., Enhertu®) including a newly synthesized camptothecin-based payload (e.g., Dxd), optimized for ADC modality, have recently attracted special attention due to successful clinical results.

The success of these ADCs is due in part to the fact that novel camptothecin has the following characteristics: (1) excellent cell growth inhibition; (2) a better safety profile than existing ADC payloads; (3) an optimized bystander effect; and (4) excellent physicochemical properties suitable for high production of DAR ADCs. However, despite the remarkable success of ADCs utilizing a camptothecin-based payload, such as Enhertu or DS-1062a, there are still clear unmet requirements, including safety profile improvements (minimizing interstitial lung disease (ILD) and neutropenia) and the development of a novel ADC having multiple MoA payloads for addressing cancer heterogeneity.

To address the unmet requirements, the present invention provides various active camptothecin derivatives of Chemical Formula 1 as candidate drugs, designed to bind to the DDX5 protein and an E3 ligase as molecular glues, and synthesizes (Preparation Examples 1 to 4) and then evaluates (Examples 2 to 9) novel camptothecin-based payloads (PBX-series compounds) represented by Chemical Formula 1.

A novel PBX-series compound represented by Chemical Formula 1 is a derivative having FL118 of Chemical Formula 2 as a parent nucleus, which is a potent dual Top1/anti-cell death pathway inhibitor with an excellent safety profile. It exhibits strong *ex-vivo* cytotoxicity compared to Dxd, an excellent safety profile in preliminary toxicity studies in mice, and rapid elimination in PK studies (Example 4, and Tables 4 and 5). This may minimize systemic side effects when a linker is cleaved early when used as a payload of ADC.

Among the PBX-series compounds, two lead compounds, PBX-7014 and PBX-7016, were selected for further evaluation as ADC payloads linked to various HER2 antibodies, including trastuzumab.

Trastuzumab-7014 and trastuzumab-7016 (DAR 7 and 8) were easily synthesized without any aggregation problems by linking PBX-7014 or PBX-7016 to trastuzumab using an existing enzyme-cleavable linker in the same manner for comparative evaluation with Enhertu® (Dxd-trastuzumab) (Preparation Example 5, and FIGS. 19 and 20).

In a Her2-high cell line (MDA-MB-453) and a Her2-low/mid cell line (FaDu), these two novel ADCs exhibited better cytotoxicity than DS-8201a (anti-HER2-DXd), whereas in a Her2 negative cell line (MDA-MB-468), they exhibited a similar level of cytotoxicity to DS-8201a.

In an NCI-N87CDX model with high Her2 expression, trastuzumab-7014 and trastuzumab-7016 exhibited anticancer efficacy in a clear dose-dependent manner which is the same or similar, or even superior to that of DS-8201, and trastuzumab-7016 also exhibits a bystander effect similar to Enhertu® (Example 9, and FIGS. 25 and 26).

Trastuzumab-7014 and trastuzumab-7016 exhibited not only a tumor regression effect for over 20 days due to its bystander effect in tumor tissue after administration, but also showed different ADMEs with just a methyl group difference of one, resulting in a dose-dependent difference in anticancer efficacy (conc. vs. effect) and a difference in anti-cancer efficacy over time (effect vs. time) in targeted tumor tissue (Example 8, and FIGS. 23 and 24).

As the hydrophobicity of the linker and the drug increases, problems such as increased aggregation of ADCs and a reduced therapeutic coefficient of the drug caused thereby arise. Surprisingly, trastuzumab-7016 with only a methyl group difference of one from trastuzumab-7014 exhibited excellent dose-dependent anticancer efficacy (conc. vs. effect) and time-dependent anticancer efficacy (effect vs. time) despite increased hydrophobicity (Example 8, and FIGS. 23 and 24).

However, in some cases, the excellent anticancer effect over time may also trigger increased side effects (persistent T cell activation and inhibitory signal accumulation caused by chronic antigen exposure, T cell exhaustion caused thereby, ILD, and neutropenia). For example, depending on the drug modality (small molecule vs. ADC) of the active camptothecin derivative of Chemical Formula 1, exceeding a certain drug concentration (high) may be important, or in some cases, the time to maintain a certain drug concentration (low) may be important.

Accordingly, considering various side effects as well as problems such as a reduced therapeutic coefficient, another key feature of the present invention is that the selection range of an ADC payload exhibiting appropriate anticancer efficacy is expanded to a group of diverse candidates including the active camptothecin derivatives of Chemical Formula 1, which are designed to bind to the DDX5 protein and an E3 ligase as molecular glues.

Despite the remarkable success of ADCs utilizing a camptothecin-based payload, such as Enhertu or DS-1062a, as a multiple MoA payload candidate for improving safety profiles (minimizing interstitial lung disease (ILD) and neutropenia) and addressing cancer heterogeneity, various active camptothecin derivatives represented by Chemical Formula 1 designed to bind to the DDX5 protein and an E3 ligase according to the present invention are provided, and thus one or more appropriate drugs of the active camptothecin derivatives of Chemical Formula 1 may be selected to precisely control desired efficacy (e.g., anticancer and combination therapy) and side effects (carcinogenic inflammation, drug resistance, ILD, and neutropenia), appropriate linkers for the above derivatives may be selected, and appropriate doses and dosages may be applied.

**[Cancer cell death and immune responses caused thereby]**

One aspect of the present invention provides an anticancer formulation, which is administered in a dosage that an active camptothecin derivative represented by Chemical Formula 1 below designed to bind to the DDX5 protein and an E3 ligase; or a prodrug thereof designed to release the active camptothecin derivative at a target site *in vivo* stimulates antigen-presenting cells (APCs) through the cell death of cancer cells to induce an antitumor immune response.

Here, the antigen-presenting cells (APCs) may be dendritic cells and macrophages.

In addition, one aspect of the present invention provides a method of preparing an active camptothecin derivative (a)-based anticancer agent, in which the bystander effect and/or ADME profile of the active camptothecin derivative (a) are/is controlled to avoid T cell exhaustion due to chronic antigen exposure, wherein

the active camptothecin derivative (a)-based anticancer agent is an anticancer formulation, which contains (a) an active camptothecin derivative designed to bind to the DDX5 protein and an E3 ligase as a molecular glue; or (b) a prodrug thereof designed to release the active camptothecin derivative (a) at a target site *in vivo.*

The method includes designing the active camptothecin derivative (a) or the prodrug thereof (b) to contain the camptothecin-based backbone represented by Chemical Formula 1 as a parent nucleus, selecting a compound designed in this way, or synthesizing the compound designed in this way.

A signaling pathway that regulates cell death is a complex network of intracellular signaling pathways, which can be strictly regulated by various proteins and activated by various internal or external stimuli such as DNA damage, oxidative stress, and growth factor deficiency. The dysregulation of such a pathway may lead to a variety of diseases, including cancer and neurodegenerative disorders.

All major signaling pathways that regulate cell death converge on the activation caspases, a family of cysteine proteases, that cleave various cell substrates and ultimately cause cell death. Caspase activation may be strictly regulated by various proteins, including Bcl-2-series proteins, which can promote or inhibit apoptosis.

The Bcl-2 protein family includes both pro-apoptotic and anti-apoptotic members, which can promote and inhibit cell suicide, respectively. The balance between pro- and anti-apoptotic Bcl-2 family members is critical to determining cell fate and sensitivity to cell death.

Other signaling pathways, such as the p53 pathway and the PI3K/Akt pathway, may also regulate cell death. The p53 pathway is activated in response to DNA damage and leads to transcription of pro-apoptotic genes such as Bax and Puma. On the other hand, the PI3K/Akt pathway may inhibit apoptosis by phosphorylation and inactivation of pro-apoptotic proteins such as Bad and Bim.

Some types of necroptosis may be programmed cell responses to stimuli such as infection or DNA damage.

Immunogenic cell death involves changes in the cell surface, and the release of cancer antigens and damage-related molecular patterns during the process of cancer cells dying from damage. Released immune-inducing materials may stimulate immune cells, such as dendritic cells and macrophages, to induce anti-tumor immune responses.

Apoptosis is a type of cell death in which damaged or aged cells die on their own, which involves the modification and decomposition of various intracellular materials. During this process, immune-inducing materials such as cancer antigens and damage-associated molecular patterns (DAMPs), which induce *in vivo* immunity to cancer cells, may be damaged by proteolytic enzymes, oxidation, etc., and thus the immunotherapy effect on residual or metastatic cancer after treatment may be limited.

Cell death caused by reactive oxygen species generated from a chemotherapeutic is apoptosis mainly mediated by a caspase, which is a proteolytic enzyme.

Necroptosis, a type of necrosis that causes self-destruction by collapsing the cell membrane, may minimize damage to a cancer marker or immune-inducing material because protein degradation or oxidation does not occur, unlike apoptosis mediated by a degrading enzyme such as a caspase.

Damage-associated molecular patterns (DAMPs) are molecules that are released during cell death to induce immune responses in cells and stimulate immune cells. Representative examples of DAMPs include calreticulin, heat shock protein 70/90, high-mobility group box 1, and ATP.

Meanwhile, inflammation is the body's natural response to infection or injury, involving the release of inflammatory cytokines and the recruitment of immune cells to an affected area. However, when inflammation persists for a long

time, it can damage tissue and increase the risk of developing cancer.

In relation to cancer, T cell exhaustion may occur by chronic antigen exposure. Tumor cells express antigens that can be recognized by T cells, but continuous exposure to these antigens may lead to T cell exhaustion. This may weaken an anti-tumor response, resulting in the growth and spread of tumors.

Overall, the relationship between T cell exhaustion, chronic antigen exposure, and disease is complex and multifaceted.

With one methyl group difference from trastuzumab-7014, trastuzumab-7016 showed excellent dose-dependent anti-cancer efficacy and time-dependent anti-cancer efficacy despite increased hydrophobicity (Example 8, and FIGS. 23 and 24), but in terms of side effects such as T cell exhaustion, ILD, and/or neutropenia, treatment with a lower dose of trastuzumab-7016 may be required.

To properly use a drug, the properties of the drug should be studied. The pharmacodynamic and pharmacokinetic parameters of a drug are helpful in understanding its properties.

Pharmacodynamics explains the magnitude and patterns of changes (effects; cell viability, a clinical effect, a therapeutic action, a toxic effect, and an adverse effect), which occur in cells or the body after binding a drug to a receptor, in relation to drug concentration.

Pharmacokinetics (PK) shows how a drug concentration changes as a drug moves through different compartments of the body through ADME depending on the drug or drug modality. Example 8, Table 6, and FIGS. 23 and 24 may predict the drug concentration of the active camptothecin derivative (a) in tumor tissue over time in various drugs/modalities.

From a pharmacodynamic perspective, failures in the development of novel drugs may be due to accumulation of toxic drugs, and useful drugs may have no advantages because doses are too small to establish treatment, and the drug may be rapidly metabolized.

Therefore, in order to properly use a drug, an appropriate drug must be selected, and an appropriate dose-dosage of the drug must be applied.

A dose refers to the amount of drug administered at one time. A drug dose is generally prescribed by a healthcare provider based on factors such as a patient's age, body weight, and health condition. On the other hand, a dosage refers to the frequency and duration of drug administration. A total drug amount given over a certain period of time is generally expressed as a daily or weekly dose. A dose and a dosage are important considerations for the safe and effective use of drugs.

In this respect, the present invention may provide various active camptothecin derivatives of Chemical Formula 1, which are molecular glues and bind to the DDX5 protein and an E3 ligase, as various drug candidates, and thus desired efficacies (e.g., anticancer therapy and combination therapy) and side effects (chronic cancer antigen exposure, drug resistance, and ILD) are precisely controlled by selecting one or more appropriate drugs and properly applying the dose-dosage of the drug.

For example, for the active camptothecin derivatives of Chemical Formula 1, depending on drug modalities (small molecule, ADC), exceeding a certain drug concentration (high) in tumor tissue may be important, and in some cases, the time for which a certain drug concentration (low) is maintained in tumor tissue may be important.

**[Prodrug]**

In this specification, a prodrug becomes biologically active or more active after being placed in a predetermined physiological environment, but the present invention is not limited thereto.

For example, a prodrug refers to a compound that is chemically modified with a physiologically active material or a therapeutically active organic compound and designed to release or liberate a parent compound under enzymatic or other conditions *in vivo.* A prodrug is changed into a target compound *in vivo* after administration. Although it is a useful drug, it has unsuitable properties such as side effects, stability, solubility, absorbability, and reaction time, but it is available for clinical use by chemical modifications.

According to the present invention, an active camptothecin derivative represented by Chemical Formula 1 below designed to bind to the DDX5 protein and an E3 ligase, for example, a camptothecin-based drug of Chemical Formula 3, Chemical Formula 3-1, Chemical Formula 4, Chemical Formula 4-1, Chemical Formula 5, Chemical Formula 5-1, Chemical Formula 6, Chemical Formula 7, Chemical Formula 8, or Chemical Formula 9 can be used as a payload for various carrier-drug conjugates. That is, various carrier-drug conjugates to which an active camptothecin derivative is connected as a payload are prodrugs of the active camptothecin derivative, which releases the active camptothecin derivative *in vivo.* Here, a carrier may be an antibody, a peptide, a repebody, and/or an aptamer.

| | ADC | PDC | ApDC | Repebodv |
|---|---|---|---|---|
| Size | > 150 kDa | 2-5 kDa | > 20 kDa | 30-50 kDa |

(continued)

| | ADC | PDC | ApDC | Repebodv |
|---|---|---|---|---|
| Advantages | Next-generation anticancer agent that is gaining attention in the global market, as can simultaneously exhibit two functions, such as target selectivity of antibodies and cytotoxicity of a drug | High tissue permeability<br>Low immunogenicity<br>Possible to precisely regulate DAR<br>Capable of introducing modified amino acid such as non-natural amino acid | High tissue permeability<br>Low immunogenicity<br>Possible to precisely regulate DAR<br>Easy chemical transformation | Relatively low immunogenicity<br>Relatively easy regulation of DAR<br>High structural stability at high temperature and pH |
| Disadvantages | High immunogenicity<br>Difficult regulation of DAR<br>Low tissue permeability | Low in vivo stability | Low in vivo stability | Necessary for related studies and optimization techniques, as early step of development |

For example, an ADC is a new drug platform that selectively delivers a payload with strong anticancer efficacy only to cancer tissue by using the high tissue selectivity of antibodies that specifically bind to specific antigens expressed on the surface of cancer cells. ADCs can selectively deliver a strong payload that kills cancer cells only to cancer tissue even at a low (pM-level) concentration, and ensure both anticancer efficacy and safety by minimizing systemic drug exposure.

The intracellular introduction of most ADCs is performed through a clathrin-coated pit function. ADCs that have moved into cells are detached from clathrin and fused with other vesicles in the cells and then proceed through an endosome-lysosome pathway. Subsequently, proteases in the acidic environment of endosomes cleave a linker, an active "free" drug moves to the cytoplasm through the lysosomal membrane and then binds to a molecular target of the drug, thereby arresting the cell cycle of tumor cells and killing cancer cells through apoptosis. A certain portion of the drug is passively diffused or actively transported in the cells, or is released outside the cells via dead cells. Here, when the released drug has cell membrane permeability, it may enter surrounding cells and cause the so-called bystander cell-killing phenomenon.

In the case of HER2-positive cancer, the active camptothecin derivative (a) or its prodrug ADC according to the present invention, which targets the DDX5 protein, may be a new therapy that can resolve anti-HER2 treatment resistance.

The prodrug of the present invention may be an immunoconjugate including an antibody or an antigen binding site-containing fragment, which is designed to release the active camptothecin derivative (a) of the present invention *in vivo,* or a pharmaceutically acceptable salt thereof.

The immunoconjugate of the present invention may be useful for the treatment or prevention of cancer because it specifically binds to an antigen of cancer cells and releases the drug within/outside cancer cells, thereby exhibiting cytotoxicity.

An aptamer-drug conjugate (ApDC) has an aptamer, instead of the antibody of the ADC. An aptamer is a single-stranded nucleic acid with a 3-dimensional structure. It is discovered through the Systematic Evolution of Ligands by Exponential Enrichment (SELEX) process. SELEX is a technology that obtains a functional nucleic acid that binds to target protein molecules in a compound library

An aptamer is also called a chemical antibody because it binds very strongly and selectively to a target. An aptamer has a size of about 20 kDa, and is known to have better cell penetration and lower immunogenicity than an antibody.

Since an aptamer can be chemically synthesized, precise design of the conjugation location and number of drug conjugates is possible in the preparation of ApDCs. ApDCs have lower production costs than ADCs.

An aptamer is generally composed of a natural nucleic acid, so it is degraded by a nuclease in the body, which decreases its *in vivo* stability. However, limitations in the stability of modified aptamers can be overcome by taking full advantage of easy chemical modification of the aptamers.

A peptide-drug conjugate (PDC) is a form of an ADC in which a peptide is introduced instead of an antibody. A

peptide consists of amino acids, and has a size ranging from 500 to 5000 daltons (Da). This is a very small size compared with antibodies of 150 kilodaltons (kDa) or more. Accordingly, a peptide-based PDC has excellent cell penetration ability compared to ADCs, and a very low possibility of developing immunogenicity. In addition, peptides can be chemically synthesized. For this reason, a PDC may not only have a very low production cost, but also allows precise regulation of the conjugation location and ratio of the peptide and the drug.

Generally, a peptide is easily degraded by a proteolytic enzyme and thus has a short biological half-life. To overcome this limitation of the peptide-based drug conjugate, strategies that utilize modified peptides, such as the introduction of a cyclic peptide and non-natural amino acid, are being proposed.

A repebody is a type of artificial antibody that does not have an antibody skeleton but has the function of recognizing antigens, like an antibody. A repebody specific for a target protein may be discovered using phage display technology.

Phage display is a technique by which a desired protein is expressed on the surface of a bacteriophage. A repebody is about 30 kDa in size, which is about 20% of an antibody drug. Therefore, repebodies are known to have relatively low immunogenicity and improved cell penetration compared to antibodies. In addition, it is expected that the thermal pH stability of a repebody can be regulated to increase structural stability. Compared to antibodies, repebodies are also considered to have relatively low production costs. Due to these advantages of repebodies, interest in the development of a repebody-drug conjugate (repebody-DC) as a strategy to replace an antibody with a repebody is also increasing.

**[Pharmaceutical composition for preventing or treating cancer]**

The present invention provides a pharmaceutical composition for preventing or treating cancer, which includes the above-described compound represented by Chemical Formula 1, for example, Chemical Formula 3, Chemical Formula 3-1, Chemical Formula 4, Chemical Formula 4-1, Chemical Formula 5, Chemical Formula 5-1, Chemical Formula 6, Chemical Formula 7, Chemical Formula 8, or Chemical Formula 9, a pharmaceutically acceptable salt thereof, a solvate thereof, or a prodrug thereof as an active ingredient.

The active camptothecin derivative (a) represented by Chemical Formula 1 or a prodrug thereof (b) according to the present invention may be included as an active ingredient in a pharmaceutical composition to be administered to a patient group in which DDX5 acts as an oncoprotein in cells.

For example, in tissue biopsy or liquid biopsy, a patient group to be administered the active camptothecin derivative (a) or a prodrug thereof (b) may be confirmed by quantitative or qualitative analysis of the DDX5 protein in cells using a ligand-containing complex targeting the DDX5 protein, which inactivates topoisomerase-1 inhibitory ability of the FL118 compound of Chemical Formula 2 or the active camptothecin derivative (a), preferably through non-cleavable linker conjugation.

The active camptothecin derivative (a) represented by Chemical Formula 1 according to the present invention, for example, the compound of Chemical Formula 3, Chemical Formula 3-1, Chemical Formula 4, Chemical Formula 4-1, Chemical Formula 5, Chemical Formula 5-1, Chemical Formula 6, Chemical Formula 7, Chemical Formula 8, or Chemical Formula 9 is a camptothecin derivative with a novel structure. Therefore, according to this, it is possible to provide a drug candidate material having more preferable properties than the Dxd drug in terms of efficacy, toxicity, selectivity, reaction time, administration, handling, stability and/or production potential. In addition, it is possible to optimize the interaction between a target and a drug candidate material, that is, pharmacodynamic properties, and to improve the pharmacokinetic ability of optimizing the approach of the drug toward the target.

In addition, in one embodiment of the present invention, a method of treating or preventing cancer is provided, which includes administering a therapeutically effective amount of the compound of Chemical Formula 1, for example, Chemical Formula 3, Chemical Formula 3-1, Chemical Formula 4, Chemical Formula 4-1, Chemical Formula 5, Chemical Formula 5-1, Chemical Formula 6, Chemical Formula 7, Chemical Formula 8, or Chemical Formula 9, a pharmaceutically acceptable salt thereof, a solvate thereof, or a prodrug thereof to a subject in need thereof. The subject may be a mammal, such as a human.

In the present invention, the cancer may include all types of cancer, which can be treated by the inhibition of topoisomerase-1, and/or the inhibition of any one or more cancer-associated survival genes selected from the group consisting of DDX5, survivin, Mcl-1, XIAP, and cIAP2, and may be solid cancer or blood cancer. For example, the cancer may be one or more selected from the group consisting of pseudomyxoma, intrahepatic cholangiocarcinoma, hepatoblastoma, liver cancer, thyroid cancer, colon cancer, testicular cancer, myelodysplastic syndrome, glioblastoma, oral cancer, lip cancer, mycosis fungiodes, acute myeloid leukemia, acute lymphoblastic leukemia, basal cell carcinoma, ovarian epithelial carcinoma, ovarian germ cell carcinoma, male breast cancer, brain cancer, pituitary adenoma, multiple myeloma, gallbladder cancer, bile duct cancer, colorectal cancer, chronic myelogenous leukemia, chronic lymphocytic leukemia, retinoblastoma, choroidal melanoma, ampulla of Vater cancer, bladder cancer, peritoneal cancer, parathyroid cancer, adrenal cancer, paranasal sinus cancer, non-small cell lung cancer, tongue cancer, astrocytoma, small cell lung

cancer, pediatric brain cancer, pediatric lymphoma, childhood leukemia, childhood leukemia, small bowel cancer, meningioma, esophageal cancer, glioma, renal pelvis cancer, kidney cancer, heart cancer, duodenal cancer, malignant soft tissue cancer, malignant bone cancer, malignant lymphoma, malignant mesothelioma, malignant melanoma, eye cancer, vulvar cancer, ureteral cancer, urethral cancer, unknown primary neoplasm, lymphoma, gastric cancer, gastric carcinoma, gastrointestinal stromal cancer, Wilms' cancer, breast cancer, sarcoma, penile cancer, pharyngeal cancer, gestational trophoblastic disease, cervical cancer, endometrial cancer, uterine sarcoma, prostate cancer, metastatic bone cancer, metastatic brain cancer, mediastinal cancer, rectal cancer, occipital carcinoid tumors, vaginal cancer, spinal cancer, vestibular schwannoma, pancreatic cancer, salivary gland cancer, Kaposi's sarcoma, Paget's disease, tonsil cancer, squamous cell carcinoma, lung adenocarcinoma, lung cancer, squamous cell lung cancer, skin cancer, anal cancer, rhabdomyosarcoma, laryngeal cancer, pleural cancer, blood cancer, and thymic carcinoma, but the present invention is not limited thereto. In addition, the caner includes not only primary cancer but also metastatic cancer.

In this specification, "patient," "individual," and "subject" refer to animals such as mammals. In a specific embodiment, the patient is a human. In another embodiment, the patient is an animal, rather than a human, such as a dog, a cat, a domestic animal (e.g., a horse, a pig, or a donkey), a chimpanzee, or a monkey.

The term "therapeutically effective amount" used herein is an amount of the compound represented by Chemical Formula 3, Chemical Formula 3-1, Chemical Formula 4, Chemical Formula 4-1, Chemical Formula 5, or Chemical Formula 5-1, a pharmaceutically acceptable salt thereof, a solvate thereof, or a prodrug thereof, which is effective in treatment or prevention of cancer. Specifically, the "therapeutically effective amount" refers to an amount sufficient for treating a disease at a reasonable benefit/risk ratio applicable for medical treatment, and an effective dose may be determined by parameters including the type of a subject, the severity of a disease, the subject's age and sex, the type of a disease, drug activity, the sensitivity to a drug, administration time, an administration route and an excretion rate, the duration of treatment and drugs simultaneously used, and other parameters well known in the medical field. The pharmaceutical composition of the present invention may be administered separately or in combination with other therapeutic agents, and may be sequentially or simultaneously administered with a conventional therapeutic agent, or administered in a single or multiple dose(s). In consideration of all of the above-mentioned parameters, it is important to achieve the maximum effect with the minimum dose without side effects. As the compound represented by Chemical Formula 3, Chemical Formula 3-1, Chemical Formula 4, Chemical Formula 4-1, Chemical Formula 5, Chemical Formula 5-1, Chemical Formula 6, Chemical Formula 7, Chemical Formula 8, or Chemical Formula 9, and a pharmaceutically acceptable salt thereof exhibits a dose-dependent effect, the administration dose may be easily determined by various factors such as a patient's condition, age, sex, and complications. The active ingredient of the pharmaceutical composition of the present invention has excellent safety, so it may be used at a dose higher than the determined administration dose.

In addition, according to one embodiment of the present invention, the present invention provides a use of the compound of Chemical Formula 3, Chemical Formula 3-1, Chemical Formula 4, Chemical Formula 4-1, Chemical Formula 5, Chemical Formula 5-1, Chemical Formula 6, Chemical Formula 7, Chemical Formula 8, or Chemical Formula 9, a pharmaceutically acceptable salt thereof, a solvate thereof, or a prodrug thereof for preparing a medicament to be used in treatment or prevention of cancer. The compound of Chemical Formula 3, Chemical Formula 3-1, Chemical Formula 4, Chemical Formula 4-1, Chemical Formula 5, Chemical Formula 5-1, Chemical Formula 6, Chemical Formula 7, Chemical Formula 8, or Chemical Formula 9, a pharmaceutically acceptable salt thereof, a solvate thereof, or a prodrug thereof for preparing a medicament may be mixed with acceptable additive, diluent and carrier, and may be prepared into a composite formulation together with other active agents to exhibit a synergistic effect of the active ingredients.

The details on the use, composition, and treatment method of the present invention are equally applied unless they are contradictory.

In this specification, the anticancer effect or therapeutic effect of an anticancer agent may refer to an action that reduces the severity of cancer, reduces a tumor size, or delays or slows the progression of cancer in a patient who is suffering from a specific cancer.

For example, the anticancer effect of an anticancer agent may be cell viability (the degree of cytotoxicity or the change in the number of cells) of cancer cells after the cancer cells are treated with the anticancer agent *in vitro* and/or *in vivo.* For example, the anticancer effect may be indirectly confirmed through a drug response test using a cell line or a non-clinical animal model (xenograft). In addition, the anticancer effect of an anticancer agent may be directly confirmed in cancer patients, and relevant data can be derived and used as a database. In addition, when designing the dosing guidelines for an anticancer agent, animal model PK parameters and/or toxicity profiles may be considered in parallel.

The anticancer effect of an anticancer agent may be inferred from *in vitro* data such as the % maximum effect of the corresponding anticancer agent *in vitro,* for example, $IC_{50}$, $IC_{60}$, $IC_{70}$, $IC_{80}$, and $IC_{90}$, and may be confirmed in non-clinical animal models and clinical cancer patients through in-vivo data, such as the maximum blood concentration (Cmax), and/or the area under the blood drug concentration-time curve (AUC).

The reactivity of an anticancer agent refers to the clinical sensitivity in terms of the anticancer effect.

When referring to treatment with an anticancer agent, "sensitivity" and "sensitive" are relative terms referring to the degree of the effect of a compound in alleviating or reducing the progression of a tumor or disease to be treated.

"Effective anticancer effect/response in patients" may be, for example, 5%, 10%, 15%, 20%, 25%, 30%, 40%, 50%, 60%, 70%, 80%, 90%, 100%, 200% or more inhibition in patient responses, as measured by any suitable means, such as gene expression, cell counts, analysis results, etc.

In this specification, the administration dose is an amount at which drug efficacy is expected. The efficacy in the present invention may be an anticancer effect. The reactivity (anticancer effect) of an anticancer agent is the degree of response and may be the % maximum effect of the corresponding anticancer agent, for example, $C_{50}$, $IC_{60}$, $IC_{70}$, $IC_{80}$, and $IC_{90}$, or the value ($LC_{50}$) of toxicity to normal cells.

For example, an oral formulation may be prepared using various formation techniques known in the art. For example, the oral formulation may include a biodegradable (hydrolyzable) polymeric carrier that is intended to adhere to the oral mucosa. It is designed to dissolve gradually over a predetermined period of time, and here, drug delivery is essentially provided as a whole.

Drug delivery in an oral formulation avoids disadvantages accompanying oral drug administration, for example, slow absorption, the degradation of an active agent by a fluid present in the gastrointestinal tract, and/or first-pass inactivation in the liver. For a biodegradable (hydrolyzable) polymeric carrier, virtually any such carrier may be used as long as the desired drug release profile is not impaired, and the carrier is compatible with any other ingredient present in an oral dosage unit. Generally, polymeric carriers include hydrophilic (water-soluble and water-swellable) polymers that adhere to the wet surface of the oral mucosa. Examples of the polymeric carriers useful herein include acrylic acid polymers (e.g., a carbomer). In some embodiments, non-limiting examples of other ingredients that may be incorporated into an oral formulation include a disintegrant, a diluent, a binder, a lubricant, a flavoring agent, a coloring agent, and a preservative. In some embodiments, for oral or sublingual administration, carriers may be in the form of a conventionally formulated tablet, lozenge, or gel. In some embodiments, when the conditions of the patient improve, the administration of the compound continues to be provided at the physician's discretion; alternatively, the dose of a drug to be administered may be temporarily reduced or stopped for certain length of time (i.e., "drug-free interval"). The length of drug-free interval may vary from two days to one year, for example, 2, 3, 4, 5, 6, 7, 10, 12, 15, 20, 28, 35, 50, 70, 100, 120, 150, 180 일, 200, 250, 280, 300, 320, 350, or 365 days. In some embodiments, the dose reduction during the drug-free interval may vary from 10% to 100%, for example, 10%, 15%, 20%, 25%, 30%, 35%, 40%, 45%, 50%, 55%, 60%, 65%, 70%, 75%, 80%, 85%, 90%, 95%, or 100%.

When the improvement in a patient's condition occurs, a maintenance dose is provided if necessary. Subsequently, the dose or administration frequency, or both may be reduced as a function of symptom to a level at which an improved disease, disorder, or condition is maintained. However, patients require intermittent treatment over a long period of time when any symptom recurs.

The amount of the given formulation corresponding to the above-mentioned dose may vary depending on factors of a subject to be treated, such as a specific compound, the severity of a disease, and identity (e.g., body weight), but can nevertheless be generally determined by, for example, a formulation to be administered, an administration route, and specific circumstances around the subject to be treated. Generally, however, a dose used in treatment of adult humans may typically range from 0.02 to 5000 mg/day, or about 1-1500 mg/day.

In this specification, the dose may be provided as a single dose, or in divided doses administered simultaneously, for example, 2, 3, 4 or more sub-doses.

In some embodiments, the oral formulation is a unit dosage form suitable for a precise single administration dose. In the unit dosage form, the formulation is divided into unit doses containing an appropriate amount of one or more compounds. In some embodiments, a unit dose is in the form of a package containing a discrete quantity of formulation. Non-limiting examples include packaged tablets or capsules, powder vials or ampoules. An aqueous suspension composition may be packaged in a single-dose non-reclosable container. Alternatively, a multi-dose reclosable container may be used, and in this case, it is typical to include a preservative in the composition.

In some embodiments, a parenteral injectable formulation is provided in a unit dosage form or multi-dose container including, but not limited to, ampoules along with an added preservative.

Typically, the parenteral injectable formulation is prepared in a unit dose injectable form together with a pharmaceutically acceptable parenteral vehicle for parenteral administration, that is, a bolus, intravenous and intratumoral injection. It is optionally mixed in the form of a lyophilized preparation or a water-soluble solution with pharmaceutically acceptable diluents, carriers, excipients, or stabilizers (Remington's Pharmaceutical Sciences (1980) 16th edition, Osol, A. Ed.).

**[Ligand that targets DDX5 protein]**

Since an active camptothecin derivative represented by Chemical Formula 1 according to the present invention, as well as FL18, is a molecular glue activating the oncoprotein DDX5 degradation, it may be used as a ligand targeting the DDX5 protein or a ligand binding to the DDX5 protein. Therefore, a ligand-containing complex (c) targeting the DDX5 protein, in which the topoisomerase-1 inhibitory ability of the FL118 compound or the active camptothecin derivative (a) is

inactivated by linker conjugation, may be provided.

The linker may be a non-cleavable linker, and a non-limiting example of the non-cleavable linker is a thioether linker.

The FL118 compound or the active camptothecin derivative (a) represented by Chemical Formula 1 binds to the DDX5 protein, and the DDX5 protein is a direct target of the active camptothecin derivative (a).

Accordingly, DDX5 may be used as a biomarker for predicting drug sensitivity or tumor sensitivity.

In tissue biopsy or liquid biopsy, a patient group to be administered the active camptothecin derivative (a) or a prodrug thereof (b) may be determined, or a relapse rate and/or prognosis during chemotherapy may be predicted through quantitative or qualitative analysis of the DDX5 protein in cells using the ligand-containing complex (c) targeting the DDX5 protein, in which the topoisomerase-1 inhibitory ability of the FL118 compound of Chemical Formula 2 or the active camptothecin derivative (a) represented by Chemical Formula 1 is inactivated through linker conjugation.

**[Cancer diagnosis composition]**

The present invention provides a cancer diagnosis composition containing a ligand-containing complex (c) that targets the DDX5 protein, in which the topoisomerase-1 inhibitory ability of the FL118 compound of Chemical Formula 2 or the active camptothecin derivative (a) represented by Chemical Formula 1 is inactivated by linker conjugation.

The cancer diagnosis composition according to the present invention may be used to determine a patient group to be administered the active camptothecin derivative (a) or a prodrug thereof (b), or to predict a drug response, a relapse rate and/or prognosis during chemotherapy.

[Advantageous Effects]

According to the present invention, a camptothecin derivative represented by Chemical Formula 1, for example, any one of Chemical Formula 3 to Chemical Formula 9, or an isomer thereof is a compound with a novel structure. Accordingly, a drug candidate material having more preferable properties than the Dxd drug in terms of efficacy, toxicity, selectivity, reaction time, administration, handling, stability and/or production potential can be provided. In addition, it is possible to optimize the interaction between a target and a drug candidate material, that is, pharmacodynamic properties, and to improve the pharmacokinetic ability of optimizing the approach of the drug toward the target.

The present invention provides a compound of Chemical Formula 1, for example, a compound of any one of Chemical Formula 3 to Chemical Formula 9, or an isomer thereof, which has the structure of the FL118 drug maintaining the advantages of the FL118 drug that can maximize the efficacy of the main drug target modulation by an additional MoA of (i) simultaneously inhibiting the Bcl family such as a resistance protein survivin and/or (ii) inhibiting the action of an efflux pump.

Therefore, the compound of Chemical Formula 1, for example, the compound of any one of Chemical Formula 3 to Chemical Formula 9, or an isomer thereof is, like the FL118 drug, is designed based on a well-designed polypharmacology-based approach obtaining excellent treatment efficacy by simultaneously targeting several drug targets requiring control, rather than an existing drug development method that limits therapeutic efficacy by targeting one drug target with one drug, and can treat intractable diseases for which existing treatments have not shown apparent therapeutic effects. In addition, recurrent/resistant cancer cannot achieve sufficient efficacy only by controlling the activity of one drug target, and there is a problem that safety cannot be guaranteed when simultaneously targeting multiple unspecified drug targets, but like the FL118 drug, a synergistic effect in terms of drug efficacy can be achieved using two or more well-selected drug targets and sufficient safety can be secured.

[Description of Drawings]

FIG. 1 shows the synthetic design concept of a novel active camptothecin derivative (a) having a dual mechanism of action (MoA) that inhibits topoisomerase-1 and degrades the oncoprotein DDX5 through an improved FL118 structure.

FIG. 2 illustrates the structural formula of camptothecin (CPT) and its binding with topoisomerase-1, which expands the molecular design concept of FL118 and PBX-7011 from camptothecin, the derivation of compounds PBX-7014 and PBX-7016 having novel structures expanded from PBX-7011, and the results of calculating their hydrophilicity.

FIG. 3 illustrates the synthesis methods of 25-4 and 25-6 from PBX-7014 and PBX-7016, similar to the process of synthesizing deruxtecan from Dxd.

FIG. 4 shows the molecular dynamics simulation and docking score that analyzes the interaction between an existing camptothecin-based drug and TOP1.

FIG. 5 shows the molecular dynamics simulation and docking score that analyzes the interaction between a novel camptothecin-based drug of the present invention and TOP1.

FIG. 6 shows the molecular dynamics simulation that analyzes the interaction between camptothecin and mutant TOP1 (E418K).

FIG. 7 shows the SN38 - TOP1 interaction in normal TOP1.

FIG. 8 shows the SN38 - TOP1 interaction in E418K mutated TOP1.

FIG. 9 shows the compound of Chemical Formula 1-1 (compound A) - TOP1 interaction in E418K mutated TOP 1.

FIGS. 10 to 14 show the Western blot results that show the degrees of inhibition of anti-apoptotic proteins by various camptothecin-based drugs (FL118 drug, SN-38 drug, exatecan drug, PBX-7011, PBX-7014, and PBX-7016) in a FaDu cell line and an A549 cell line to verify a dual MoA (topoisomerase I inhibition & DDX5 degradation) according to Example 2-1. FIGS. 12 and 14 are graphic representations of the concentrations in the results of Western blot experiments (FIGS. 11 and 13) performed on the FaDu cell line and the A549 cell line.

FIG. 15 shows the results of *in vitro* cell viability comparison evaluation for various camptothecin-based drugs in a FaDu cell line or A549 cell line.

FIG. 16 shows the results of *in vitro* cell viability comparison evaluation for various camptothecin-based drugs in an MDA-MB-453(HER2++) cell line and a FaDu (HER2+) cell line.

FIG. 17 shows the results of carboxylate to lactone form conversion at pH 6.0.

FIG. 18 shows the results of lactone to carboxylate form conversion at pH 7.4.

FIGS. 19 and 20 show the results of confirming ADC preparation using SEC and SDS PAGE.

FIG. 21 shows the results of comparing the binding strength of trastuzumab, a trastuzumab- and PBX-7014-conjugated ADC, and a trastuzumab- and PBX-7016-conjugated ADC to Her2.

FIG. 22 shows the results of *in vitro* cell viability comparison evaluation for various camptothecin-based drugs and ADCs using them as payloads in an MDA-MB-453 (HER2++) cell line, a FaDu (HER2+) cell line, and an MDA-MB-468(HER2-) cell line.

FIG. 23 shows the results of the efficacy test for ADCs using various camptothecin-based drugs as payloads in *in vivo* xenograft mouse models using a JIMT-1 cell line.

FIG. 24 shows the results of changes in mouse body weights by ADCs using various camptothecin-based drugs as payloads in *in vivo* xenograft mouse models using a JIMT-1 cell line.

FIG. 25 shows the results of counting cell numbers through FACS based on the presence or absence of GFP expression in ADCs using camptothecin-based drugs as payloads after co-culturing an MDA-MB-453(HER2++) cell line and a GFP-expressing MDA-MB-468(HER2-) cell line.

FIG. 26 shows the results of counting cell numbers through FACS using a HER2-FITC antibody based on the presence or absence of HER2 expression in ADCs using camptothecin-based drugs as payloads after co-culturing an MDA-MB-453(HER2++) cell line and an MDA-MB-468(HER2-) cell line.

FIG. 27 shows the comparison of the LogPs, cLogPs, and topological polar surface areas (tPSAs) of FL118, exatecan, SN-38, Dxd, compound A (PBX-7011), compound B (PBX-7012), compound C (PBX-7014), compound D (PBX-7015), compound E (PBX-7016), and compound F (PBX-7017).

FIG. 28 shows the comparison of the LogPs, cLogPs, and tPSAs of various camptothecin-based drugs, compound G, compound H, compound I, compound J, compound G', compound H', compound I', compound J' , which are derived from Chemical Formula 1.

FIG. 29 is a detailed classification diagram of cell death related to programmed cell death.

[Modes of the Invention]

Hereinafter, the present invention will be described in further detail with reference to examples. However, the following examples are intended only to clearly illustrate the technical features of the present invention and do not limit the scope of the present invention.

**Preparation Example 1: Synthesis of active camptothecin derivatives of Chemical Formulas 3 and 3-1 (PBX-7011 and PBX-7012)**

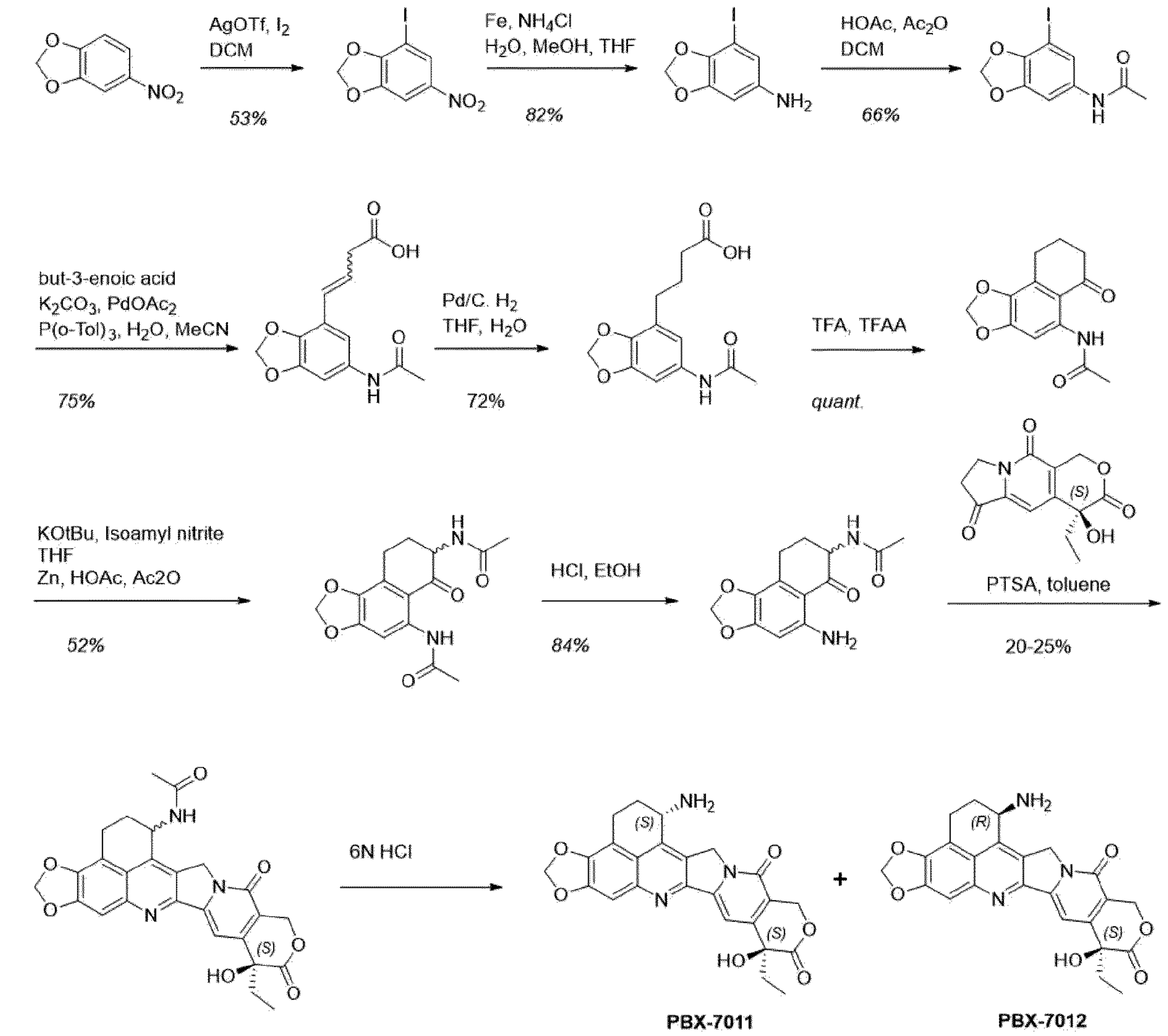


PBX-7011 PBX-7012

### 1-1. Synthesis of Compound 2

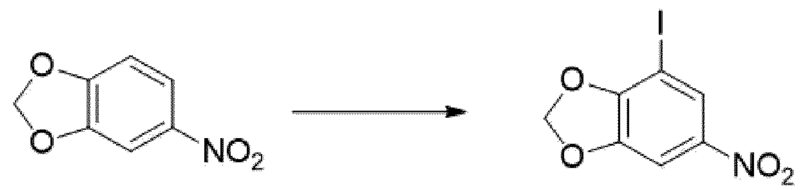

1 2

Silver triflate (57.7 g, 224 mmol) and iodine (57.0g, 224 mmol) were added to a 5-nitrobenzo[d][1,3]dioxol (25g, 150 mmol) solution in a dichloromethane (748 mL) flask. The solution was stirred in a room-temperature dark room under an $N_2$ atmosphere.

AgI was removed by filtration and the solid was washed with dichloromethane (100 mL). The solvent was removed under reduced pressure and the residue was distributed between EtOAc (250 mL) and a 5%(v/v) $NH_4OH/H_2O$ solution (200 mL). An organic layer was separated, washed with 1M $Na_2SO_3$ (5 x 200 mL) and brine (200 mL), dried with $Na_2SO_4$, treated with activated carbon, and filtered through Celite. The solvent was evaporated under reduced pressure to obtain a crude product as a brown solid. This was triturated in ice-cold EtOH (400 mL) and filtered, and the solid was washed with ice-cold EtOH (100 mL) to obtain a product as a pale brown-gray solid (14.3 g). The solvent was removed from

the filtrate, and the crude product was triturated again in ice-cold EtOH (300 mL). The solid was collected by filtration and washed with EtOH (50 mL) to obtain an additional amount of product (10.2 g) as a dark brown-gray solid. Both batches were used as is without additional purification.
SC_ACID: m/z 294.2 [M+H]$^+$

**1-2. Synthesis of Compound 3**

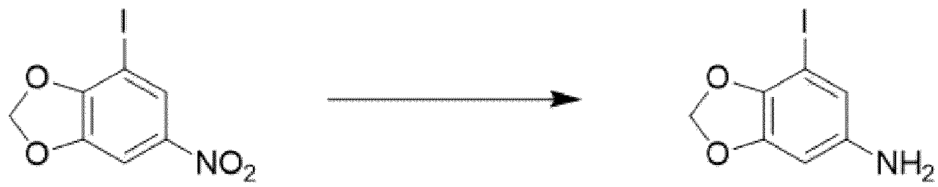

2 3

Iron powder (6.67 g, 119 mmol) and ammonium chloride (6.39 g, 119 mmol) were added to a suspension of 4-iodo-6-nitrobenzo[d][1,3]dioxol (8.75g, 29.9 mmol) in a mixture of water (80 mL), methanol (40.0 mL) and tetrahydrofuran (40.0 mL). The suspension was heated to 75 °C and stirred for 16.5 hours. The reaction mixture was cooled to room temperature and concentrated under reduced pressure. The resulting black solid was suspended in ethyl acetate (100 mL) and the solution was filtered. The residue was washed with EtOAc (3 x 50 mL). The mixture was transferred to a separatory funnel, water was added, and the aqueous layer was removed. The organic layer was washed with a saturated aqueous $NaHCO_3$ solution (150 mL) and brine (150 mL). The organic layer was dried over $Na_2SO_4$, and the solvent was removed under reduced pressure to obtain a brown solid (4.75g, 60% yield). An additional product was recovered by thoroughly washing the iron residue with ethyl acetate. Subsequently, the organic fraction was washed with a saturated aqueous $NaHCO_3$ solution (150 mL) and brine (150 mL), and dried over $Na_2SO_4$. The solvent was removed under reduced pressure to obtain a brown solid (1.74g, 22% yield).
SC_ACID: m/z 264.0 [M+H]$^+$

**1-3. Synthesis of Compound 4**

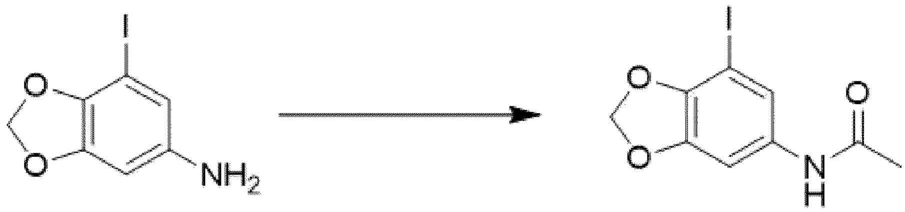

3 4

Acetic anhydride (2.7 5mL, 29.2 mmol) and triethylamine (4.06 mL, 29.2 mmol) were added to a solution of 7-iodobenzo[d][1,3]dioxol-5-amine (6.39 g, 24.29 mmol) in dichloromethane (49 mL). The reaction mixture was stirred at room temperature overnight. After several hours, additional DCM (10 mL) was added. The suspension was filtered through a sintered funnel and washed with ice-cold DCM (10 mL). The product was additionally dried under reduced pressure to obtain a light gray solid (4.46 g). The filtrate was concentrated under reduced pressure, the residue was dissolved in EtOAc, washed with water and brine, dried over $Na_2SO_4$ and concentrated under reduced pressure to obtain a brown solid (~3 g). The brown solid was purified by flash column chromatography (80 g Si, 0-100% ethyl acetate in heptane). All batches of product were triturated with ice-cold EtOAc (5-10 mL). Two batches were combined and further dried, thereby obtain an off-white solid.

Total yield: 5.0 g, 66%
SC_ACID: m/z 306.0 [M+H]$^+$

$^1$H NMR (400 MHz, DMSO-d6) δ 9.88 (s, 1H), 7.39 (d, *J* = 1.9 Hz, 1H), 7.17 (d, *J* = 1.9 Hz, 1H), 6.04 (s, 2H), 1.99 (s, 3H).

**1-4. Synthesis of Compound 5**

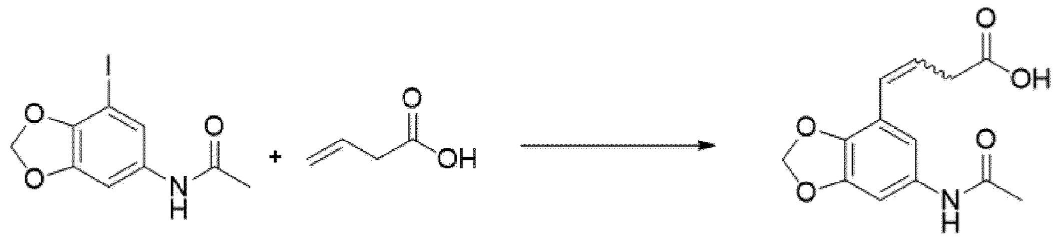

4 5

A slurry of N-(7-iodobenzo[d][1,3]dioxol-5-yl)acetamide (5.06 g, 16.59 mmol), but-3-enoic acid (1.69 mL, 19.90 mmol) and potassium carbonate (2.98 g, 21.56 mmol) in acetonitrile (40 mL) were cooled to 0 to 5 °C in a condenser-equipped 3-neck flask. Water (13.33 mL) was slowly added to generate gas. When gas generation stopped, the mixture was degassed with Ar for 30 minutes. Tri-o-tolylphosphine (0.505 g, 1.659 mmol) and palladium acetate (0.186 g, 0.829 mmol) were added, the mixture was degassed again for 30 minutes and then heated to reflux under Ar. The reaction product was cooled to room temperature and filtered through Celite. The filter cake was washed with $H_2O$ and EtOAc. The organic solvent was removed from the filtrate under vacuum and the aqueous material was acidified with concentrated HCl until pH = 1-2. The aqueous layer was extracted with EtOAc, and the combined organic phases were washed with brine, dried over $Na_2SO_4$, filtered, and concentrated under reduced pressure to obtain a crude product. The crude product was triturated in ice-cold EtOAc (30 mL) and the solid was collected by filtration to obtain a product as a brown solid. The mother liquid was concentrated under reduced pressure and purified through flash chromatography (80 g Si, 0 - 100% EtOAc in heptane). The product fraction was concentrated under reduced pressure to obtain a light brown foam. Total yield: 3.46g, 75%. A mixture of E/Z isomers was obtained.
SC_ACID: m/z 264.4 $[M+H]^+$

**1-5. Synthesis of Compound 6**

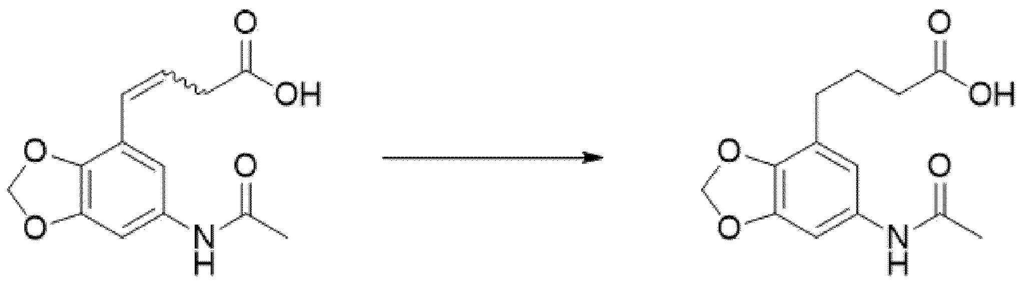

5 6

A suspension of 4-(6-acetamidobenzo [d][1,3]dioxol-4-yl)but-3-enoic acid (3.47 g, 13.18 mmol) in tetrahydrofuran (50 mL)/water (50 mL) was degassed with $N_2$ for 15 minutes. Before heating to 40 °C under $H_2$ (balloon), Pd/C (2.97 g, 1.397 mmol) was added and the suspension was degassed with $H_2$ for 5 minutes. After 24 hours, nitrogen and additional Pd/C (2.97 g, 1.397 mmol) were added to the reaction mixture. The reaction mixture was bubbled with hydrogen for 10 minutes and stirred overnight at 45 °C under hydrogen. The reaction product was filtered through Celite. A filter cake was washed with $H_2O$ (50 mL), and EtOAc (50 mL) and the organic solvent were removed from the filtrate under vacuum. The aqueous solution was acidified with concentrated HCl until pH = 1, and the dark brown/green precipitate was collected by filtration through a sintered funnel. The aqueous filtrate was extracted. The combined organic phases were washed with brine, dried over $Na_2SO_4$, and filtered, and the solvent was removed under vacuum to obtain a brown oil. Since a product

recovery rate was low, a filter cake was washed with EtOAc (50 mL), water (200 mL), and/or NaOH (400 mL). The water/EtOAc flush was added to a flask containing the solid from an MeOH flush. The aqueous layer was acidified to a thick concentration. Until pH = 1, HCl and an off-gray precipitate were generated, which were collected by filtration through a sintered funnel. After extracting the aqueous filtrate with EtOAc (3x200 mL), LCMS showed that all products were removed from the aqueous phase. The combined organic phases were washed with brine, dried over $Na_2SO_4$, and filtered, and the solvent was removed under vacuum to obtain a light brown solid. All product batches were combined and purified through flash column chromatography (40g Si, 0-10% MeOH in DCM). The product fractions were concentrated to obtain a light brown solid. Yield: 2.52g, 72%.

SC_ACID: 266.2 $[M+H]^+$
$^1$H NMR (400 MHz, DMSO-d6) δ 12.07 (s, 1H), 9.78 (s, 1H), 7.14 (d, *J* = 2.0 Hz, 1H), 6.80 (d, *J* = 2.2 Hz, 1H), 5.95 (s, 2H), 2.50 - 2.46 (m, 2H), 2.23 (t, *J* = 7.4 Hz, 2H), 1.98 (s, 3H), 1.84 - 1.70 (m, 2H).

**1-6. Synthesis of Compound 7**

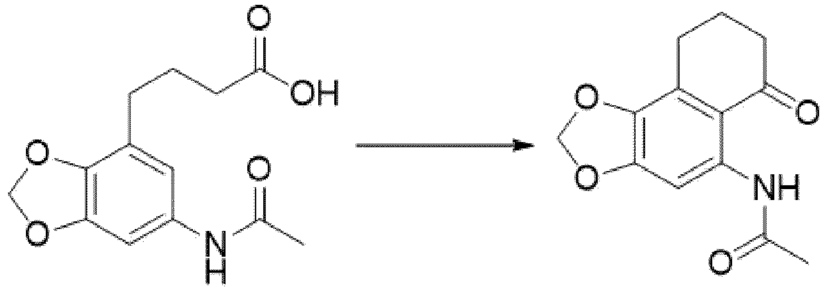

6 7

A suspension of 4-(6-acetamidobenzo[d][1,3]dioxol-4-yl)butanoic acid (150 mg, 0.565 mmol) in TFA(433 μL, 5.65 mmol) was cooled on ice. TFAA(157 μL, 1.131 mmol) was added. The mixture was stirred at 0-5 °C for 1 hour, and over time it turned into a dark solution. The reaction mixture was added dropwise to an ice-cold saturated aqueous $NaHCO_3$ solution (10 mL), and the aqueous solution was extracted with ethyl acetate (3 x 25 mL). The combined organic layers were dried over sat. $NaHCO_3$, the brine was dried over $Na_2SO_4$ and then filtered, and the solvent was removed under vacuum to obtain a salmon pink solid. Yield: 140 mg, 100%

SC_ACID: m/z 248.2 $[M+H]^+$
$^1$H NMR (400 MHz, DMSO-d6) δ 12.34 (s, 1H), 8.11 (s, 1H), 6.13 (s, 2H), 2.80 (t, *J* = 6.2 Hz, 2H), 2.66 - 2.58 (m, 2H), 2.12 (s, 3H), 2.01 - 1.91 (m, 2H).

**1-7. Synthesis of Compound 8**

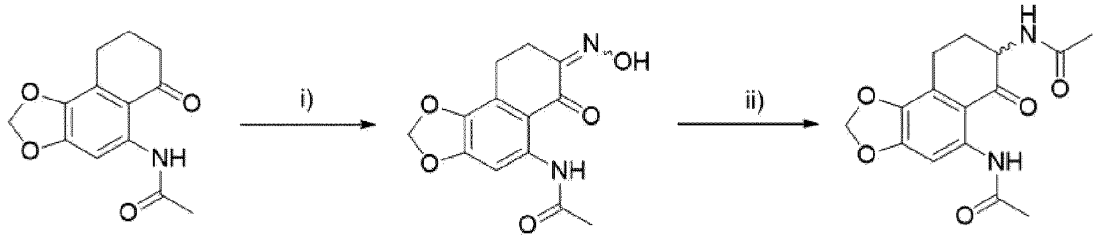


7 8

A suspension of N-(6-oxo-6,7,8,9-tetrahydronaphto [1,2-d][1,3]dioxol-5-yl)acetamide (50 mg, 0.202 mmol) in tetrahydrofuran (1.2 mL) was cooled to 0 °C, and potassium tert-butoxide (27.2 mg, 0.243 mmol) and isoamyl nitrite (35.0

μL, 0.263 mmol) were added. The dark green mixture was stirred on ice (< 5°C) for 1.5 hours. Acetic acid (170 μL, 2.94 mmol), acetic anhydride (170 μL, 1.810 mmol), and zinc dust (66.1 mg, 1.011 mmol) were added to the reaction mixture. The suspension was stirred at 0 °C for 2 hours. The reaction mixture was filtered over Celite and flushed with DCM. The filtrate was concentrated under reduced pressure to obtain a black oily material. A crude product was purified through flash column chromatography (4g Si, 0-4% MeOH in DCM). A product fraction was concentrated to obtain a gray solid (32 mg, 52%) with 80 to 90% purity. A higher purity sample may be obtained by purification using preparative MPLC.

SC_ACID: m/z 305.4 $[M+H]^+$
1H NMR (400 MHz, DMSO-d6) δ 12.10 (s, 1H), 8.21 (d, J = 8.0 Hz, 1H), 8.12 (s, 1H), 6.15 (d, J = 9.3 Hz, 2H), 4.66 - 4.56 (m, 1H), 2.93 (dd, J = 8.9, 4.0 Hz, 2H), 2.14 (s, 3H), 2.13 - 1.93 (m, 2H), 1.91 (s, 3H).

**1-8. Synthesis of Compound 9**

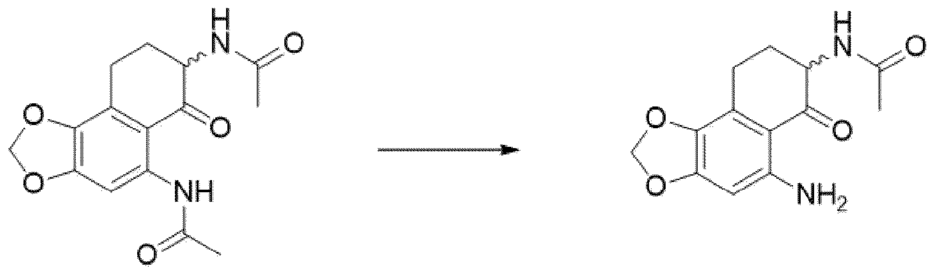

8 9

N,N'-(6-oxo-6,7,8,9-tetrahydronaphto[1,2-d][1,3]dioxol-5,7-diyl)diacetamide (244 mg, 0.802 mmol) was suspended in 2M hydrochloric acid(4.69 mL, 9.38 mmol) in ethanol/water (5/1). The mixture was heated to 55 °C for 4 hours. The black mixture was cooled to 0 to 5 °C. Triethylamine (1.4 mL, 10.04 mmol) was added dropwise while stirring. The mixture was then diluted with EtOH and evaporated and dried. The residue was distributed between water and DCM. The layers were separated and the aqueous layer was extracted once with DCM. The combined organic layers were dried over $Na_2SO_4$ and concentrated to obtain a product as a brown solid (178 mg, 73%) with 86% purity.

SC_ACID: m/z 263.0 $[M+H]^+$
1H NMR (400 MHz, DMSO) δ 8.04 (d, J = 8.0 Hz, 1H), 6.20 (s, 1H), 5.94 (d, J = 6.0 Hz, 2H), 4.48 - 4.41 (m, 1H), 3.08 (s, 2H), 2.88 - 2.69 (m, 2H), 2.15 - 2.03 (m, 1H), 1.88 (s, 3H), 1.86 - 1.75 (m, 1H).

**1-9. Synthesis of Compound 10**

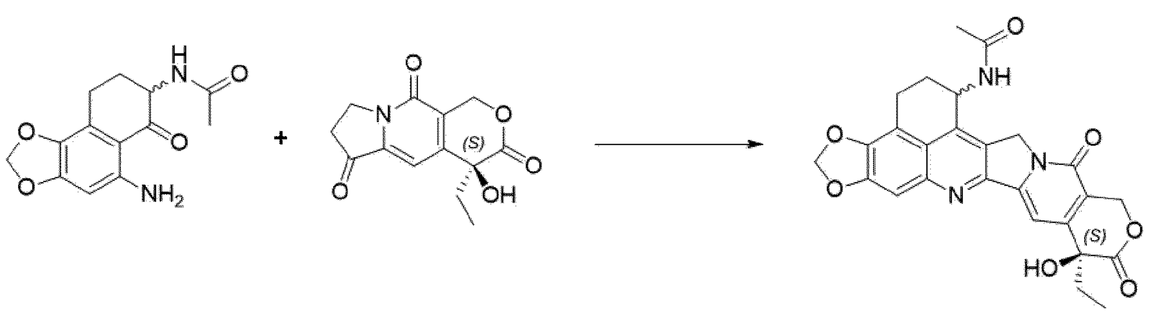

9 10

(4S)-4-ethyl-7,8-dihydro-4-hydroxy-1H-pyrano[3,4-f]indolizine-3,6,10(4H)-trione (146 mg, 0.555 mmol) and N-(5-amino-6-oxo-6,7,8,9-tetrahydronaphto[1,2-d][1,3]dioxol-7-yl)acetamide (112 mg, 0.427 mmol) were added to dry

toluene (4.5 mL). PPTS (21 mg, 0.085 mmol) was added, and the reaction mixture was stirred at 115 °C for 40 hours.

The reaction mixture was cooled to room temperature. The suspension was diluted with 2 mL of DCM and filtered. A black residue (210 mg) was obtained.

The crude product was purified by column chromatography (12g Si, 0 to 7% methanol in DCM) to obtain a product (45 mg, 21%) as a brown solid.

LCMS analysis showed two diastereomers.

SC_ACID: m/z 490.2 $[M+H]^+$

$^1$H NMR (400 MHz, DMSO-d6) δ 8.47 (t, *J*= 9.3 Hz, 1H), 7.42 (s, 1H), 7.24 (s, 1H), 6.49 (s, 1H), 6.29 (d, *J* = 5.2 Hz, 2H), 5.57 - 5.49 (m, 1H), 5.41 (s, 2H), 5.23 - 5.07 (m, 2H), 3.09 - 3.00 (m, 2H), 2.11 - 2.01 (m, 2H), 1.91 (s, 3H), 1.89 - 1.79 (m, 2H), 0.87 (t, *J* = 7.1 Hz, 3H).

**1-10. Synthesis of PBX-7011 and PBX-7012**

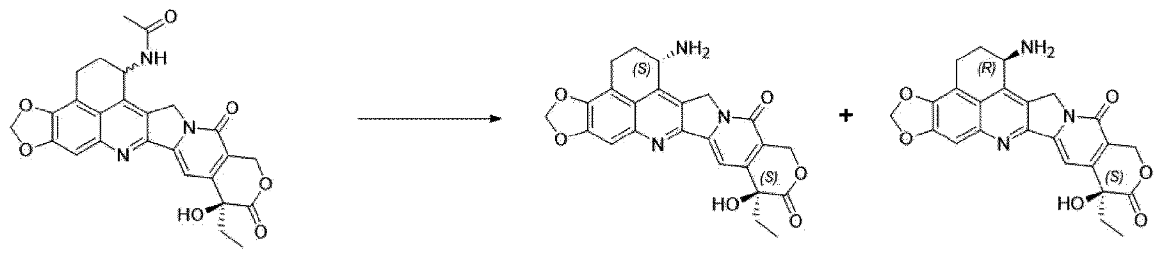

10 PBX-7011 PBX-7012

N-((10S)-10-ethyl-10-hydroxy-11,14-dioxo-2,3,10,11,14,16-hexahydro-1H,13H-benzo[de][1,3]dioxolo[4,5-g]pyrano[3',4':6,7]indolizino[1,2-b]quinolin-1-yl)acetamide (73.5 mg, 0.150 mmol) was dissolved in 1.0 mL of 6N HCl, and stirred at 90 °C for 8 hours and then overnight at room temperature. The reaction mixture was concentrated under reduced pressure and purified twice through acidic preparative MPLC (Luna2-30) twice. A fraction containing the separated diastereomer was acidified with 5 drops of 3N HCl and lyophilized to obtain a product as a yellow solid.

1st eluting isomer: PBX-7011, 23 mg, 34% yield

U_AN_ACID: m/z 448.4 $[M+H]^+$

$^1$H NMR (400 MHz, DMSO-d6) δ 8.54 (s, 3H), 7.50 (s, 1H), 7.27 (s, 1H), 6.52 (s, 1H), 6.34 (d, *J* = 13.3 Hz, 2H), 5.77 (d, *J* = 19.3 Hz, 1H), 5.44 (s, 2H), 5.37 (d, *J* = 19.3 Hz, 1H), 5.05 (s, 1H), 3.19 - 3.02 (m, 2H), 2.46 (s, 1H), 2.20 - 2.03 (m, 1H), 1.94 - 1.81 (m, 2H), 0.88 (t, *J* = 7.3 Hz, 3H).

2nd eluting isomer: PBX-7012, 28 mg, 41% yield

U_AN_ACID: m/z 448.2 $[M+H]^+$

$^1$H NMR (400 MHz, DMSO-d6) δ 8.57 (d, *J* = 4.7 Hz, 3H), 7.51 (s, 1H), 7.27 (s, 1H), 6.52 (s, 1H), 6.34 (d, *J* = 12.2 Hz, 2H), 5.76 (d, *J* = 19.4 Hz, 1H), 5.44 (s, 2H), 5.37 (d, *J* = 19.4 Hz, 1H), 5.08 (s, 1H), 3.16 - 2.98 (m, 2H), 2.46 (s, 1H), 2.18 - 2.06 (m, 1H), 1.94 - 1.80 (m, 2H), 0.87 (t, *J* = 7.3 Hz, 3H).

**Preparation Example 2: Synthesis of active camptothecin derivatives of Chemical Formulas 4 and 4-1 (PBX-7014 and PBX-7015)**

This example illustrates the synthesis of compounds PBX-7014 and PBX-7015, starting from two separated diastereomers of the exatecan-hybrid compounds (PBX-7011 and PBX-7012).

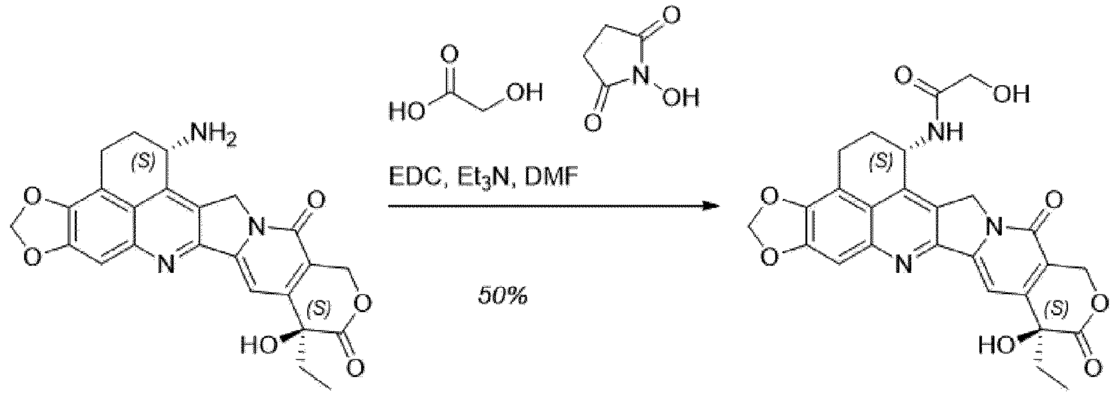


**PBX-7011** **PBX-7014**

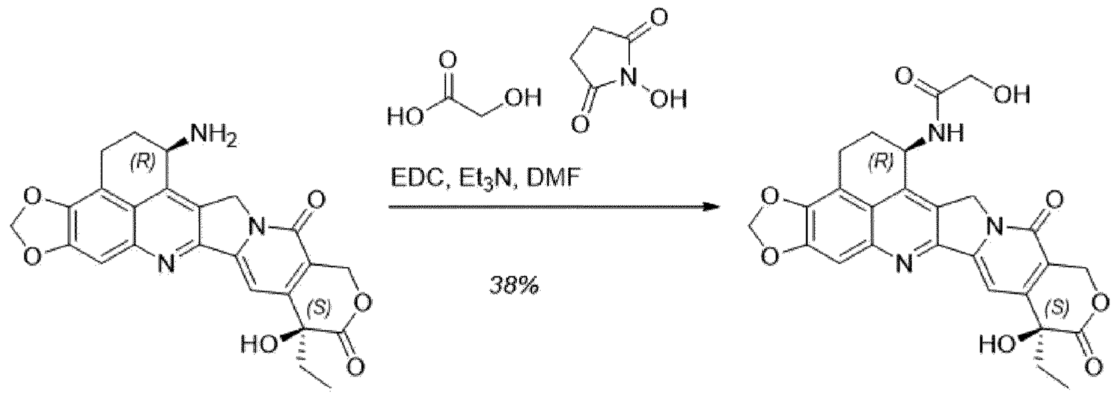


**PBX-7012** **PBX-7015**

**2-1: Synthesis of PBX-7014**

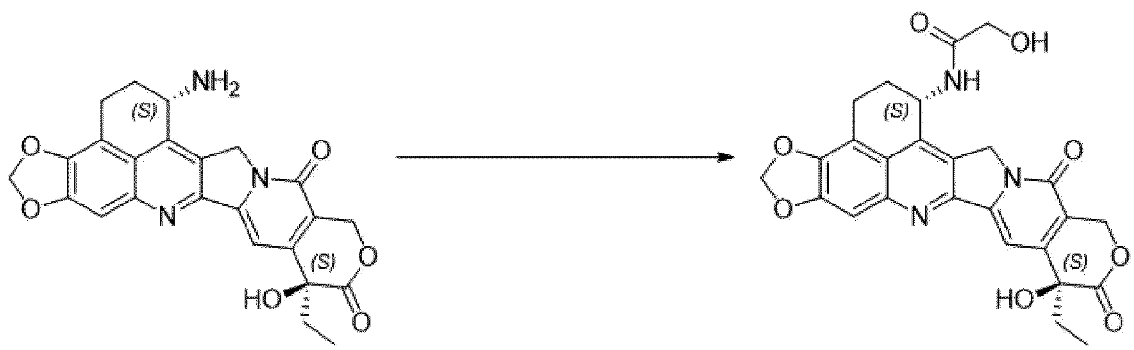

**PBX-7011** **PBX-7014**

A stock solution of activated glycolic acid was prepared according to the following procedure: Glycolic acid (17 mg, 0.224 mmol) was dissolved in 1 mL of N,N-dimethylformamide. HOSu (25.7 mg, 0.223 mmol) and EDC (42.8 mg, 0.223 mmol) were added. The reaction mixture was stirred at room temperature for 1 hour.

Subsequently, 0.4 mL of activated acid solution was added to a suspension of (1S,10S)-1-amino-10-ethyl-10-hydroxy-1,2,3,10,13,16-hexahydro-11H,14H-benzo[de][1,3]dioxolo[4,5-g]pyrano[3',4':6,7]indolizino[1,2-b]quinolin-11,14-dione (40 mg, 0.089 mmol) and triethylamine (0.025 mL, 0.179 mmol) in N,N-dimethylformamide (2.5 mL). The mixture was stirred at room temperature for 3 hours. 0.05 mL of a freshly prepared active acid solution was added.

Subsequently, the reaction mixture was further stirred at room temperature for 2 hours. The reaction mixture was evaporated and dried. A crude product was purified by column chromatography (0 to 8% methanol in chloroform). Thereby, a yellow solid containing the PBX-7014 product and residual succinimide was obtained. The product was further purified by acidic preparative MPLC (Luna5-40) and a product fraction was lyophilized, thereby obtaining a light yellow solid. Yield: 20 mg, 50%

U_AN_ACID: m/z 506.2 $[M+H]^+$
$^1$H NMR (400 MHz, DMSO-d6) δ 8.40 (d, *J* = 8.9 Hz, 1H), 7.40 (s, 1H), 7.23 (s, 1H), 6.49 (s, 1H), 6.28 (d, *J* = 4.6 Hz, 2H), 5.61 - 5.45 (m, 2H), 5.45 - 5.35 (m, 2H), 5.19 - 5.06 (m, 2H), 3.95 (s, 2H), 3.13 - 2.96 (m, 2H), 2.21 - 2.02 (m, 2H), 1.94 - 1.77 (m, 2H), 0.87 (t, *J* = 7.3 Hz, 3H).

**2-2: Synthesis of PBX-7015**

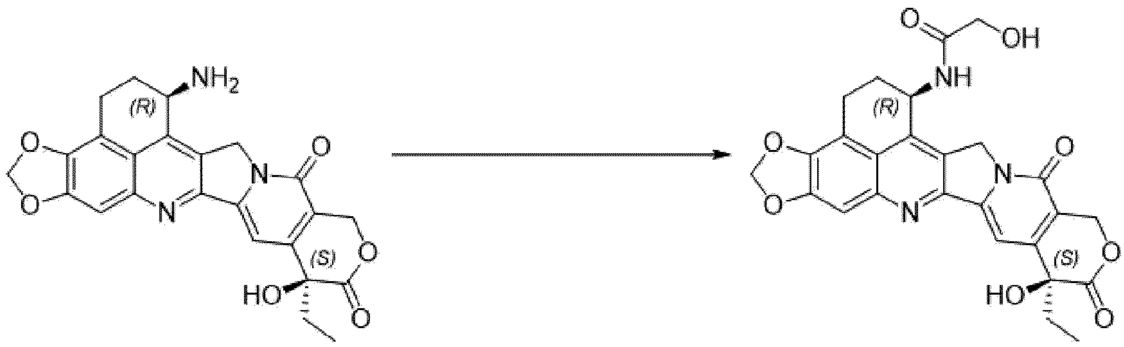

PBX-7012 PBX-7015

A stock solution of activated acid was prepared according to the following procedure:
Glycolic acid (17.00 mg, 0.223 mmol) was dissolved in 1 mL of N,N-dimethylformamide. HOSu (25.7 mg, 0.223 mmol) and EDC (42.8 mg, 0.223 mmol) were added. The reaction mixture was stirred at room temperature for 1 hour.
Subsequently, 0.25 mL of activated acid solution was added to a suspension of (1R,10S)-1-amino-10-ethyl-10-hydroxy-1,2,3,10,13,16-hexahydro-11H,14H-benzo[de][1,3]dioxolo[4,5-g]pyrano[3',4':6,7]indolizino[1,2-b]quinolin-11,14-dione (25 mg, 0.056 mmol) and triethylamine (0.016 mL, 0.112 mmol) in N,N-dimethylformamide (2.5 mL). The mixture was stirred overnight at room temperature. 0.03 mL of a freshly prepared activated acid solution was added. Subsequently, the reaction mixture was stirred at room temperature for 2 hours. The reaction mixture was combined with a previous smaller batch, evaporated, and dried. A crude product was purified by column chromatography. Thereby, a yellow solid containing the PBX-7015 product and residual succinimide was obtained. The product was purified by acidic preparative MPLC (Luna5-40). A product fraction was lyophilized to obtain a light yellow solid. Yield: 15 mg, 38%

U_AN_ACID: 506.2 $[M+H]^+$
$^1$H NMR (400 MHz, DMSO) δ 8.44 (d, *J* = 9.0 Hz, 1H), 7.41 (s, 1H), 7.24 (s, 1H), 6.48 (s, 1H), 6.29 (d, *J* = 2.3 Hz, 2H), 5.61 - 5.44 (m, 2H), 5.44 - 5.36 (m, 2H), 5.20 - 5.07 (m, 2H), 3.96 (s, 2H), 3.10 - 2.96 (m, 2H), 2.20 - 2.06 (m, 2H), 1.95 - 1.79 (m, *J* = 7.3 Hz, 2H), 0.87 (t, *J* = 7.3 Hz, 3H).

**Preparation Example 3: Synthesis of active camptothecin derivative of Chemical Formula 5 (PBX-7016)**

This example illustrates the synthesis of compound PBX-7016 starting from the exatecan-hybrid compound (PBX-7011).

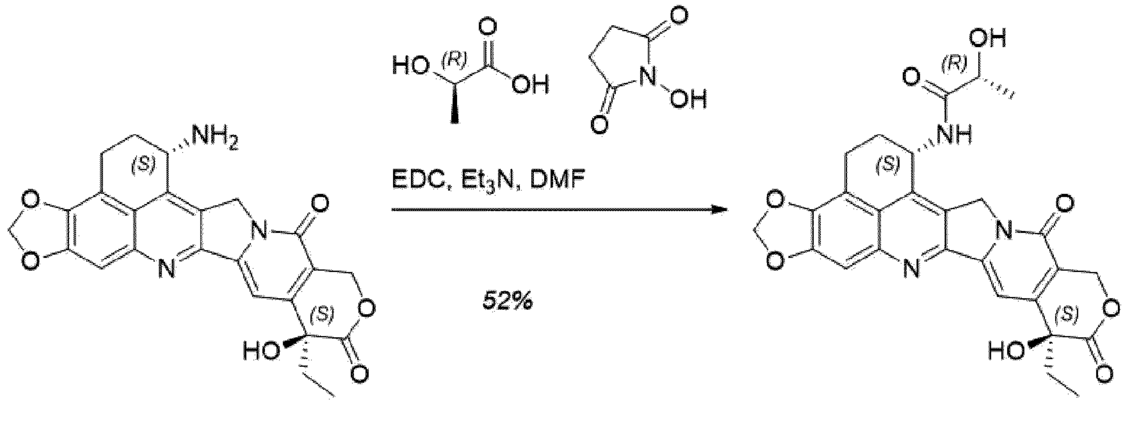


PBX-7011

PBX-7016

A stock solution of activated D-lactic acid was prepared as follows.
D-lactic acid (22 mg, 0.244 mmol) was dissolved in 1 mL of N,N-dimethylformamide. HOSu (27 mg, 0.235 mmol) and EDC (38.6 mg, 0.201 mmol) were added. The reaction mixture was stirred at room temperature for 2 hours.

Subsequently, 0.3 mL of activated acid solution was added to a solution of (1S,10S)-1-amino-10-ethyl-10-hydroxy-1,2,3,10,13,16-hexahydro-11H,14H-benzo[de][1,3]dioxolo[4,5-g]pyrano[3',4':6,7]indolizino[1,2-b]quinolin-11,14-dione (36 mg, 0.080 mmol) and triethylamine (0.022 mL, 0.161 mmol) in N,N-dimethylformamide (2.5 mL). The mixture was stirred at room temperature for 6 hours. 0.05 mL of the prepared active acid solution was added. Subsequently, the reaction mixture was stirred overnight at room temperature. The reaction mixture was directly purified by acidic preparative MPLC (Luna10-50), and a product fraction was lyophilized, thereby obtaining a light yellow solid.

Yield: 22 mg, 52%
U_AN_ACID: m/z 520.2 $[M+H]^+$
$^1H$ NMR (400 MHz, DMSO) δ 8.43 (d, J = 9.1 Hz, 1H), 7.41 (s, 1H), 7.23 (s, 1H), 6.50 (s, 1H), 6.29 (d, J = 2.1 Hz, 2H), 5.62 - 5.58 (m, 1H), 5.58 - 5.51 (m, 1H), 5.41 (s, 2H), 5.21 - 5.01 (m, 2H), 4.17 - 4.07 (m, 1H), 3.14 - 2.95 (m, 2H), 2.19 - 2.04 (m, 2H), 1.92 - 1.78 (m, 2H), 1.39 (d, J = 6.8 Hz, 3H), 0.87 (t, J = 7.3 Hz, 3H).

As described above, since PBX-7016 of Chemical Formula 5 can be synthesized from PBX-7011 of Chemical Formula 3, PBX-7017 of Chemical Formula 5-1 can be synthesized from PBX-7012 of Chemical Formula 3-1 in the same manner.

**Preparation Example 4: Synthesis of active camptothecin derivative of Chemical Formula 9 (PBX-7016)**

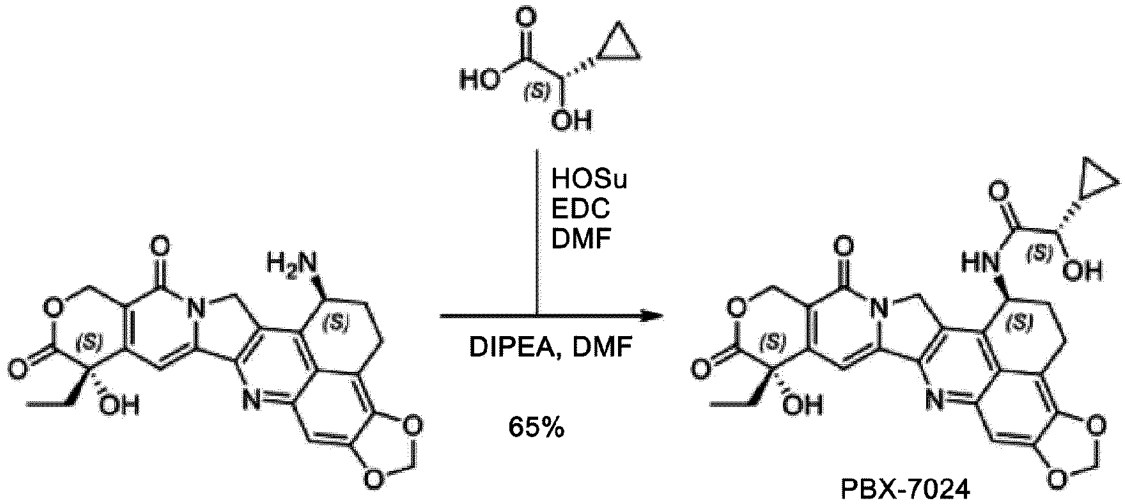


PBX-7011

PBX-7024

20 mg shipped

PBX-7024 was prepared with high yield by binding (2S)-2-cyclopropyl-2-hydroxyacetic acid to the PBX-7011 compound.

(2S)-2-cyclopropyl-2-hydroxyacetic acid (26 mg, 0.244 mmol) was dissolved in 1 mL of N,N-dimethylformamide. HOSu (26 mg, 0.226 mmol) and EDC (42 mg, 0.219 mmol) were added. The reaction mixture was stirred at room temperature for 2 hours. Subsequently, 0.6 mL of activated acid solution was added to a solution of (1S,10S)-1-amino-10-ethyl-10-hydroxy-1,2,3,10,13,16-hexahydro-11H,14H-benzo[de][1,3]dioxolo[4,5-g]pyrano[3',4':6,7]indolizino[1,2-b]quinolin-11,14-dione (40 mg, 0.089 mmol) and DIPEA (0.047 mL, 0.268 mmol) in N,N-dimethylformamide (2.5 mL). The mixture was stirred overnight at room temperature. The reaction mixture was directly purified by acidic preparative MPLC (Luna10-50), and a product fraction was lyophilized, thereby obtaining a light white solid.

Yield: 32 mg, 65%

U_AN_ACID: m/z 520.2 $[M+H]^+$

$^1$H NMR (400 MHz, DMSO-d6) δ 8.33 (d, *J* = 8.7 Hz, 1H), 7.39 (s, 1H), 7.23 (s, 1H), 6.48 (s, 1H), 6.28 (d, *J* = 4.9 Hz, 2H), 5.50 (q, *J* = 6.7 Hz, 1H), 5.40 (s, 3H), 5.23 - 5.06 (m, 2H), 3.62 (d, *J* = 6.3 Hz, 1H), 3.03 (q, *J* = 6.2 Hz, 2H), 2.21 - 2.02 (m, 2H), 1.92 - 1.79 (m, 2H), 1.18 - 1.08 (m, 1H), 0.87 (t, *J* = 7.3 Hz, 3H), 0.47 - 0.30 (m, 4H).

**Example 1: Analysis of binding mode of camptothecin derivative with TOP1/DNA**

[Table 2]

| Camptothecin derivative | Docking score (the lower the more stable) | |
|---|---|---|
| | Normal TOP 1 | Mutated TOP1 |
| 1. SN-38 | -10310 | -10.314 |
| 2. Exatecan | -11.023 | -10.362 |
| 3. Dxd | -9.989 | -10.377 |
| 4. FL118 | -9.969 | -9.361 |
| FBX-7011 of Chemical Formula 3 | -11.328 | -9.094 |
| FBX-7012 of Chemical Formula 3-1 | -10.358 | -10.061 |

According to the results of predicting a binding mode (docking score) through molecular dynamics simulation, all camptothecin derivatives in Table 2 were basically bound between DNA bases by strong π-π stacking (FIGS. 4 and 5), the ester of lactone formed a hydrogen bond (H-bond) with Arg488 of TOP1, the ethyl and OH groups formed a hydrogen bond with Asp533 of TOP1 (FIG. 2), which plays a role in the camptothecin derivatives binding well between the TOP1 protein and DNA.

The SN-38 drug is stabilized by H-bonding with Glu356 and Arg364 of TOP1 (FIG. 4).

The exatecan drug was further stabilized by H-bonding with a surrounding DNA base (FIG. 4).

The methylenedioxo (-$OCH_2O$-) pentagonal ring of the FL118 drug is weaker than hydrogen bonding (unlike actual cytotoxicity) and shows a worse docking score than the SN-38 drug or the exatecan drug, but such bonding is made perfectly between DNA bases and also helps solvation (FIG. 4).

As shown in FIG. 5, the compound (PBX-7011) of Chemical Formula 3 and the compound (PBX-7012) of Chemical Formula 3-1, which were designed by mixing functional groups of the FL118 drug and the exatecan drug according to the present invention, are diastereomeric, so they are predicted to share the same binding pose and stably bind to TOP 1. Particularly, the compound (PBX=7011) of Chemical Formula 3 had a higher docking score than the SN-38 drug.

The compound (PBX-7011) of Chemical Formula 3 having the same chirality as the exatecan drug showed slightly better binding than the compound (PBX-7012) of Chemical Formula 3-1.

Meanwhile, regarding the TOP1 mutation (E418K) that is resistant to Trodelvy (payload: SN-38), as shown in FIG. 6, the E418K mutation is far away from the drug binding site.

As shown in FIGS. 7 and 8, as a result of performing energy minimization after E418K mutation, it was confirmed through MD simulation that newly introduced Lys418 changed a part of the drug binding site to a relatively polar environment and continued ionic bonding with the surrounding Glu356. MD simulation showed that when part of the binding site becomes polar by mutation, materials with polar functional groups like the SN-38 drug move slightly away from the existing binding site to facilitate the ionic interaction between Lys418 and Glu356. This can weaken the interaction between the lactone and Asp533 and Lys532, which is the inhibitory activity principle the same series of TOP1 inhibitors have in common.

When the SN-38 drug was docked to the mutated TOP1 structure, although the SN-38 drug moved away from the binding site toward a side, it formed a new hydrogen bond (H-bonding), thereby showing a high docking score. Therefore, there is a limit to explaining the phenomenon in which the mutated TOP1 is resistant to SN-38 with the docking score.

As shown in FIGS. 7 and 8, when analyzing the interaction between the SN-38 drug and TOP1 during MD simulation, it was confirmed that, due to mutation, the SN-38 interaction with Asp533 and Arg488 (89%) decreased to 10% or less, whereas the interaction with Asn352 and Glu356 increased from 40% to 80%.

When docking is carried out after TOP1 mutation, the SN-38 drug moves to a newly modified binding site and binds in a new form, thereby showing a score similar to the previous score (Table 2).

On the other hand, as shown in Table 2, it was confirmed that the exatecan drug, the FL118 drug, the compound (PBX-7011) of Chemical Formula 3, and the compound (PBX-7012) of Chemical Formula 3-1, which lack a polar functional group (-OH) at C-10 of an A-ring, have relatively lower docking scores with the mutated TOP1 due to a partial environmental change at the new binding site. Nevertheless, MD simulation indicated that the existing interaction between the lactone and Asp533 and Lys532 is maintained in the exatecan drug, the FL118 drug, the compound (PBX-7011) of Chemical Formula 3, and the compound (PBX-7012) of Chemical Formula 3-1.

As shown in FIG. 9, even with TOP1 mutation, the compound (PBX-7011) of Chemical Formula 3 still maintains the existing binding mode (particularly, the lactone and -OH group).

Consequently, while the E418K mutation does not directly interact with a TOP1 inhibitor, to explain the situation in which the FL 118 drug inhibits the TOP1 mutation that shows resistance to the SN-38 drug by E418K at a high level, it can be assumed that, unlike the docking results, the new binding of SN-38 to the binding site modified by the mutation weakens the interaction between the lactone and Asp533 and Lys532, thereby failing to inhibit the action of TOP1.

Under the assumption, the exatecan drug, the FL118 drug, the compound (PBX-7011) of Chemical Formula 3, and the compound (PBX-7012) of Chemical Formula 3-1 are expected to show less resistance due to a continuous interaction with Asp533 and Lys532 of mutated TOP1.

## Example 2

ForFL118, exatecan, SN-38, Dxd, exatecan-lactic acid, PBX-7011, PBX-7014, PBX-7016, and PBX-7024, the expression inhibition assay and the *in vitro* cell viability assay of anti-apoptotic proteins were performed as follows.

### 2-1: Confirmation of ability to inhibit the expression of cancer-associated survival genes DDX5, survivin, Mcl-1, XIAP and cIAP2 through Western blotting

(1) Protein extraction

AFaDu cell line was seeded in a 6-well plate at 200,000 cells/well and cultured (37 °C, 5% $CO_2$). After 24 hours, the wells were respectively treated with drugs (FL118 drug, SN-38 drug, exatecan drug, PBX-7011, PBX-7014, and PBX-7016) at a concentration of 0 nM, 10 nM, and 100 nM, and cultured (37 °C, 5% $CO_2$) for 24 hours. The wells were

treated with 100 μL of RIPA buffer dissolved in a protease inhibitor cocktail. The plate was placed on ice and subjected to culture on an orbital shaker for 2 hours. The lysed cell-containing RIPA buffer was transferred to an EP tube and centrifuged (16000 rcf, 20 min, 4 °C). Subsequently, only the supernatant was transferred to a new EP tube. The protein concentration was confirmed through a protein assay.

(2) Protein separation through electrophoresis

A protein sample and 4x SDS-PAGE loading buffer were mixed in a 3:1 ratio, boiled at 95 °C for 10 minutes, and then cooled. The samples were loaded into the wells of a gel to so that the amount of protein was the same. The gel was subjected to electrophoresis at 60 V.

(3) Transfer of protein from gel to membrane

Seven sheets of activated filter paper, a PVDF membrane, a gel, and another seven sheets of filter paper were sequentially placed into cassettes, the lid was closed, the cassette was placed in the Trans-Blot® Turbo™ Transfer System, and then the protocol was run.

(4) Antibody incubation

The transferred membrane was immersed in blocking buffer and incubated (RT, 1h). Subsequently, it was cultured in a primary antibody solution (4 °C, overnight). The membrane was rinsed with TBST buffer for 3 minutes (repeated three times). The membrane was cultured in an HRP-conjugated secondary antibody solution (RT, 1h). It was rinsed with TBST buffer for 3 minutes (repeated three times).

(5) Imaging and result analysis

After immersing the membrane in an ECL substrate for about 3 to 5 minutes, signals were confirmed using the ChemiDoc™ MP Imaging System. The light emitted by oxidization of the luminol of ECL by HRP bound to the secondary antibody was detected and displayed as an image. Since the thickness of the band is proportional to the amount of protein, the amount of the protein may be compared through band thickness.

As shown in FIGS. 10 and 12, how protein expression levels change by the treatment of drugs (FL118 drug, SN-38 drug, exatecan drug, PBX-7011, PBX-7014, and PBX-7016) was confirmed. GAPDH is an enzyme involved in glycolysis, an essential metabolic process in cells. Since it is a gene that is always expressed in cells and its expression level does not change much, it is an indicator of whether equal amounts of protein samples were loaded onto a gel.

Instead of a FaDu cell line that does not express ABCG2, A549 overexpressing ABCG2 was seeded in the same manner as above, and 4 μg of protein was loaded to perform Western blotting (FIGS. 13 and 14).

**Review: Evaluation of PBX-7011, PBX-7014, and PBX-7016**

As a result of confirming the degree of expression inhibition of anti-apoptotic proteins in the FaDu cell line through Western blotting, PBX-7011, PBX-7014, and PBX-7016 showed that the expression of all of survivin, cIAP2, Mcl-1, and XIAP decreased as the concentration increased, and compared to the existing FL118, SN-38, and exatecan drug, the degree of expression inhibition was similar to FL118 and the exatecan drug, and higher than SN-38.

Example 2-1 showed a dual inhibitory action that inhibits topoisomerase 1 and degrades DDX5, which confirms the heterogeneous characteristics of DDX5 degradation and its anticancer effect.

Specifically, as a result of treating two cell lines (FaDu and A549) with certain concentrations (0, 10, and 100 nM) of drugs, and confirming the expression level of each protein (DDX, survivin, Mcl-1, and XIAP) through Western blotting, the DDX5 degradation by FL118 was superior to other substances, followed by 7011, 7016, and 7014 in this order.

The trends of not only DDX5 but also other anti-apoptotic proteins downstream thereof were similar.

In short, PBX-7014 and PBX-7016 showed a dual MoA, which not only inhibits topoisomerase-1 but also acts as a degrader of the oncoprotein DDX5 (p68) that regulates survivin, Mcl-1, and XIAP.

The evaluation results showed that among PBX-7014 and PBX-7016, PBX-7016 inhibited DDX5 in a concentration-dependent manner, and by observing that PBX-7016 inhibited survivin, Mcl-1, and XIAP, not only did PBX-7016 show the role of FL118 as a DDX5 degrader as well as a topoisomerase I inhibitor, it was confirmed that PBX-7016 works better than PBX-7014 (FIGS. 11 to 14).

### 2-2: Cell viability assay for FaDu cell line/ A549 cell line

The FaDu cell line (cancer cells not expressing ABCG2) or the A549 cell line (cancer cells overexpressing ABCG2) was seeded in a 96-well plate at 3,000 cells per well and cultured (37 °C, 5% $CO_2$). After 24 hours, the cells were treated with 100 μL of each drug at nine different concentrations (1/5 serial dilutions from 1000 nM). Here, the cells were treated with the Dxd drug, PBX-7014, PBX-7016, or PBX-7024. Here, a control (drug concentration: 0) that was not treated with a drug was also prepared. The cells were cultured (37°C, 5% $CO_2$) for 3 or 6 days. 100 μL of the CellTiter-Glo reagent (using the CellTiter-Glo® Luminescent Cell Viability Assay kit (Promega, G7571)) was added to each well and pipetted. After incubating at room temperature (RT) for 10 minutes, luminescence was measured. Provided that the luminescence value is 100% when the drug concentration is 0, the concentration that exhibits a 50% luminescence value is the $IC_{50}$ value.

Each of two cell lines (FaDu and A549) were treated with two compounds (PBX-7014, PBX-7016 and PBX-7024) and reference compounds (Dxd, SN-38, exatecan, and FL118), and incubated for 3 days to observe cell viability.

As shown in FIG. 15, PBX-7024 exhibited potent apoptotic efficacy not only in the FaDu cell line not expressing ABCG2 but also the cancer cell line A549 overexpressing ABCG2. That is, it was confirmed that, compared to Dxd, which is the payload used in the existing Enhertu, PBX-7024 has the same or higher $IC_{50}$ level.

In the ABCG2-overexpressing cancer cell line, A549, as shown in FIG. 15, camptothecin compounds, including Dxd, showed an increased $IC_{50}$ value, whereas it can be confirmed that PBX-7016 and PBX-7024 still maintain potent efficacy.

That is, when novel camptothecin compounds, PBX-7016 and PBX-7024, are used, unlike ADCs using the existing camptothecin compounds, such as SN-38 and Dxd, they have the effect of overcoming the resistance mechanism caused by ABCG2 overexpression.

## Example 3. New PBX-series compounds as candidate for ADC payload: *in vitro* cell viability test

Cell viability assays were performed on an MDA-MB-453 (HER2++) cell line and a FaDu (HER2+) cell line in the same manner as Example 2-2.

Each of two cell lines (MDA-MB-453 and FaDu) was treated with five compounds (PBX-7014, PBX-7016, PBX-7018, PBX-7020, and PBX-7022) and one reference compound (Dxd), and incubated for 3 days to observe cell viability. The results are shown in FIG. 16.

As shown in FIG. 16, the evaluation results confirmed that PBX-7016, PBX-7018, PBX-7020, and PBX-7022 have an $IC_{50}$ that is the same or higher than that of Dxd. In addition, it was confirmed that the FaDu cell line not exhibiting ABCG2 has an $IC_{50}$ value that is the same or higher than that of Dxd.

## Example 4. PBX-7016 IV and IP pharmacokinetic profile in NOD SCID mice

An experimental method is shown in Table 3 below. The IV plasma concentration-time data of PBX-7016 is shown in Table 4, and the PBX-7016 IP plasma concentration-time data is shown in Table 5.

[Table 3]

| | | | In-life | | |
|---|---|---|---|---|---|
| Standard PK | | | | **Species/strain:** | NOD SCID Mice |
| 3 | | | | **Sex:** | Female |
| IV | 1 | mg/kg | | | |
| IP | 1 | mg/kg | | | |
| IV | 5% DMSO and 95%(0.1% (w/v) hydroxypropyl-beta-cyclodextrin in saline) adjust PH to 8-9 | | | | |
| IP | 5% DMSO and 95%(0.1% (w/v) hydroxypropyl-beta-cyclodextrin in saline) adjust PH to 8-9 | | | | |
| IV | 0.5 | mg/mL | | | |
| IP | 0.2 | mg/mL | | | |
| IV | 0.167, 1, 4, 12, 24 and 48 hr post dose. | | | | |

(continued)

| | | In-life | | |
|---|---|---|---|---|
| Standard PK | | | Species/strain: | NOD SCID Mice |
| 3 | | | Sex: | Female |
| IP | 0.167, 1, 4, 12, 24 and 48 hr post dose. | | | |

[Table 4]

| IV Dose | 1 mg/kg | | | | | |
|---|---|---|---|---|---|---|
| Time (h) | Concentration (ng/mL) | | | Mean (ng/mL) | SD (ng/mL) | CV (%) |
| | Mouse 1 | Mouse 2 | Mouse 3 | | | |
| 0.167 | 76.2 | 54.8 | 46.8 | 59.3 | 15.2 | 25.6 |
| 1 | 10.6 | 6.85 | 3.35 | 6.9 | 3.6 | 52.3 |
| 4 | BLOQ | BLOQ | BLOQ | NA | NA | NA |
| 12 | BLOQ | BLOQ | BLOQ | NA | NA | NA |
| 24 | BLOQ | BLOQ | BLOQ | NA | NA | NA |
| 48 | BLOQ | BLOQ | BLOQ | NA | NA | NA |

[Table 5]

| IP Dose | 1 mg/kg | | | | | |
|---|---|---|---|---|---|---|
| Time (h) | Concentration (ng/mL) | | | Mean (ng/mL) | SD (ng/mL) | CV (%) |
| | Mouse 4 | Mouse 5 | Mouse 6 | | | |
| 0.167 | 158 | 186 | 149 | 164 | 19 | 11.7 |
| 1 | 10.5 | 23.9 | 14.0 | 16.1 | 7.0 | 43.1 |
| 4 | BLOQ | BLOQ | BLOQ | NA | NA | NA |
| 12 | BLOQ | BLOQ | BLOQ | NA | NA | NA |
| 24 | BLOQ | BLOQ | BLOQ | NA | NA | NA |
| 48 | BLOQ | BLOQ | BLOQ | NA | NA | NA |

From the results in Tables 4 and 5, when the PBX-7016 compound is administered intravenously or intraperitoneally to mice, it was confirmed that the PBX-7016 compound cleared from the blood very quickly, so it is expected that the PBX-7016 compound alone, not in the form of a prodrug or ADC, will show high safety when administered *in vivo.* Since the PBX-7016 compound is released from the body within a very short time upon IV administration, it may act as a safety device to reduce the systemic exposure of a drug even when released prematurely into the body after entering the body.

In addition, this experiment shows that a PK of the PBX-7016 compound alone, used as a payload, is a safe payload in which the clearance of the PBX-7016 drug occurs in the blood within a short period of time. That is, after the PBX-7016 compound exerts a bystander effect at a tumor site, it is safely released from the body within a very short period of time even when circulating in the blood.

**Example 5. Carboxylate-to-lactone conversion rate of PBX-7016 at pH 6.0**

Example 5 (FIGS. 17 and 18) shows an experiment related to the activation of a lacto moiety of camptothecin, known as an activator of Top1 inhibitors.

**5-1. Carboxylate to lactone form conversion at pH 6.0**

PBX-7016 powder was prepared by dissolving it in DMSO at 5 mM. Since PBX-7016 dissolved in DMSO is present in a lactone form, it was diluted 1/10 with 0.1 N NaOH and incubated in a 25 °C water bath for 30 minutes to convert it to a carboxylate form. The carboxylate form of PBX-7016 was diluted to 3 μM using 4 °C PBS (pH 6.0) containing 10% DMSO. Here, the pH change was minimized by adding the same amount of HCl as the 0.1 N NaOH used. After dilution, centrifugation was performed under conditions of 4 °C, 2 minutes, and 13000 RCF, and the supernatant was used as a

sample.

When the PBX-7016 lactone form is generated, two solvents (Solvent A: 100 mM acetate buffer (pH 5.5), and Solvent B: CAN) were used to separate it through RP-HPLC. The column used was a Cortecs C18 2.7 μm, 4.6x50 mm column, and analysis was conducted under the following conditions: 1) 20 min : 5% B, 2) sample injection, 3) 2 min : 5% B, 4) 10 min : 5% B → 50% B, and 5) 1 min : 50% B.

To measure changes over time, a sample chamber was maintained at 37 °C and was set to be injected at each measurement time. The measurement time was set to 0, 20, 40, 60, 90, 120, 180, 240, 300, 360, 480, 600, 720, 840, 960, 1200, and 1440 minutes.

The content of each of lactone and carboxylate was calculated from the peak area thereof. Since the absorption coefficients of lactone and carboxylate are different, the lactone area was obtained by multiplying the carboxylate area by the equation below.

$$\text{lactone } EC_{390} \text{ / carboxylate } EC_{390} = 0.918$$

The experimental results of the carboxylate-to-lactone conversion at pH 6.0 are shown in FIG. 17. As shown in FIG. 17, since it was confirmed that various camptothecin compounds change their form from carboxylate to lactone, an active form which inhibits topoisomerase I, at pH 6.0 at a similar rate and in equivalent amounts, the same level of activity is expected in a tumor microenvironment, which is generally pH 6.0.

**5-2. Lactone to carboxylate form conversion at pH 7.4**

PBX-7016 powder was prepared by dissolving it in DMSO at 5 mM, and diluted with 4°C PBS (pH 7.4) containing 10% DMSO to prepare 3 μM PBX-7016. Subsequently, it was centrifuged under conditions of 4 °C, 2 minutes, and 13000 RCF, and a supernatant was used as a sample. PBX-7016 dissolved in DMSO is present in a lactone form.

When the PBX-7016 carboxylate form is generated, two solvents (Solvent A: 100 mM acetate buffer (pH 5.5), and Solvent B: CAN) were used to separate it through RP-HPLC. The column used was a Cortecs C18 2.7 μm, 4.6x50 mm column, and analysis was conducted under the following conditions: 1) 20 min : 5% B, 2) sample injection, 3) 2 min : 5% B, 4) 10 min : 5% B → 50% B, and 5) 1 min : 50% B.

To measure changes over time, a sample chamber was maintained at 37 °C and a sample was set to be injected at each measurement time. The measurement time was set to 0, 20, 40, 60, 90, 120, 180, 240, 300, 360, 480, 600, and 720 minutes.

The content of each of lactone and carboxylate was calculated from the peak area thereof. Since the absorption coefficients of lactone and carboxylate are different, the lactone area was obtained by multiplying the carboxylate area by the equation below.

$$\text{lactone } EC_{390} \text{ / carboxylate } EC_{390} = 0.918$$

The experimental results of the lactone-to-carboxylate conversion at pH 7.4 are shown in FIG. 18. From the results in FIG. 18, it was confirmed that at blood pH 7.4, the PBX-7016 compound was converted from a lactone form to a carboxylate form more rapidly and at a higher rate than other reference compounds (exatecan, Dxd, SN-38, and FL118). Therefore, the PBX-7016 compound is expected to have higher safety in normal cells because it is present in the inactive carboxylate form, not the active lactone form, which shows cytotoxicity in the blood at a faster and higher rate than the reference compounds.

**Preparation Example 5: Synthesis of 25-4 and 25-6 from PBX-7014 and PBX-7016 and preparation of their ADCs (DAR 7 and 8) (trastuzumab-25-4 and trastuzumab-25-6)**

Linker-payloads, 25-4 (Chemical Formula 10) and 25-6 (Chemical Formula 11), which include two compounds (PBX-7014 and PBX-7016) and an enzymatically cleavable linker system GGFG, were synthesized.

[Chemical Formula 10]

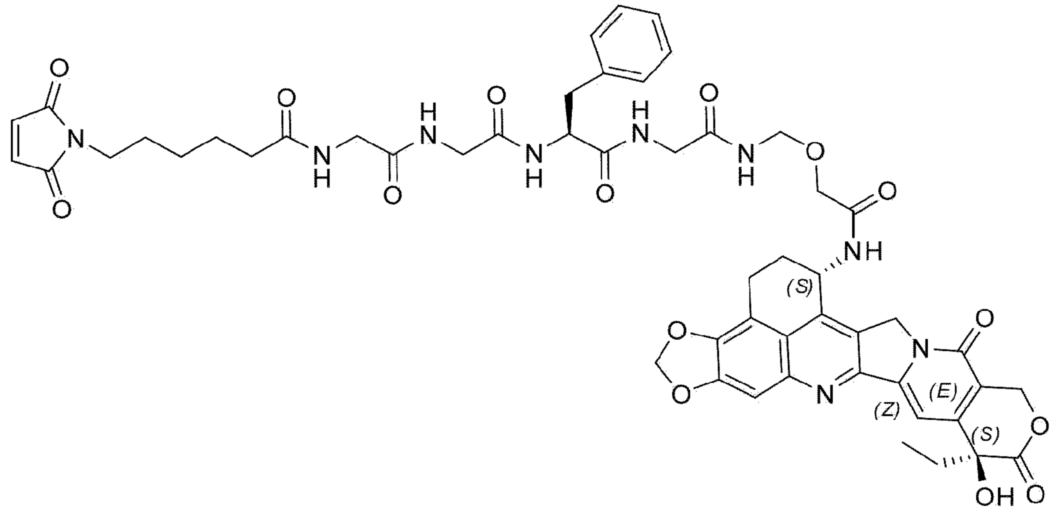

[Chemical Formula 11]

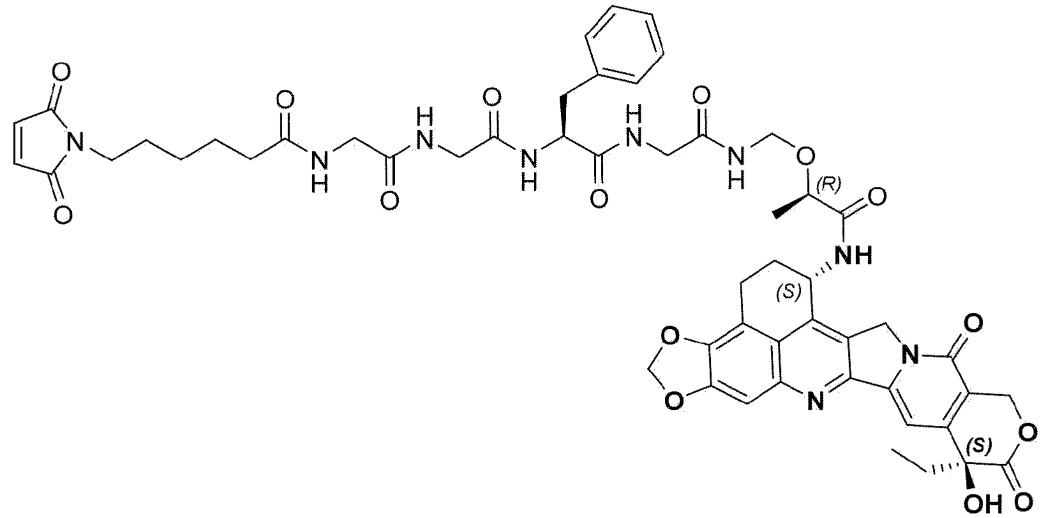

The molecular structure of 25-4 is GGFG-PBX-7014, and the molecular structure of 25-6 is GGFG-PBX-7016. That is, each uses the same GGFG linker as Enhertu®.

Further, ADCs were prepared with PBX-7014 and PBX-7016 as payloads using the same GGFG linker and trastuzumab antibody as Enhertu®.

The linker-payload compounds of Chemical Formula 10 and Chemical Formula 11 were conjugated with trastuzumab, which is a Her2 target antibody, thereby synthesizing ADCs (Tra-25-4 and Tra-25-6, both DAR 8).

Trastuzumab-25-4 (trastuzumab-7014) was prepared as follows. The prepared trastuzumab was prepared by treating 825 μM TCEP with 27.5 μM antibodies at 25 °C for 2 hours after buffer exchange with a reaction buffer (150 mM NaCl, 50mM histidine pH 6.0) using a PD-10 desalting column to create a thiol site required for a reaction from a disulfide of the antibody.

Subsequently, excess TCEP was removed using a PD-10 desalting column, and a first conjugation reaction was performed by reacting a 61.9 μM 25-4 drug linker (Chemical Formula 10) and 13.8 μM reduced trastuzumab in a reaction buffer containing 10% DMSO at 25 °C for 1 hour.

Trastuzumab-25-6 (trastuzumab-7016) was prepared in the same manner as described above, and instead of a 61.9 μM 25-4 drug linker (Chemical Formula 10), the same concentration of a 25-6 drug linker (Chemical Formula 11) was used.

Afterward, each ADC was purified using SEC, and confirmed to be purified as a monomer without aggregation (FIG. 19). SDS-PAGE showed that the drug was conjugated through the band shift of a light chain and a heavy chain (FIG.

20).

**Example 6. Anti HER2 ADCs with PBX-payloads: binding affinity analysis**

A coating buffer was prepared by diluting a target antigen to 0.66 μg/mL using an ELISA plate coating buffer (R&D Systems, #DY006). Coating was performed using 100 μL/well of the prepared coating buffer was used. After covering with a plate cover, the resulting sample was maintained at 2 to 8 °C and reacted overnight (12 to 18 hrs). The plate used was Corning, #3590. The plate was washed three times with 200 μL or more of TBS Tween-20 buffer (Thermo, #28360). During washing, the liquid was removed first with a pipette, and excess buffer was removed by gently tapping the plate on a paper towel. Blocking was performed at room temperature for 1 hour using 200 μL of assay buffer. The liquid was removed first with a pipette, and excess buffer was removed by gently tapping the plate on a paper towel. Control antibodies to be measured and each ADC were prepared in assay buffer at 1 μg/mL, and then subjected to 1/5 serial dilution to prepare the following concentrations in the required amount or more. For example, based on the duplication standard [240 μL assay buffer + 60 μL of sample at the previous concentration], the resulting solution as diluted to the following concentrations to prepare samples: 1 μg/mL, 0.4 μg/mL, 0,08 μg/mL, 0.016 μg/mL, 0.0032 μg/mL, 0.00064 μg/mL, and 0.000128 μg/mL. The diluted control antibodies and ADCs were bound in duplicate at 100 μL per coated well. The reaction was performed at room temperature for 1 hour. The resulting product was washed with 100 μL of TBS Tween-20 buffer (Thermo, #28360) three times. During washing, the liquid was removed first with a pipette, and excess buffer was removed by gently tapping the plate on a paper towel. Detection antibodies were prepared by dilution with assay buffer according to the manufacturer's manual. Human antibodies were prepared by diluting the detection antibodies 1/2000 using Goat anti-Human Fc-HRP (Novex, #A18817). 100 μL of the diluted detection antibodies were added to each well and incubated at room temperature for 1 hour. The resulting product was washed three times with 100 μL of TBS Tween-20 buffer (Thermo, #28360). During washing, the liquid was removed first with a pipette, and excess buffer was removed by gently tapping the plate on a paper towel. The resulting product was reacted with 100 μL of TMB substrate solution (Thermo, #N301) at room temperature for 30 minutes. The reaction was stopped using 100 μL of Stop solution (Thermo, N600). After stopping the reaction, absorbance was measured at 450 nm. The reaction was completed within 30 minutes after adding the stop solution. When bubbles were present, 100 to 150 μL was transferred to a new plate. Therefore, trastuzumab, a trastuzumab- and PBX-7014-conjugated ADC, and a trastuzumab- and PBX-7016-conjugated ADC were measured to obtain the results in FIG. 21. As shown in FIG. 21, when the binding strength of the ADC in which PBX-7014 or PBX-7016 was conjugated to trastuzumab to the Her2 antigen was compared with the binding strength of trastuzumab to the Her2 antigen at various concentrations, it was confirmed that there was no difference in binding strength.

**Example 7. Anti HER2 ADCs with PBX-payloads: *in vitro* potency and target selectivity**

For the ADC using each of Dxd, PBX-7014, and PBX-7016 as a payload, *in vitro* cell viability assays were performed on an MDA-MB-453 (HER2++) cell line, a FaDu (HER2+) cell line, and an MDA-MB-468 (HER2-) cell line in the same manner as Example 2-2.

Here, a HER2 targeting drug (Herceptin; generic name: trastuzumab), the Dxd drug, PBX-7014 (Preparation Example 2), PBX-7016 (Preparation Example 3), and their ADCs, Tra-25-4 and Tra-25-6 (Preparation Example 5), and Tra-Dxd (Enhertu®), were treated.

Three cell lines with different Her2 expression levels, MDA-MB-453 (Her2++), FaDu (Her2+), and MDA-MB-468 (Her2-), were treated with Enhertu (Tra-Dxd, DAR 8) as a reference, and incubated for 3 days to observe cell viability (FIG. 22).

As shown in FIG. 22, it was confirmed that anti Her2 ADCs with PBX-payloads have excellent target selectivity.

**Review: Evaluation of PBX-7014 and PBX-7016 as ADC payloads**

As shown in FIG. 22, it was confirmed that the ADC (Tra-25-6) containing PBX-7016 as a payload has an $IC_{50}$ value that was about 5 times better than Enhertu (Tra-Dxd) in Her2++ MDA-MB-453, and an equivalent $IC_{50}$ level in the negative cell line, MDA-MB-468. Thus, when the linker-payload systems of Chemical Formula 10 and Chemical Formula 11 are developed as ADCs, it was confirmed that they not only work by distinguishing between positive/negative cell lines, but also show superior efficacy compared to the reference, Enhertu.

**Review: Originality and excellence of PBX-7016**

Presented is a new camptothecin-based compound, PBX-7016, which is newly designed and synthesized from Chemical Formula 1 according to the present invention. PBX-7016 is a compound newly derived from the MD-CPT backbone FL118 and a new topoisomerase 1 inhibitor that degrades DDX5, like FL118 acting not only as a topoisomerase

1 inhibitor but also as a DDX5 degrader (FIGS. 11 to 14).

In addition, when simply comparing cell viability, PBX-7016 showed an $IC_{50}$ value, which was 1.5 to 2 times higher than that of Dxd, but surprisingly, when used as an ADC payload, it was confirmed that the PBX-7016-containing ADC has cell viability about 5 times better than the Dxd-containing ADC (Enhertu) (FIG. 22).

### Example 8. Anti HER2 ADCs with PBX-payloads: *in vivo* efficacy

#### 8-1. Cell culture

JIMT-1 breast cancer cells were cultured in DMEM containing 10% FBS, 100 U/mL of penicillin, and 100 μg/mL of streptomycin under 5% $CO_2$ and 37 °C conditions, and subcultured through trypsin-EDTA treatment by a common method twice a week. Cells growing in the exponential growth phase were harvested and counted for tumor inoculation.

#### 8-2. BALB/c nude mice

Both SCID mice and BALB/c nude mice are used as animal models to study the immune system and test treatments for treating various diseases.

Both SCID mice and BALB/c nude mice are immunocompromised, but the underlying mechanisms of their immune defects are different.

SCID mice are genetically engineered mice that lack functional T and B cells, resulting in a severely compromised immune system. They are used to study immune system development and function and to test potential treatments for diseases that affect the immune system, such as autoimmune disorders and certain types of cancer.

On the other hand, BALB/c nude mice are mice that lack a thymus and thus cannot produce T cells. They are also born without hair because of a defective hairless gene. These mice are used to study the role of T cells in immune responses and to test potential treatments for diseases affecting T cell function, such as certain types of cancer.

#### 8-3. Tumor inoculation and drug treatment

JIMT-1 tumor cells (5 x $10^6$) in 0.2 mL of DPBS with Matrigel were injected subcutaneously into the right anterior flank of each BALB/c nude mouse. When the average tumor volume reached about 215 $mm^3$, the animals were randomly grouped and then treatment for drug efficacy studies was initiated as follows.

Treatment**:** I.V, Q1W
Vehicle group: Vehicle,0 mg/kg, 5 μL/g,i.v., twice (day 0,day 6)
Isotype-Dxd(3) group: CTP63-Dxd, 3 mg/kg, 5 μL/g,i.v., twice (day 0,day 6)
Isotype-Dxd(10) group: CTP63-Dxd, 10 mg/kg, 5 μL/g,i.v., twice (day 0,day 6)
Isotype-PBX7016(3) group; CTP63-PBX7016, 3 mg/kg, 5 μL/g,i.v., twice (day 0,day 6)
Isotype-PBX7016(10) group: CTP63-PBX7016,10 mg/kg, 5 μL/g,i.v., twice (day 0,day 6)
Isotype-PBX7014(3) group: CTP63-PBX7014, 3 mg/kg, 5 μL/g,i.v., twice (day 0,day 6)
Isotype-PBX7014(10) group: CTP63-PBX7014,10 mg/kg, 5 μL/g,i.v., twice (day 0,day 6)
Trastuzumab-PBX7014(3) group: CTP6-PBX7014,3 mg/kg, 5 μL/g,i.v., twice (day 0,day 6)
Trastuzumab-PBX7014(10) group: CTP6-PBX7014, 10 mg/kg, 5 μL/g,i.v., twice (day 0,day 6)
Trastuzumab-PBX7016(3) group: CTP6-PBX7016, 3 mg/kg, 5 μL/g,i.v., twice (day 0,day 6)
Trastuzumab-PBX7016(10) group: CTP6-PBX7016, 10 mg/kg, 5 μL/g,i.v., twice (day 0,day 6)
Enhertu (3) group: Enhertu, 3 mg/kg, 5 μL/g,i.v., twice (day 0,day 6)
Enhertu (10) group: Enhertu, 10 mg/kg, 5 μL/g,i.v., twice (day 0,day 6)

#### 8-4. Tumor measurement and endpoint

The time of confirming whether tumor growth can be delayed or a mouse can be cured was determined as the main endpoint. The two-dimensional size of a tumor was measured twice a week using a caliper, and the volume was expressed in $mm^3$ using the formula ($V = 0.5 a \times b^2$, where a and b are the long diameter and short diameter of the tumor, respectively).

Tumor growth inhibition (TGI) for each group was calculated.

$$TGI (\%) = [1 - (T_i - T_0) / (V_i - V_0)] \times 100$$

($T_i$ is an average tumor volume of a treatment group on a given day, $T_0$ is an average tumor volume of a treatment group on day 1 of treatment, $V_i$ is an average tumor volume of the vehicle control on the same day as $T_i$, and $V_0$ is an average tumor volume of the vehicle group on day 1 of treatment).

Animals with a weight loss of more than 15% or a tumor volume of 3,000 $mm^3$ or more were euthanized as a humane endpoint.

**8-5. Statistical analysis**

The results were expressed as mean and standard deviation (mean ± SEM). Data was analyzed using a two-way RM ANOVA Dunnett's multiple comparison test using Graphpad Prism 6.0 software, and $p < 0.05$ was considered statistically significant.

Table 6 below shows the trend (effect vs. time) of tumor volume ($mm^3$) over time.

[Table 6]

| Group | 6+0 days | 6+3 days | 6+7 days | 6+10 days | 6+14 days |
|---|---|---|---|---|---|
| Vehicle group | 175.16 | 262.35 | 414.51 | 458.39 | 658.48 |
| Isotype-Dxd(3) group | 175.16 | 264.78 | 356.71 | 396.97 | 498.12 |
| Isotype-Dxd(10) group | 174.99 | 272.38 | 348.88 | 362.98 | 451.89 |
| Isotype-PBX7016(3) group | 175.73 | 277.19 | 418.38 | 462.90 | 530.39 |
| Isotype-PBX7016(10) group | 175.95 | 264.88 | 334.74 | 366.55 | 428.60 |
| Isotype-PBX7014(3) group | 175.46 | 252.90 | 335.66 | 388.03 | 468.99 |
| Isotype-PBX7014(10) group | 175.23 | 298.40 | 383.04 | 420.42 | 506.52 |
| Trastuzumab-PBX7014(3) group | 175.63 | 279.28 | 331.04 | 367.43 | 392.67 |
| Trastuzumab-PBX7014(10)group | 176.33 | 273.52 | 261.47 | 260.96 | 274.31 |
| Trastuzumab-PBX7016(3) group | 174.79 | 269.91 | 266.46 | 267.00 | 251.11 |
| Trastuzumab-PBX7016(10)group | 175.76 | 314.16 | 274.22 | 257.78 | 225.68 |
| Enhertu (3)group | 175.58 | 295.80 | 325.74 | 354.87 | 405.93 |
| Enhertu (10) group | 175.87 | 270.11 | 254.45 | 249.13 | 249.1.8 |

FIG. 23, which is a graphical representation of Table 6, shows the results of efficacy tests on ADCs with various camptothecin-based drugs as payloads in mice transplanted with a JIMT-1 cell line, that is, *in vivo* xenograft models.

**8-6. Body weight of JIMT-1 tumor model**

The body weight of the JIMT-1 tumor mouse model was monitored regularly (FIG. 24).

**Example 9: Anti HER2 ADCs with PBX-payload: Bystander effect study**

The bystander effect is one of the factors that significantly affect the efficacy of ADC drugs. To confirm the bystander effect, analysis was performed using FACS under 40 nM ADC treatment conditions.

To verify the efficacy and utility of an antibody-drug conjugate (ADC) in which a payload-linker binds to the trastuzumab antibody targeting the HER2 protein as an ADC drug when using the lead substance of the present invention, PBX-7016, as a payload, an *in vitro* bystander effect was confirmed through a flow cytometry analysis experiment. In this example, the bystander effect was defined as the induction of cell death by ADC treatment in a HER2-positive cell line, MDA-MB-453, and a drug (payload) released during the cell death exhibits toxicity in a surrounding HER2-negative cell line, leading to cell death.

In Preparation Example 5, to evaluate the *in vitro* bystander effect of the prepared ADC, the presence of the bystander effect caused by ADC treatment was evaluated under conditions where a HER2 expression-negative cell line and a HER2 expression-positive cell line were mixed and co-cultured at different ratios on a 6-well cell culture plate.

Specifically, after mixing and co-culturing GFP-MDA-MB-468 cells (3 x 10^5 cells) manufactured to express a green fluorescent protein (GFP) in a HER2 expression-negative cell line, MDA-MB-468, and aHER2 expression-positive cell line, i.e., MDA-MB-453 cells (1 x 10^5 cells), in a 3:1 ratio for 24 hours, the cell mixture was treated with each of 40 nM trastuzumab antibody, and five types of ADC samples (Trastuzumab-Dxd, Isotype IgG-Dxd, Trastuzumab-25-6, Isotype IgG-25-6, and Enhertu) and cultured for 6 days, and then among living cells, the GFP fluorescence and cell count of the HER2 expression-negative cell line, i.e., GFP-MDA-MB-468 cells, were checked through flow cytometry.

In addition, after mixing and co-culturing the HER2 expression-negative cell line, i.e., MDA-MB-468 cells (3 x 10^5 cells), and the HER2 expression-positive cell line, i.e., MDA-MB-453 cells (1 x 10^5 cells) in a 3:1 ratio for 24 hours on a 6-well cell culture plate, the cell mixture was treated with each of 40 nM trastuzumab antibody, and five types of ADC samples (Trastuzumab-Dxd, Isotype IgG-Dxd, Trastuzumab-25-6, Isotype IgG-25-6, and Enhertu) and cultured for 6 days. Then, after cell staining using an anti-HER2-fluorescein isothiocyanate (FITC) antibody that was designed to exhibit fluorescence by labeling a FITC fluorochrome dye to an antibody that can recognize the HER2 protein, among living cells, the FITC fluorescence and cell count of the HER2 expression-positive cell line, i.e., MDA-MB-453 cells, were checked through flow cytometry.

For flow cytometry, after the cells present in each well of the 6-well cell culture plate were harvested and diluted with a buffer (400 μL), GFP or FITC fluorescence was measured for each sample at the same time (1 min each) and same flow rate using FL1 laser.

As shown in FIGS. 25 and 26, from the results confirmed through flow cytometry, it was determined that, compared with Dxd-based ADCs, Trastuzumab-Dxd and Enhertu, the PBX-7016-based ADC, Trastuzumab-25-6 not only killed HER2 expression-positive cells but also simultaneously killed HER2 expression-negative cells, showing equivalent level of a bystander effect at the same level as the existing Dxd-based Enhertu. On the other hand, it was confirmed that negative control ADCs, Isotype IgG-Dxd and Isotype IgG-25-6, do not greatly induce HER2 expression-negative cell death, compared to non-ADC-treated conditions.

In brief, an anti HER2 ADC using PBX-7016 exhibited a bystander effect similar to Enhertu.

## Claims

1. An active camptothecin derivative represented by Chemical Formula 1 below, which is designed to bind to a DDX5 protein and an E3 ligase; a prodrug thereof designed to release the active camptothecin derivative at an *in vivo* target site; or a ligand-containing complex targeting the DDX5 protein, wherein the topoisomerase-1 inhibitory ability of the active camptothecin derivative or an FL11 8 compound of Chemical Formula 2 is inactivated through linker conjugation:

[Chemical Formula 1]

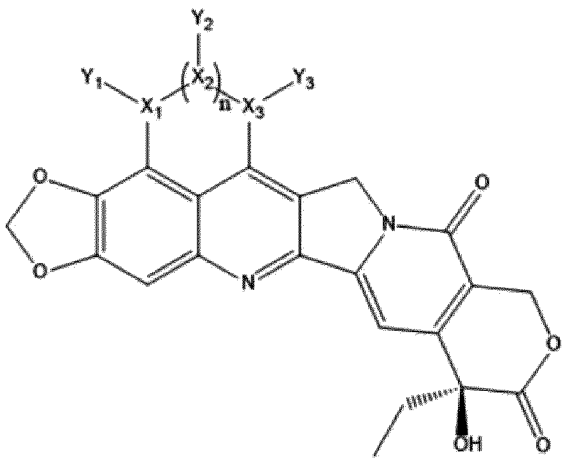

$X_1$ and $X_3$ are each independently carbon, oxygen, nitrogen, or sulfur, and are the same or different,
$X_2$ is carbon, oxygen, nitrogen, sulfur, a single bond, or a double bond,
$X_1$, $(X_2)_n$ and $X_3$ form a 6- or 7-membered ring (n= 1-2), and
$Y_1$, $Y_2$ and $Y_3$ are each independently hydrogen, or a functional group containing oxygen, nitrogen, phosphorus or sulfur.

2. The camptothecin derivative or prodrug of claim 1, wherein the active camptothecin derivative designed to bind to the DDX5 protein and an E3 ligase acts as a ligand (binder) that binds to the E3 ligase as a molecular glue.

3. The camptothecin derivative or prodrug of claim 1, wherein the active camptothecin derivative designed to bind to the DDX5 protein and an E3 ligase kills DDX5 protein-expressing target cells through a molecular glue mechanism of action (MoA).

4. The camptothecin derivative or prodrug of claim 1, wherein the active camptothecin derivative designed to bind to DDX5 protein and an E3 ligase has an MoA that inhibits topoisomerase-1 and degrades the oncoprotein DDX5.

5. The camptothecin derivative or prodrug of claim 4, wherein, in General Formula 1 or Chemical Formula 2, Group C optionally binds to topoisomerase-1 and Group A binds to DNA, such that the covalent bonding of the topoisomerase-DNA complex is stabilized, preventing the re-ligation of cleaved DNA fragments, and/or in General Formula 1 or Chemical Formula 2, Group A binds to DDX5 and Group C optionally binds to an E3 ligase, thereby inducing DDX5 degradation,

[General Formula 1]

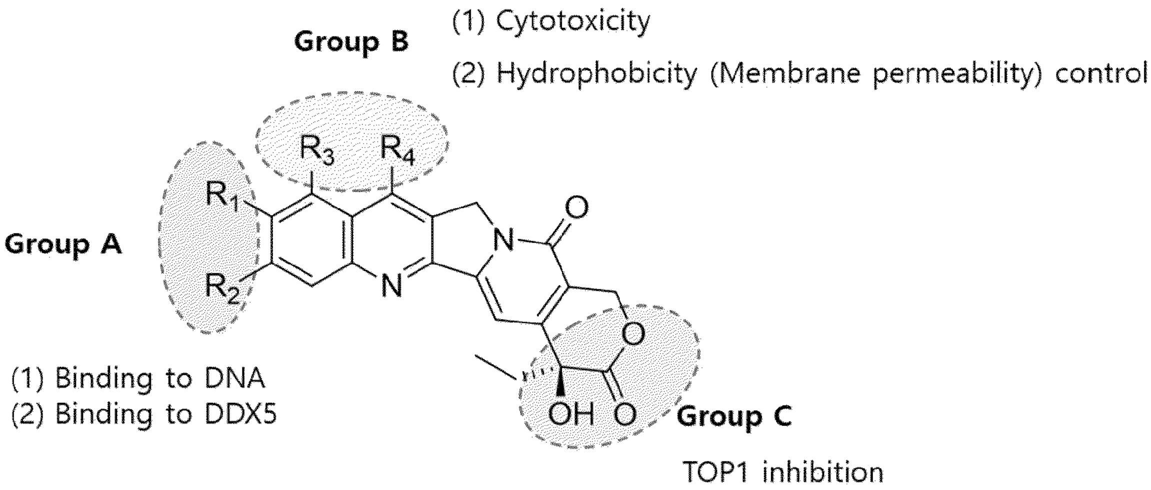


[Chemical Formula 2]

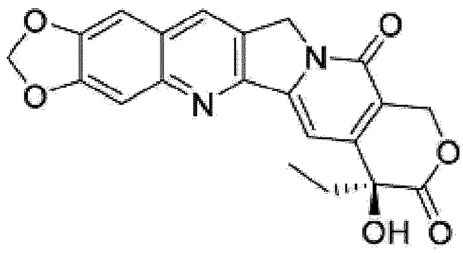

FL118

6. A pharmaceutical composition for preventing or treating cancer or a cancer diagnostic composition, comprising: the active camptothecin derivative, prodrug thereof, or ligand-containing complex targeting the DDX5 protein, according to any one of claims 1 to 5.

7. The pharmaceutical composition of claim 6, which is used as a first-line treatment after cancer diagnosis or administered to a subject (over)expressing DDX5 in cancer tissue.

8. The pharmaceutical composition of claim 6, which is administered to treat HER2-positive cancer, breast cancer, lung cancer, or colorectal cancer.

9. The pharmaceutical composition of claim 6, wherein the DDX5 protein is used as a biomarker for predicting drug sensitivity or tumor sensitivity.

**10.** The pharmaceutical composition of claim 6, wherein the DDX5 protein is a drug target of an active camptothecin derivative, so that drug resistance, resistance to target treatment, and/or resistance during treatment is avoided.

**11.** The pharmaceutical composition of claim 6, wherein the transcriptional induction of anti-apoptotic genes is blocked by the active camptothecin derivative.

**12.** The pharmaceutical composition of claim 6, wherein the transcription cofactor DDX5 protein is degraded by the active camptothecin derivative, thereby maintaining or improving the sensitivity of cancer cells to chemotherapy or radiotherapy.

**13.** The pharmaceutical composition of claim 6, which transforms a solid tumor agglomerated due to high cell density into scattered cancer with a low cell density, and/or a cold tumor with low immune activity into a hot tumor with high immune activity.

**14.** The pharmaceutical composition of claim 6, which is used to determine a patient group to be administered the active camptothecin derivative or prodrug thereof, or to predict a drug response, relapse rate and/or prognosis during chemotherapy.

**15.** A method of preparing an active camptothecin derivative represented by Chemical Formula 1 below designed to bind to a DDX5 protein and an E3 ligase; a prodrug thereof, which is designed to release the active camptothecin derivative at an *in vivo* target site; or a ligand-containing complex targeting the DDX5 protein, wherein a topoisomerase-1 inhibitory ability of the active camptothecin derivative or an FL118 compound of Chemical Formula 2 is inactivated through linker conjugation, comprising:

designing an active camptothecin derivative to include a camptothecin-based backbone represented by Chemical Formula 1 as a parent nucleus, selecting a compound designed in this way, or synthesizing a compound designed in this way,

[Chemical Formula 1]

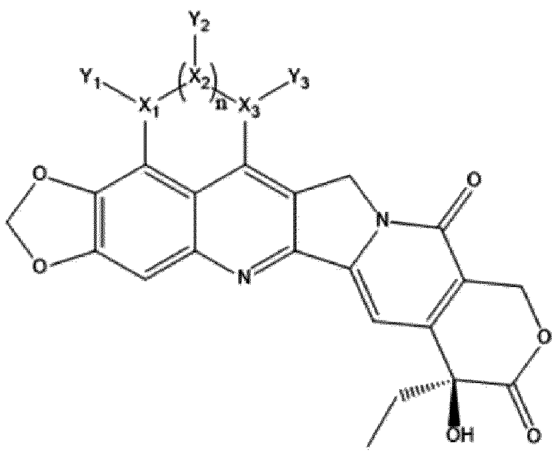

$X_1$ and $X_3$ are each independently carbon, oxygen, nitrogen, or sulfur, and are the same or different,
$X_2$ is carbon, oxygen, nitrogen, sulfur, a single bond, or a double bond,
$X_1$, $(X_2)_n$ and $X_3$ form a 6- or 7-membered ring (n= 1-2), and
$Y_1$, $Y_2$ and $Y_3$ are each independently hydrogen, or a functional group containing oxygen, nitrogen, phosphorus or sulfur.

**16.** The method of claim 15, wherein the active camptothecin derivative designed to bind to the DDX5 protein and the E3 ligase has an MoA that inhibits topoisomerase-1 and degrades the oncoprotein DDX5.

**17.** The method of claim 15, wherein the designing of the active camptothecin derivative to include the camptothecin-based backbone represented by Chemical Formula 1 as a parent nucleus, or the selecting of the compound designed in this way includes

(1) selecting an active camptothecin derivative designed to have a mechanism of action (MoA) that inhibits topoisomerase-1 and/or an MoA that degrades the oncoprotein DDX5 from a library of compounds including a camptothecin-based backbone represented by Chemical Formula 1 as a parent nucleus, and/or

(2) confirming whether the compounds comprising the camptothecin-based backbone represented by Chemical Formula 1 as a parent nucleus inhibit topoisomerase-1 and/or degrade the oncoprotein DDX5 through an *in vitro* and/or *in vivo* experiment(s).

**18.** The method of claim 15, wherein the active camptothecin derivative is designed exhibit a bystander effect in cancer tissue or control the degree of the effect by adjusting cell membrane permeability as desired through $X_1$, $(X_2)_n$, $X_3$, $Y_1$, $Y_2$ and/or $Y_3$ modifications.

**19.** The method of claim 15, wherein the active camptothecin derivative or prodrug thereof is administered to a patient group in which DDX5 acts as an oncoprotein in cells.

**20.** The method of claim 15, further comprising:
confirming a patient group to be administered the active camptothecin derivative or prodrug thereof by quantitative or qualitative analysis of the DDX5 protein in cells using the ligand-containing complex targeting the DDX5 protein, in which the topoisomerase-1 inhibitory ability of the FL118 compound of Chemical Formula 2 or the active camptothecin derivative is inactivated through linker conjugation, in tissue biopsy or liquid biopsy.

**21.** A method of preparing an active camptothecin derivative, which binds to DDX5 acting as an oncoprotein in cells to induce cell death through DDX5 protein degradation, a prodrug thereof, which is designed to release the active camptothecin derivative *in vivo,* or a ligand-containing complex targeting the DDX5 protein, wherein a topoisomerase-1 inhibitory ability of the active camptothecin derivative is inactivated through linker conjugation, comprising:

a first step of selecting the active camptothecin derivative from the group consisting of a compound of Chemical Formula 3, a compound of Chemical Formula 4, a compound of Chemical Formula 5, a compound of Chemical Formula 6, a compound of Chemical Formula 7, a compound of Chemical Formula 8, a compound of Chemical Formula 9, and isomers thereof, which are shown below; and

a second step of optionally selecting a prodrug thereof, which is designed to release the active camptothecin derivative selected in Step 1 *in vivo,*

[Chemical Formula 3]

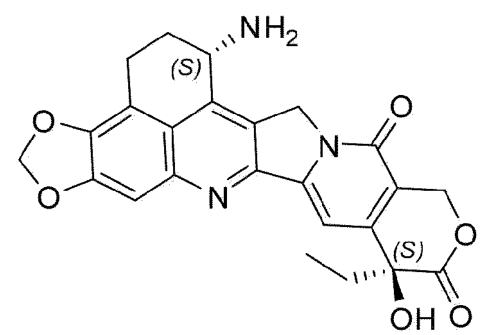

[Chemical Formula 4]

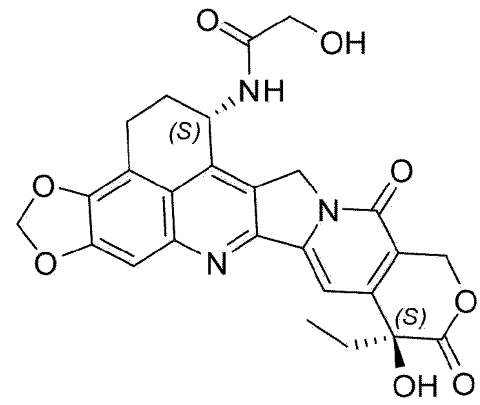

[Chemical Formula 5]

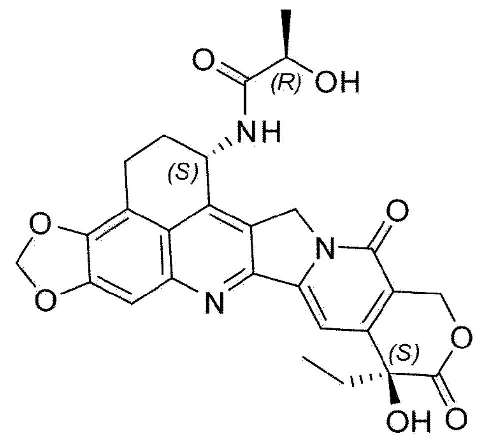

[Chemical Formula 6]

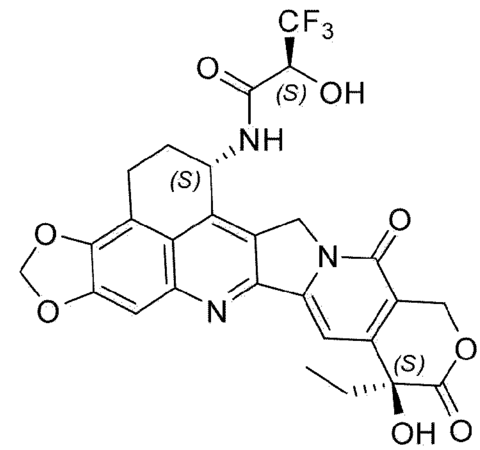

[Chemical Formula 7]

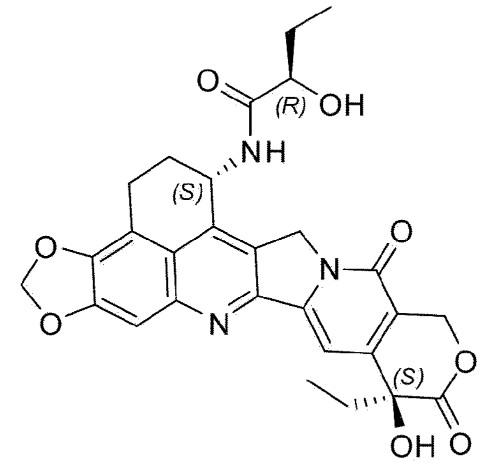

[Chemical Formula 8]

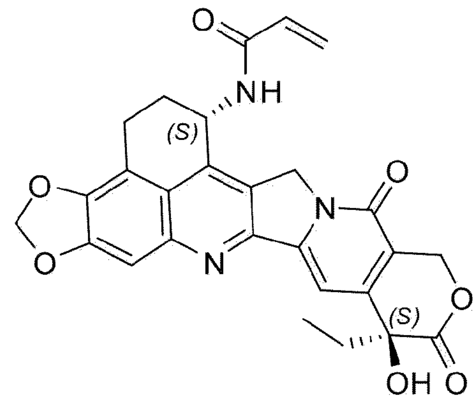

[Chemical Formula 9]

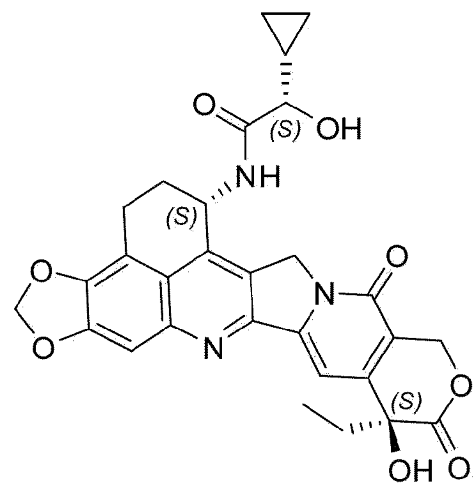

.

**22.** The method of claim 21, wherein the active camptothecin derivative or prodrug thereof is administered to a patient group in which DDX5 in cells acts as an oncoprotein.

**23.** The method of claim 21, further comprising:
confirming a patient group to be administered the active camptothecin derivative or prodrug thereof by quantitative or qualitative analysis of the DDX5 protein in cells using the ligand-containing complex targeting the DDX5 protein, in which the topoisomerase-1 inhibitory ability of FL118 compound of Chemical Formula 2 or the active camptothecin derivative is inactivated through linker conjugation, in tissue biopsy or liquid biopsy.

**24.** A compound represented by Chemical Formula 3, Chemical Formula 3-1, Chemical Formula 4, Chemical Formula 4-1, Chemical Formula 5, Chemical Formula 5-1, Chemical Formula 6, Chemical Formula 7, Chemical Formula 8, or Chemical Formula 9 below, or a pharmaceutically acceptable salt thereof:

[Chemical Formula 3]

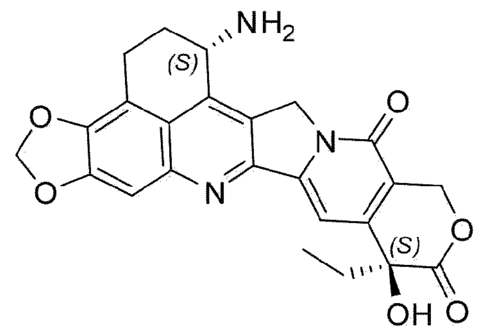

[Chemical Formula 3-1]

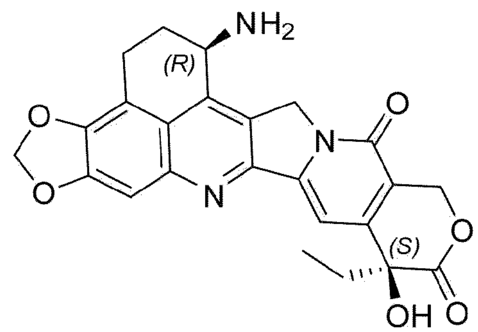

[Chemical Formula 4]

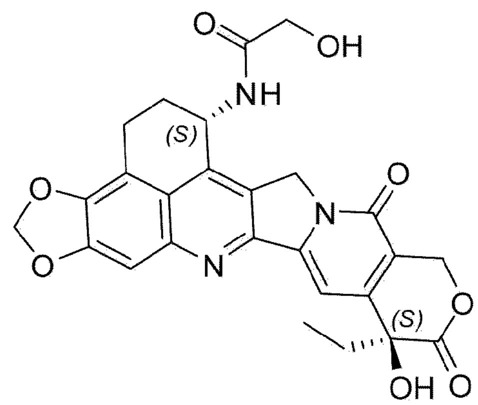

[Chemical Formula 4-1]

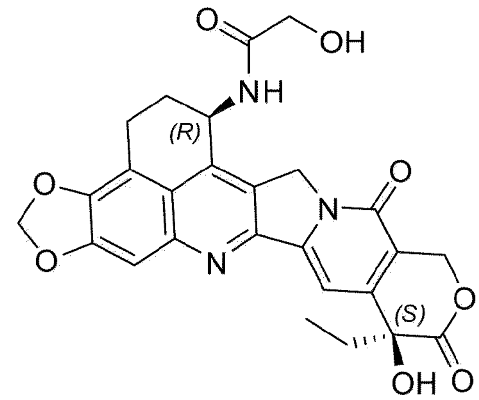

[Chemical Formula 5]

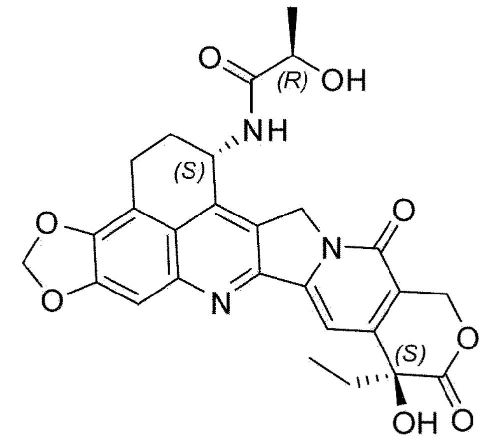

[Chemical Formula 5-1]

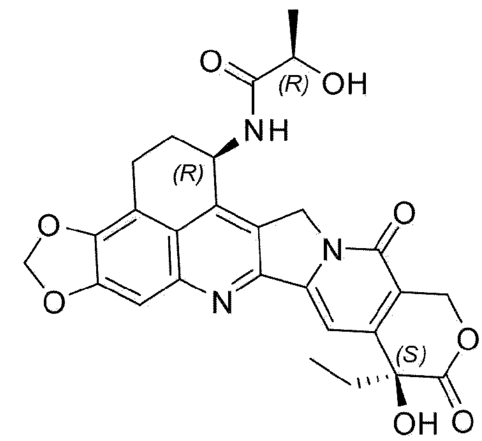

[Chemical Formula 6]

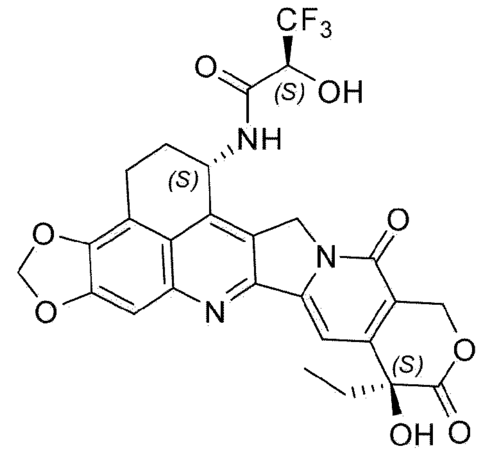

[Chemical Formula 7]

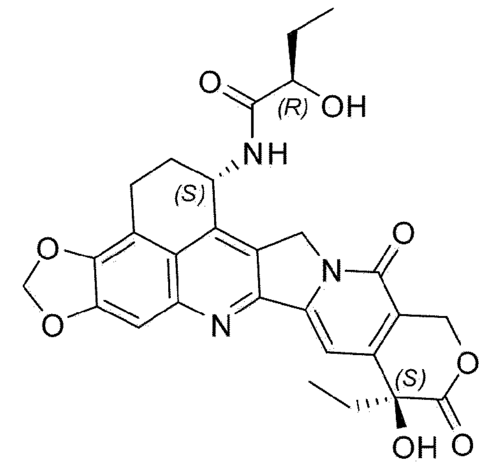

[Chemical Formula 8]

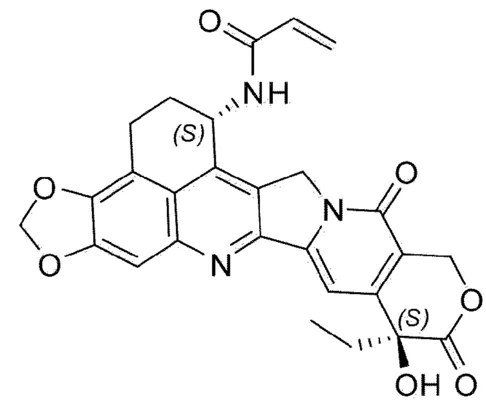

[Chemical Formula 9]

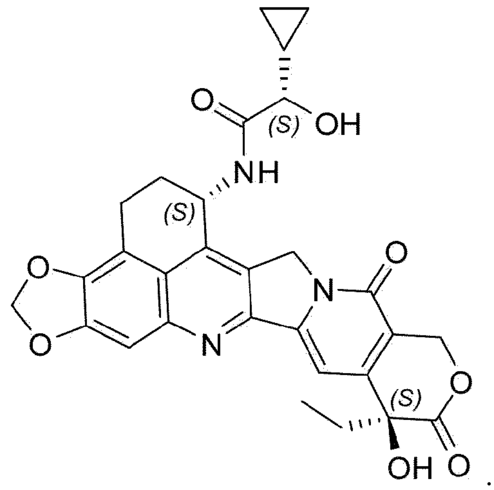

25. A pharmaceutical composition for preventing or treating cancer, comprising a compound represented by Chemical Formula 3, Chemical Formula 3-1, Chemical Formula 4, Chemical Formula 4-1, Chemical Formula 5, Chemical Formula 5-1, Chemical Formula 6, Chemical Formula 7, Chemical Formula 8, or Chemical Formula 9 below, a pharmaceutically acceptable salt thereof, a solvate thereof, or a prodrug thereof:

[Chemical Formula 3]

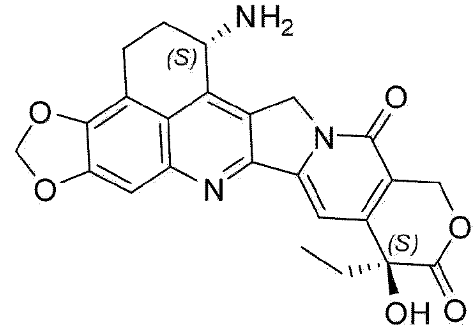

[Chemical Formula 3-1]

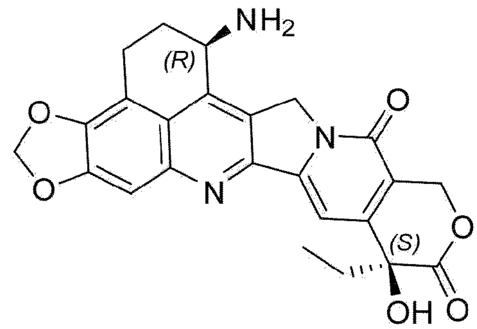

[Chemical Formula 4]

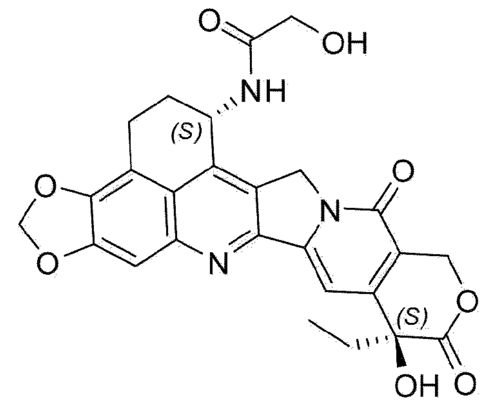

[Chemical Formula 4-1]

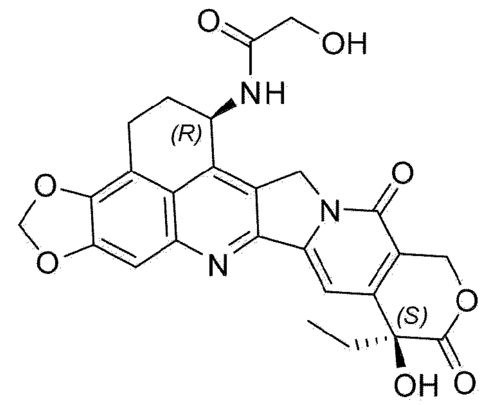

[Chemical Formula 5]

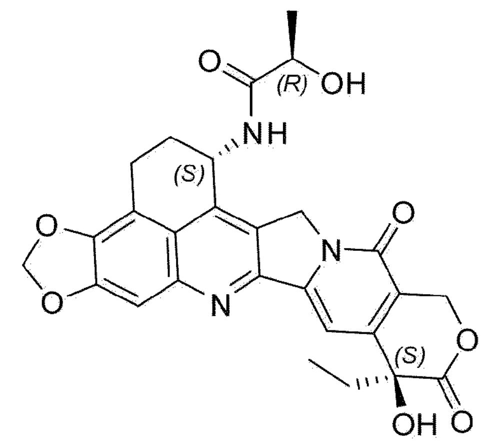

[Chemical Formula 5-1]

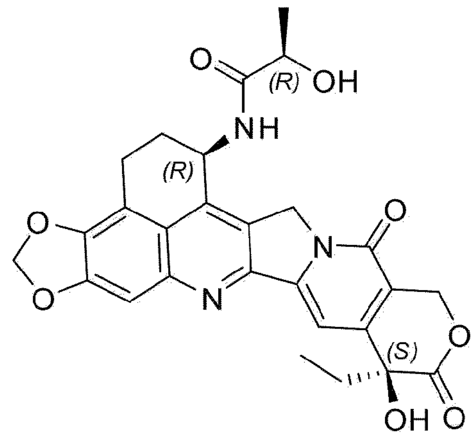

[Chemical Formula 6]

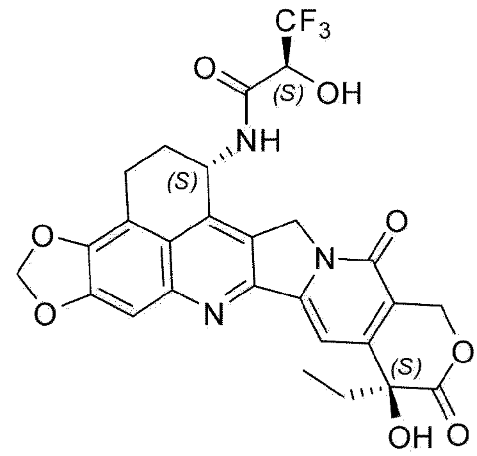

[Chemical Formula 7]

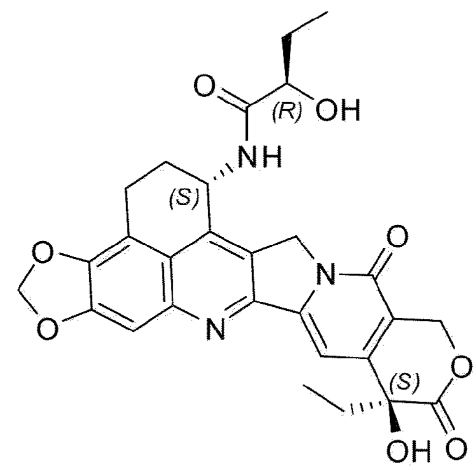

[Chemical Formula 8]

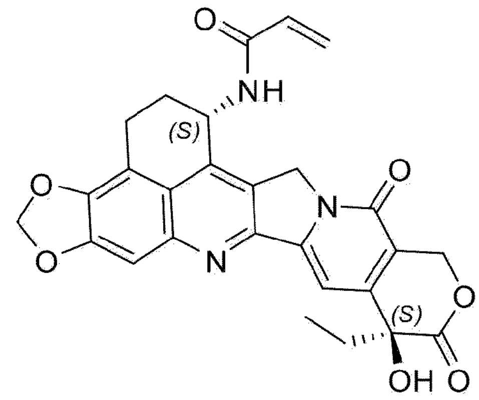

[Chemical Formula 9]

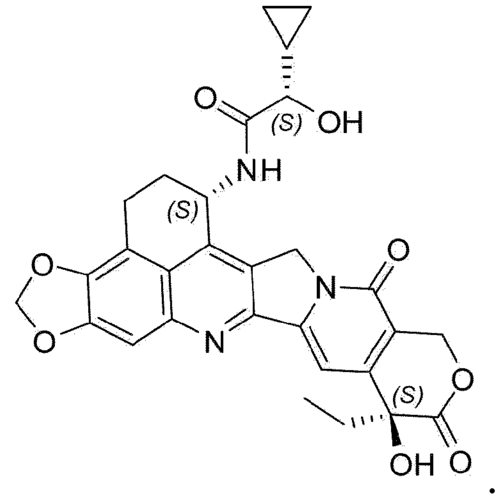

26. A pharmaceutical composition for killing cells, comprising:

an active camptothecin derivative represented by Chemical Formula 1 below, which is designed to bind to a DDX5 protein and an E3 ligase; or a prodrug thereof designed to release the active camptothecin derivative at a target site *in vivo*:

[Chemical Formula 1]

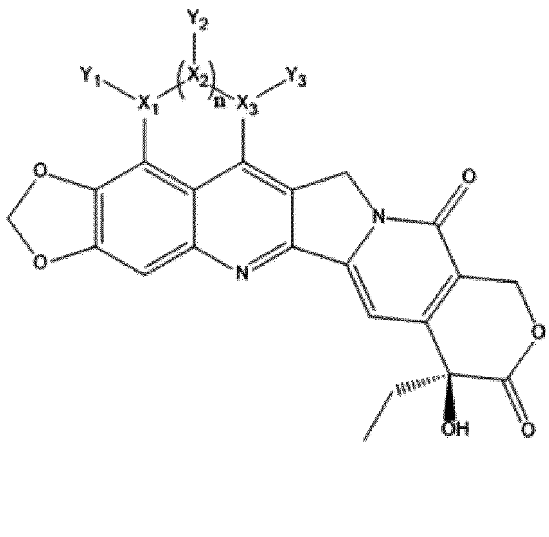

$X_1$ and $X_3$ are each independently carbon, oxygen, nitrogen, or sulfur, and are the same or different,
$X_2$ is carbon, oxygen, nitrogen, sulfur, a single bond, or a double bond,
$X_1$, $(X_2)_n$ and $X_3$ form a 6- or 7-membered ring (n= 1-2), and
$Y_1$, $Y_2$ and $Y_3$ are each independently hydrogen, or a functional group containing oxygen, nitrogen, phosphorus or sulfur.

**27.** The pharmaceutical composition of claim 26, wherein the active camptothecin derivative designed to bind to the DDX5 protein and an E3 ligase kills target cells expressing the DDX5 protein through a molecular glue mechanism of action (MoA).

**28.** The pharmaceutical composition of claim 26, wherein the active camptothecin derivative designed to bind to the DDX5 protein and an E3 ligase has an MoA that inhibits topoisomerase-1 and degrades the oncoprotein DDX5.

**29.** The pharmaceutical composition of claim 26, wherein the active camptothecin derivative is designed to exhibit a bystander effect in cancer tissue or control the degree of the effect by adjusting cell membrane permeability as desired through $X_1$, $(X_2)_n$, $X_3$, $Y_1$, $Y_2$ and/or $Y_3$ modifications.

**30.** The pharmaceutical composition of claim 27, wherein the target cells are cancer cells or senescent cells.

**31.** An anticancer formulation, which is administered in a dose that an active camptothecin derivative represented by Chemical Formula 1 below, which is designed to bind to the DDX5 protein and an E3 ligase; or a prodrug thereof, which is designed to release the active camptothecin derivative at a target site *in vivo,* stimulates antigen-presenting cells (APCs) through the cell death of cancer cells to induce an antitumor immune response:

[Chemical Formula 1]

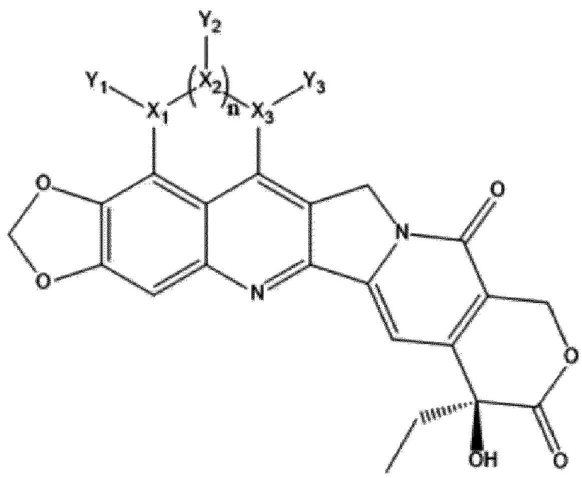

$X_1$ and $X_3$ are each independently carbon, oxygen, nitrogen, or sulfur, and are the same or different,
$X_2$ is carbon, oxygen, nitrogen, sulfur, a single bond, or a double bond,
$X_1$, $(X_2)_n$ and $X_3$ form a 6- or 7-membered ring (n= 1-2), and
$Y_1$, $Y_2$ and $Y_3$ are each independently hydrogen, or a functional group containing oxygen, nitrogen, phosphorus or sulfur.

**32.** The anticancer formulation of claim 31, which is designed to target tumor tissue.

**33.** A method of preparing an active camptothecin derivative-based anticancer agent, in which the bystander effect and/or ADME profile of the active camptothecin derivative are/is controlled to avoid T cell exhaustion due to chronic antigen exposure,

wherein the active camptothecin derivative-based anticancer agent is an anticancer formulation, which includes an active camptothecin derivative designed to bind to a DDX5 protein and an E3 ligase as a molecular glue; or a prodrug thereof designed to release the active camptothecin derivative at a target site *in vivo,* and

the method comprises designing the active camptothecin derivative or prodrug thereof to include the camptothecin-based backbone represented by Chemical Formula 1 as a parent nucleus, selecting a compound designed in this way, or synthesizing a compound designed in this way:

[Chemical Formula 1]

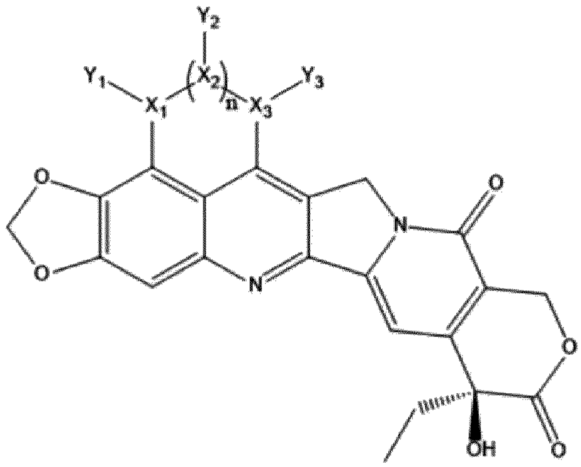

$X_1$ and $X_3$ are each independently carbon, oxygen, nitrogen, or sulfur, and are the same or different,
$X_2$ is carbon, oxygen, nitrogen, sulfur, a single bond, or a double bond,
$X_1$, $(X_2)_n$ and $X_3$ form a 6- or 7-membered ring (n= 1-2), and
$Y_1$, $Y_2$ and $Y_3$ are each independently hydrogen, or a functional group containing oxygen, nitrogen, phosphorus or sulfur.

**34.** The method of claim 33, wherein the active camptothecin derivative-based anticancer agent is a formulation that includes an active camptothecin derivative designed to have a mechanism of action that inhibits topoisomerase-1 and degrades the oncoprotein DDX5, or a prodrug thereof designed to release the active camptothecin derivative *in vivo.*

[FIG. 1]

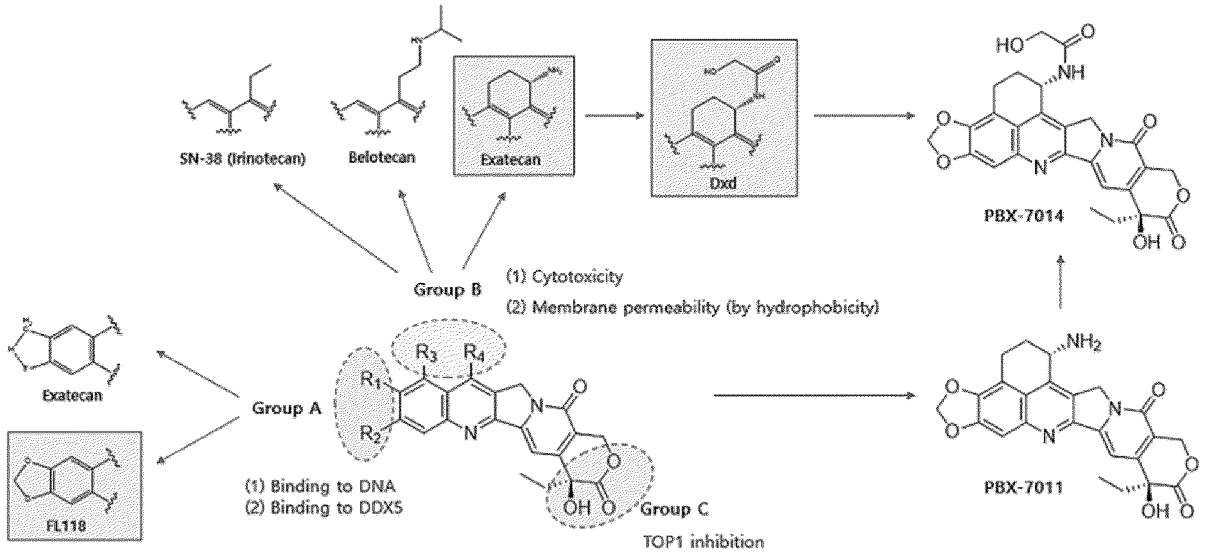


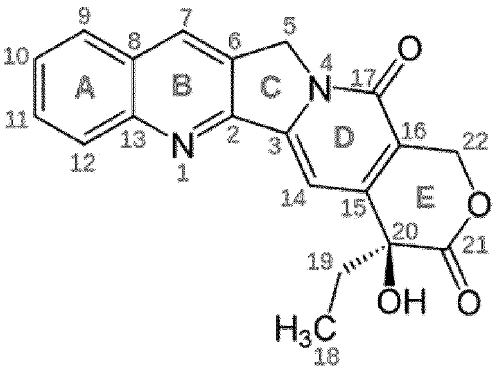

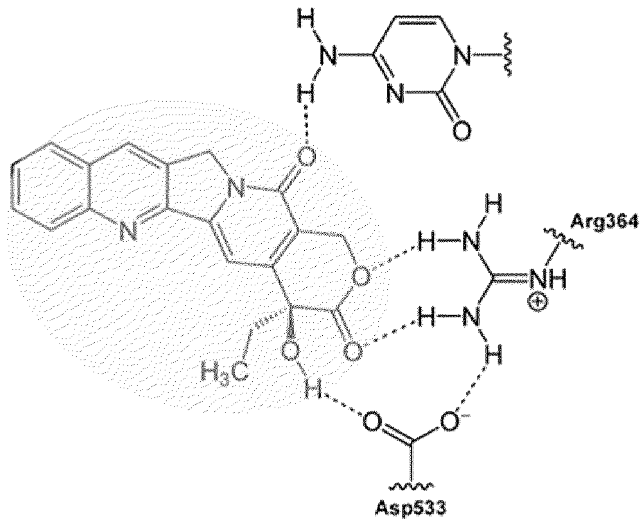


Binding of CPT to topoisomerase I

[FIG. 2]

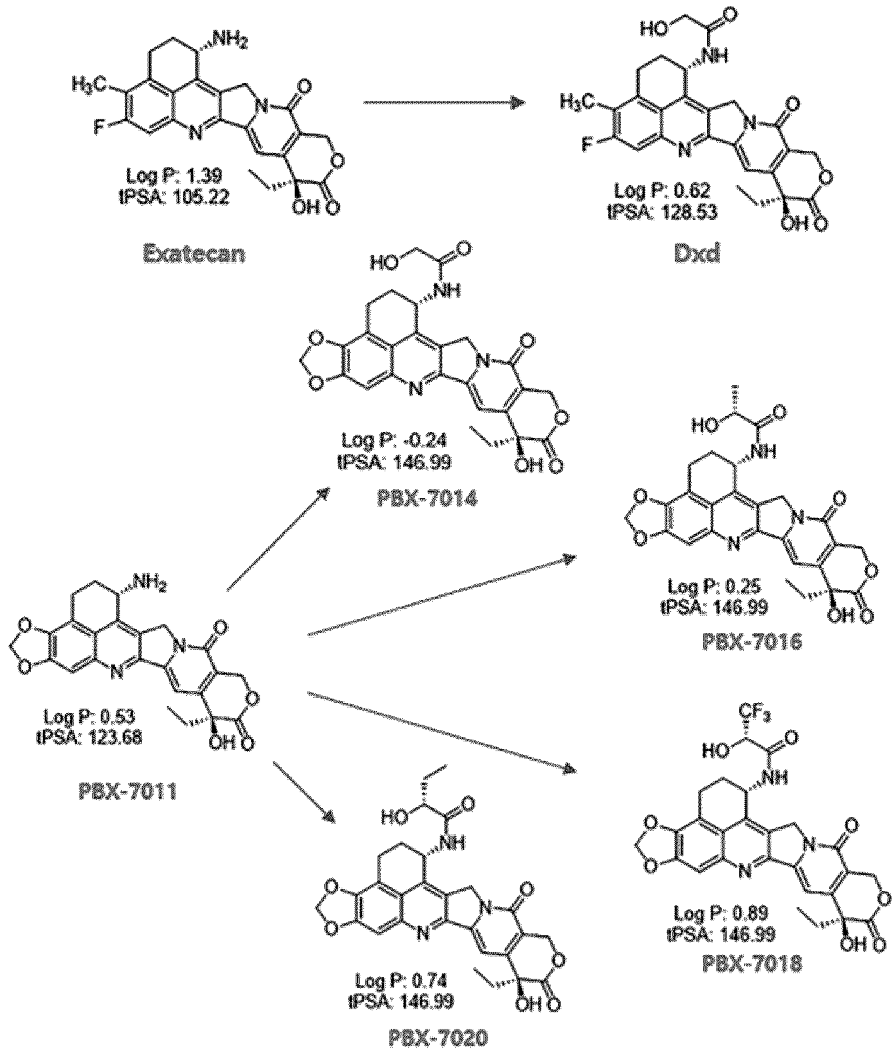


| Compound | LogP | tPSA | Remarks |
|---|---|---|---|
| Exatecan | 1.39 | 105.22 | |
| **Dxd** | **0.62** | **128.53** | Exatecan + Glycolic acid |
| PBX-7011 | 0.53 | 123.68 | |
| **PBX-7014** | -0.24 | 146.99 | PBX-7011 + Glycolic acid |
| **PBX-7016** | **0.25** | **146.99** | |
| PBX-7018 | 0.89 | 146.99 | |
| PBX-7020 | **0.74** | **146.99** | |

* LogP and tPSA values were calculated by ChemDraw based on 2D structures

[FIG. 3]

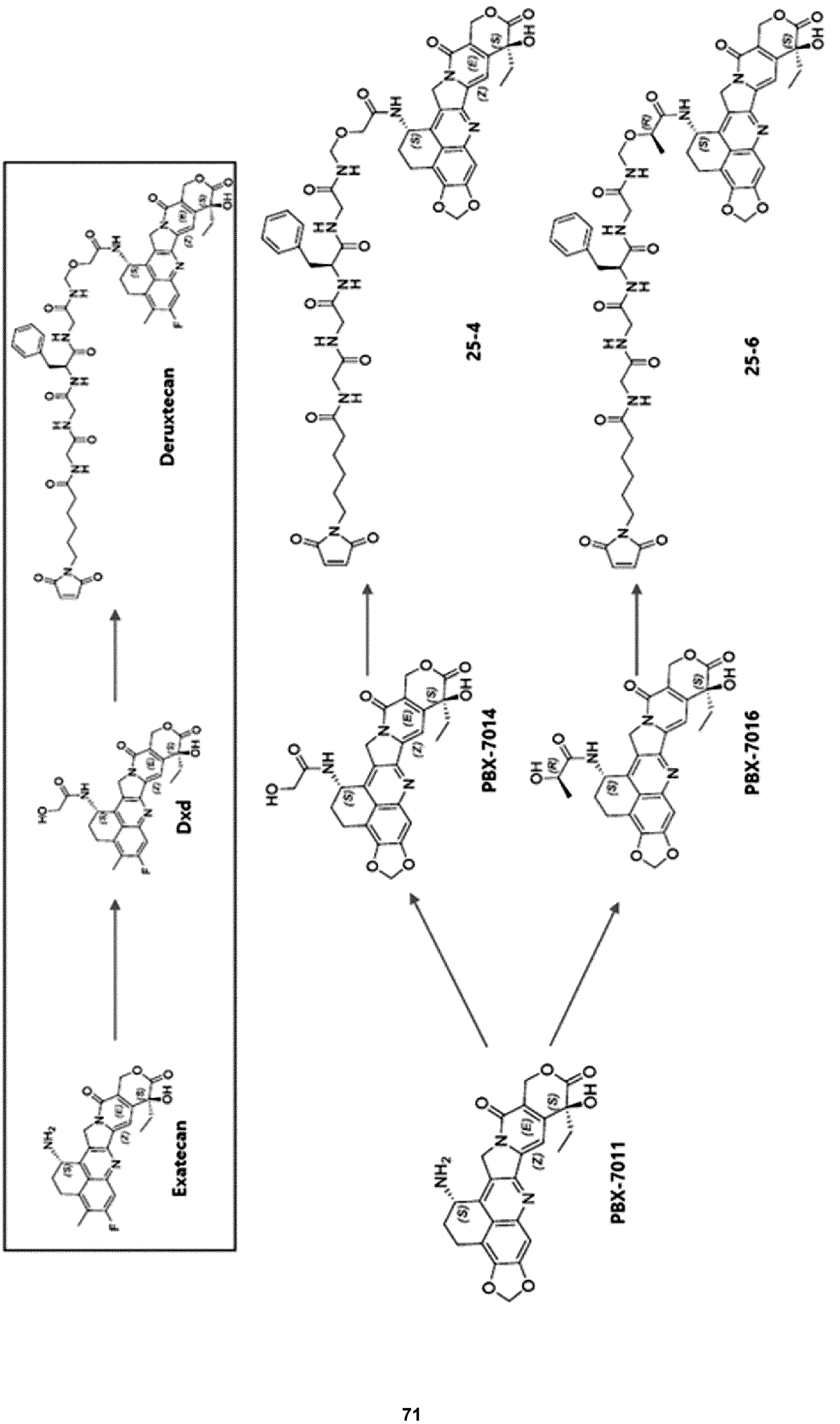

Exatecan

Dxd

Deruxtecan

PBX-7011

PBX-7014

25-4

PBX-7016

25-6

[FIG. 4]

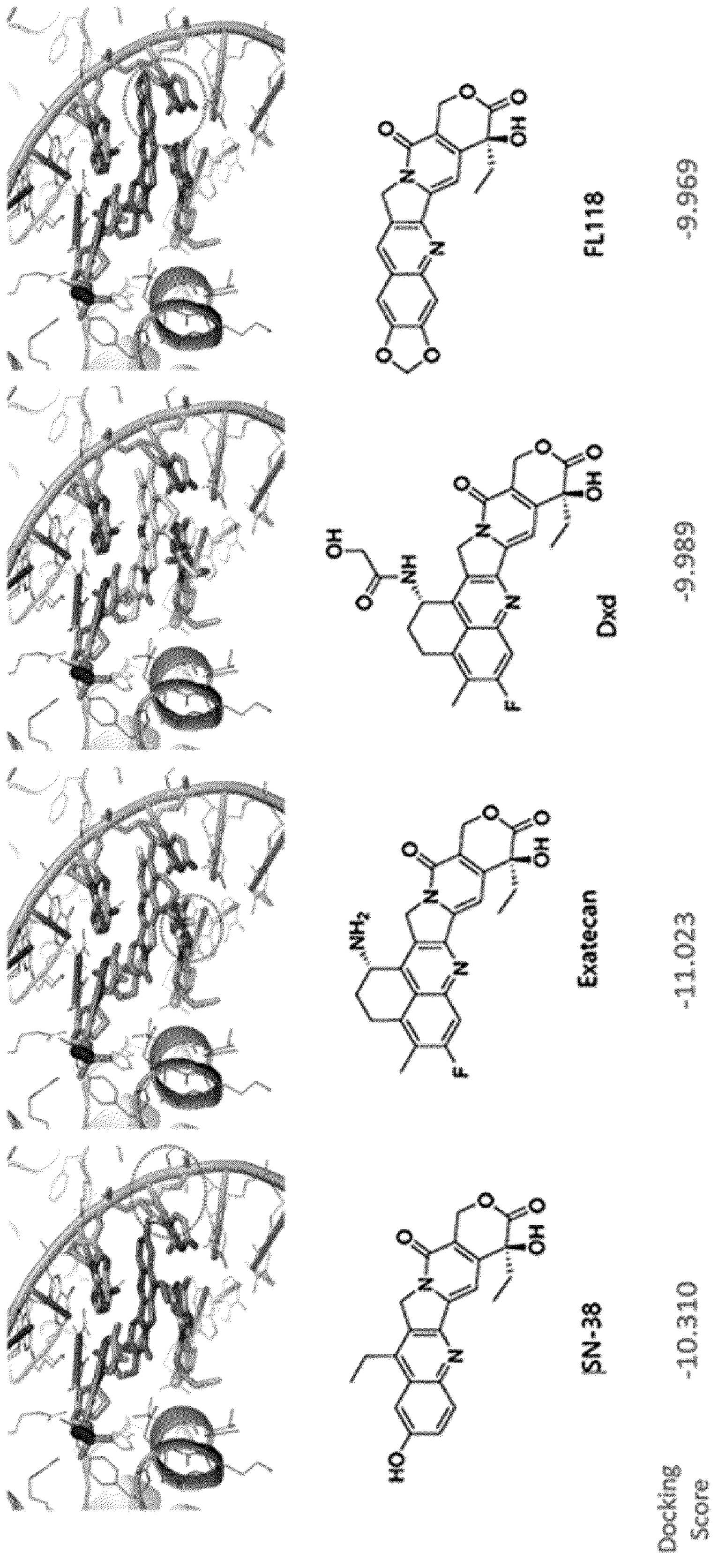
FL118
-9.969
Dxd
-9.989
Exatecan
-11.023
SN-38
-10.310
Docking Score

[FIG. 5]

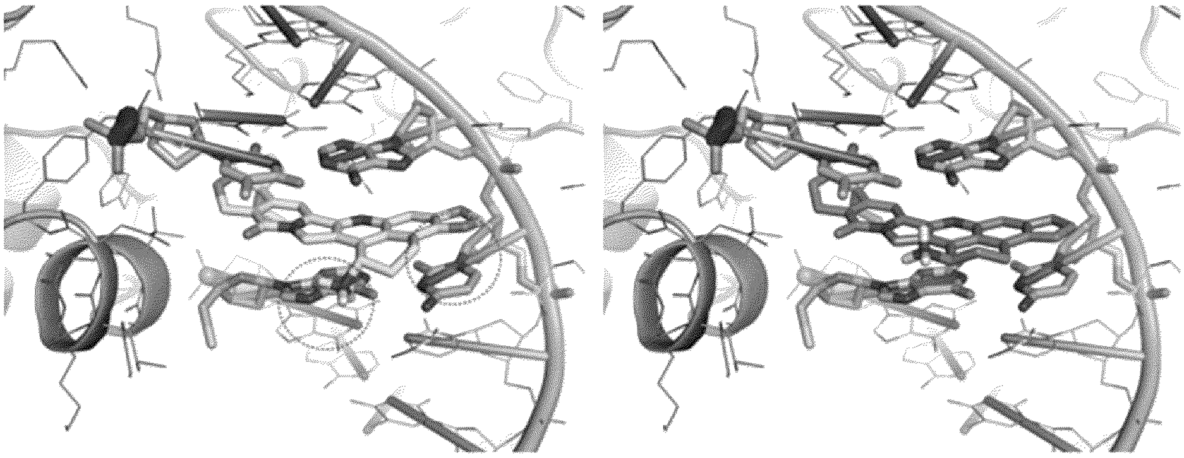

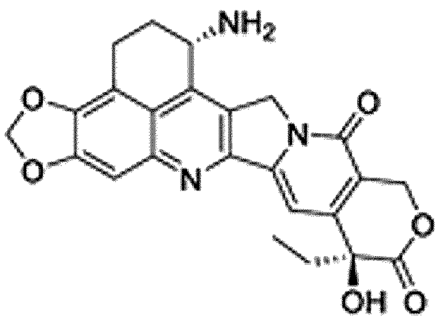

Hybrid-1 (isomer A)

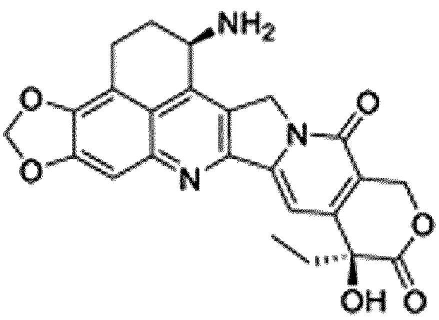

Hybrid-1 (isomer B)

| | Hybrid-1 (isomer A) | Hybrid-1 (isomer B) |
|---|---|---|
| Docking Score | -11.328 | -10.358 |

[FIG. 6]

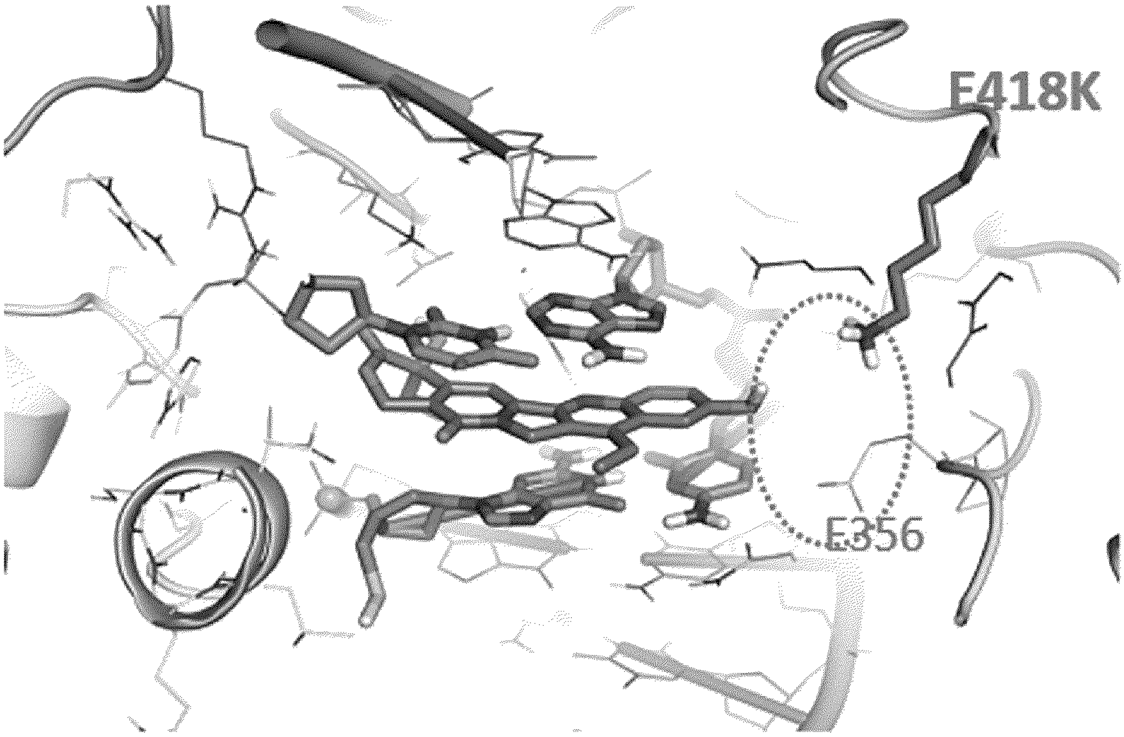

[FIG. 7]

# SN38-TOP1 INTERACTION IN NORMAL TOP1

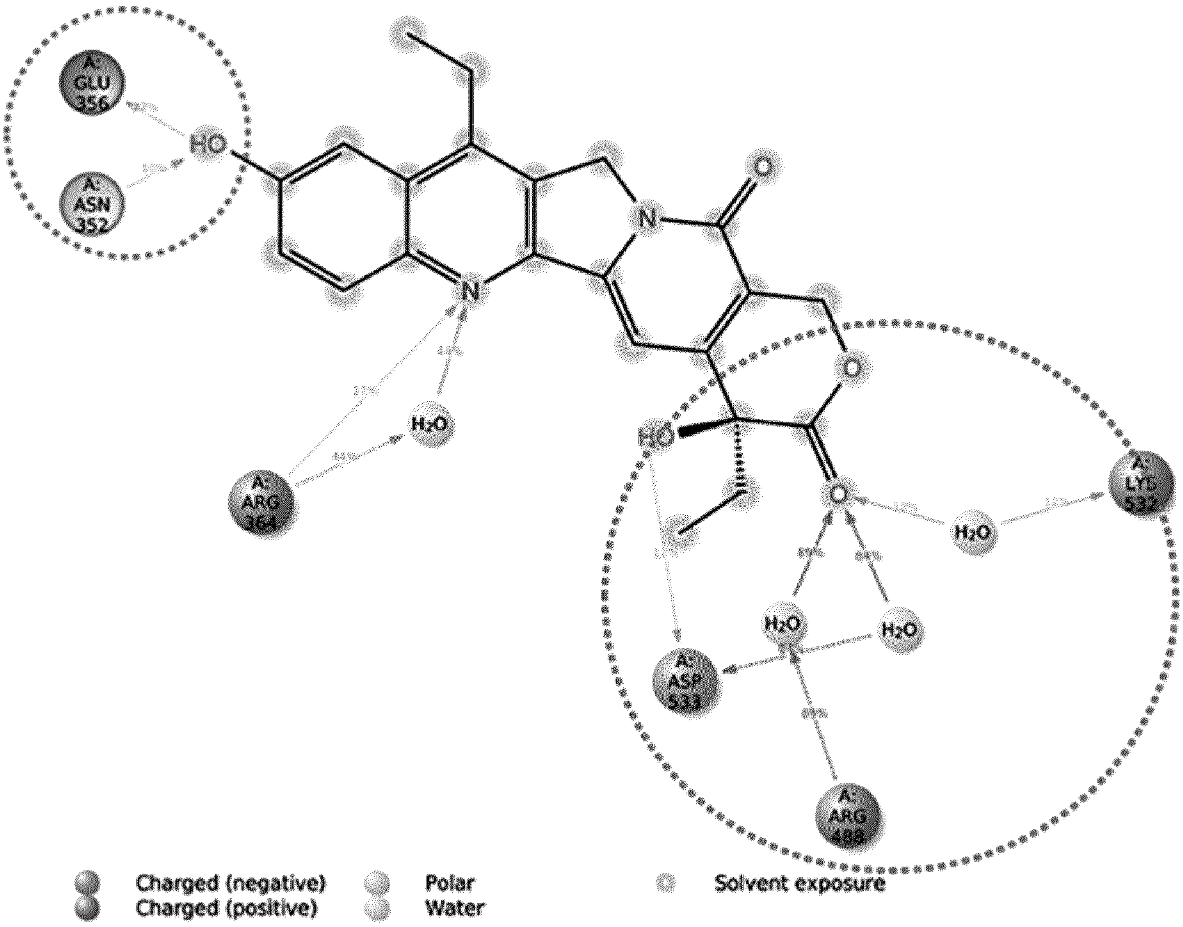


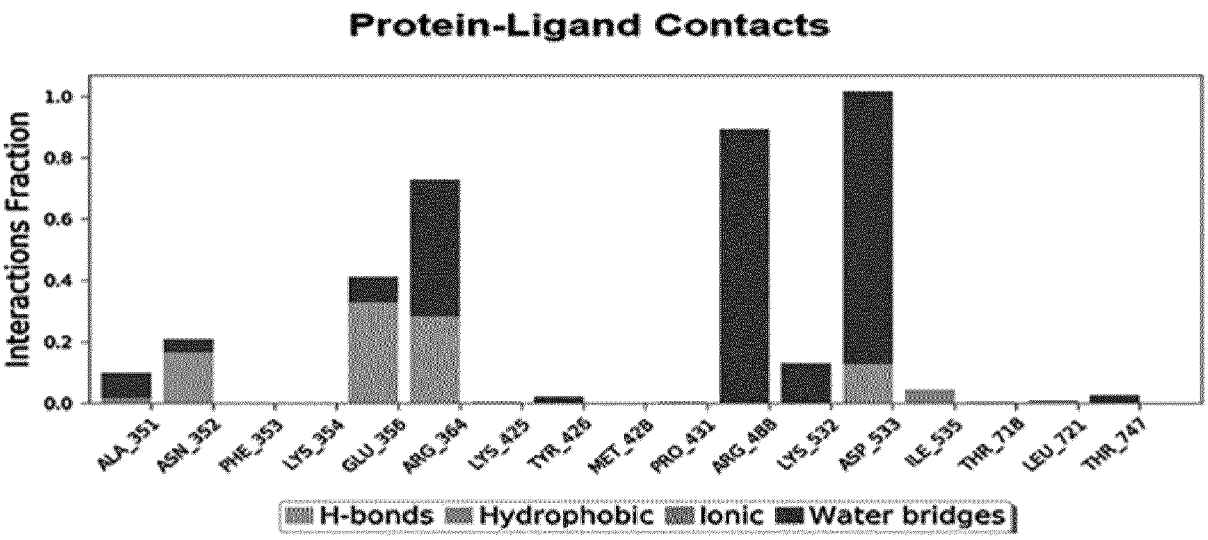

[FIG. 8]

# SN38-TOP1 INTERACTION IN E418K MUTATED TOP1

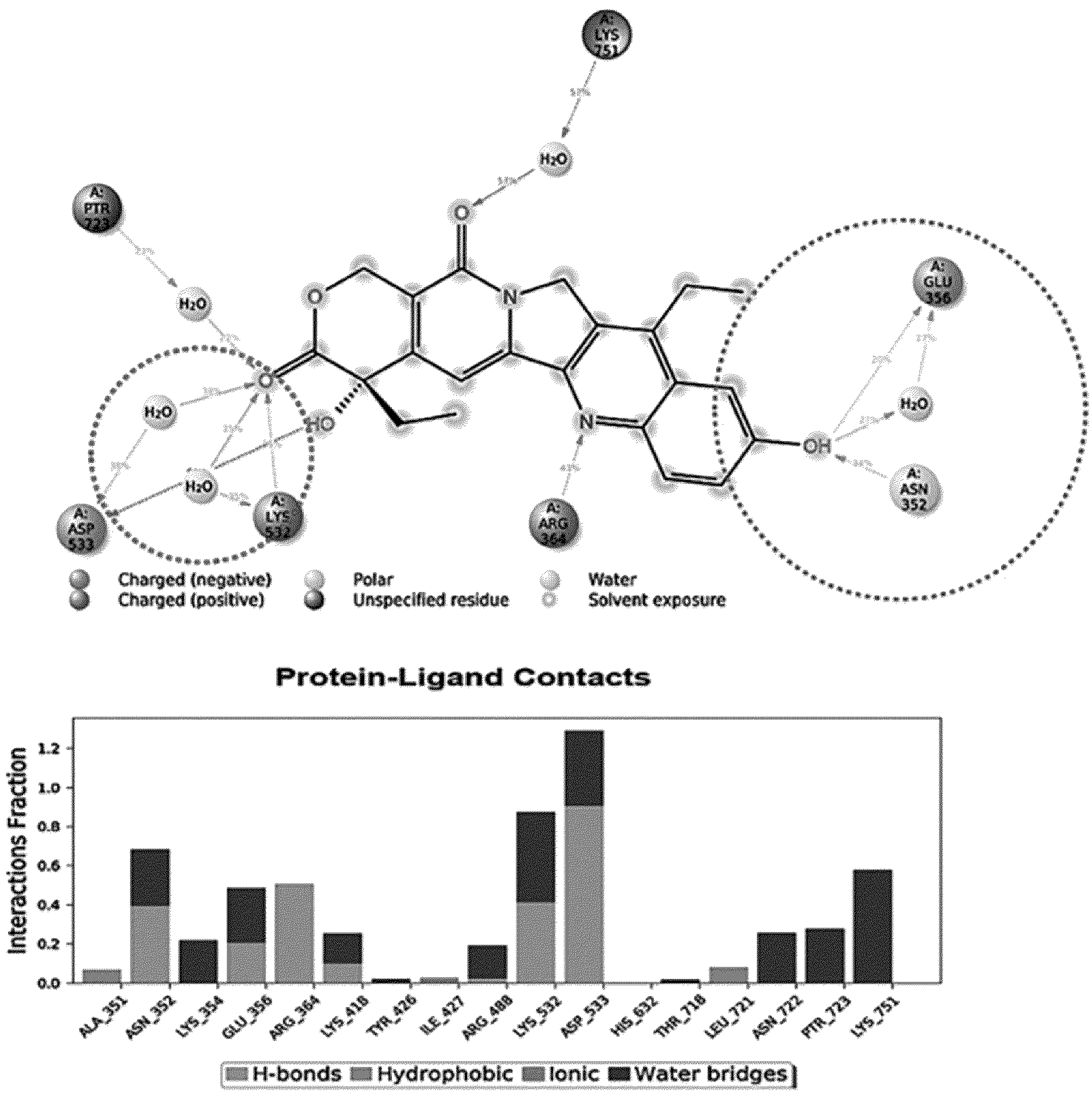

[FIG. 9]

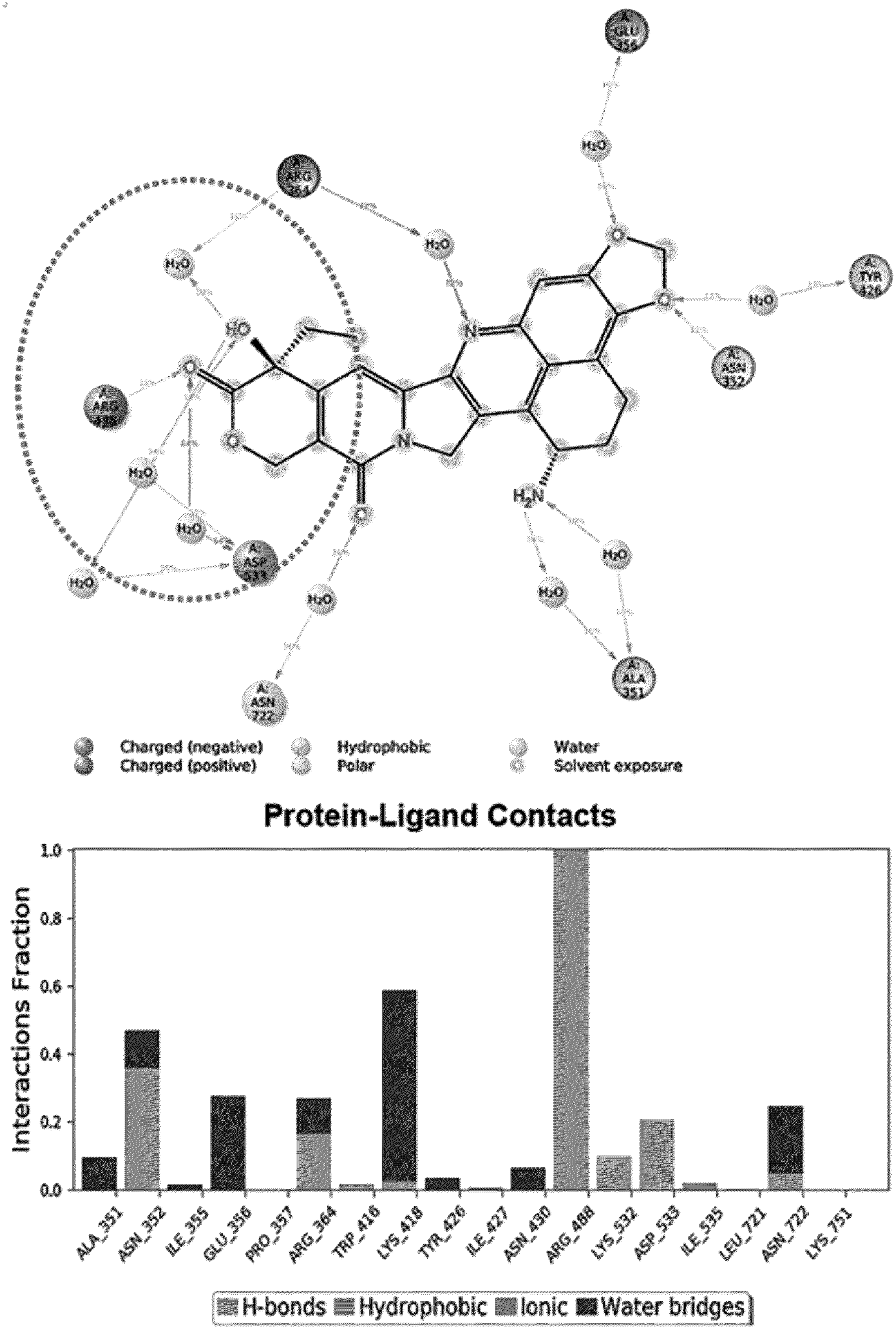
Charged (negative)
Charged (positive)
Hydrophobic
Polar
Water
Solvent exposure
Protein-Ligand Contacts
Interactions Fraction
1.0
0.8
0.6
0.4
0.2
0.0
ALA_351
ASN_352
ILE_355
GLU_356
PRO_357
ARG_364
TRP_416
LYS_418
TYR_426
ILE_427
ASN_430
ARG_488
LYS_532
ASP_533
ILE_535
LEU_721
ASN_722
LYS_751
H-bonds
Hydrophobic
Ionic
Water bridges

[FIG. 10]

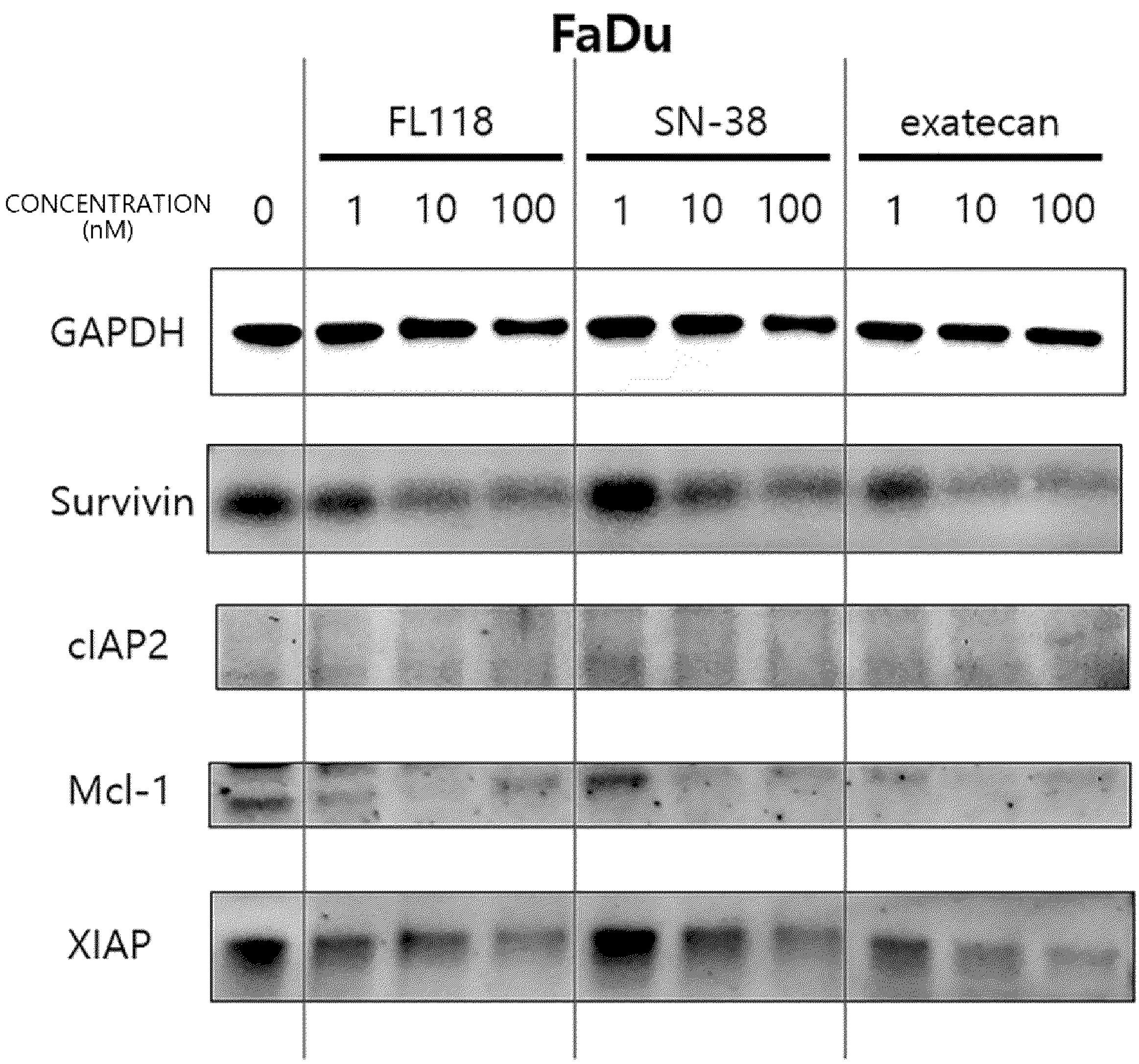
FaDu
FL118
SN-38
exatecan
CONCENTRATION (nM)
0
1 10 100
1 10 100
1 10 100
GAPDH
Survivin
cIAP2
Mcl-1
XIAP

[FIG. 11]

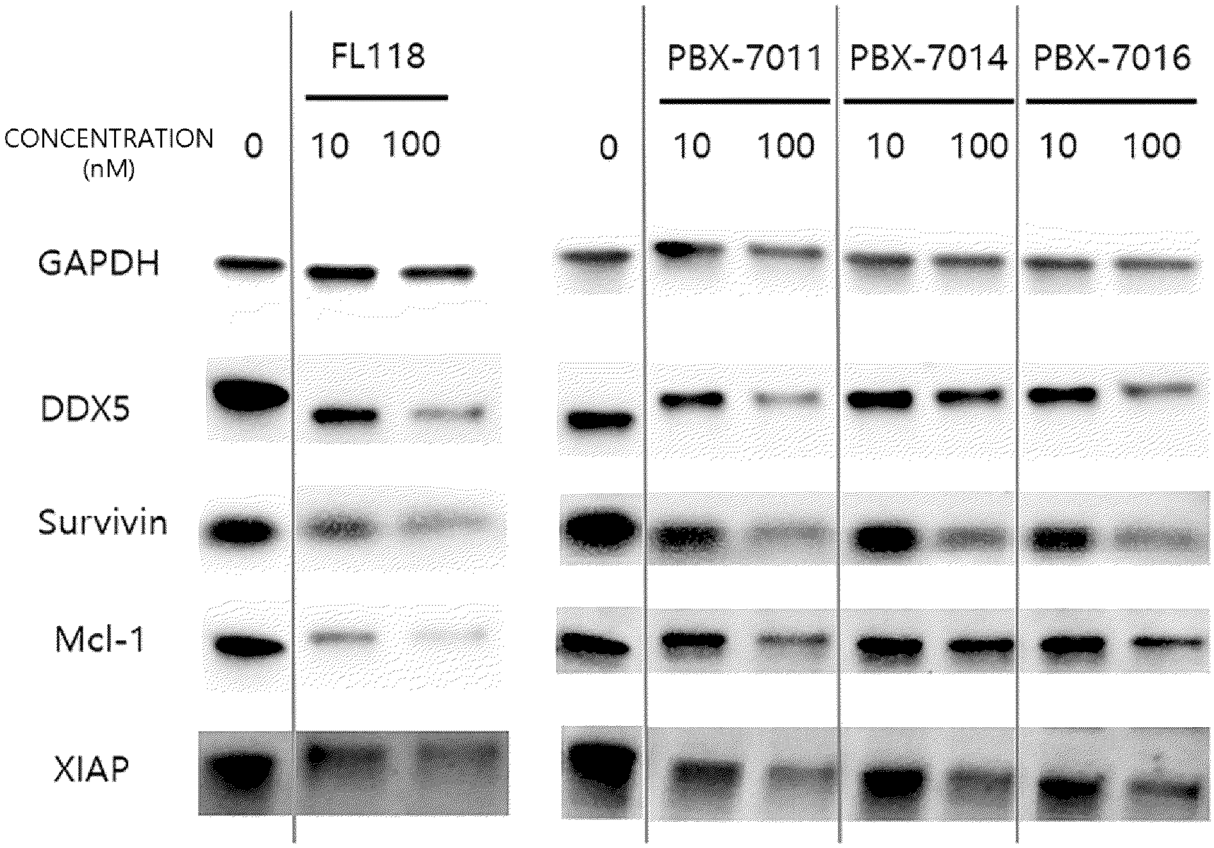
FL118
PBX-7011
PBX-7014
PBX-7016
CONCENTRATION (nM)
0
10
100
0
10
100
10
100
10
100
GAPDH
DDX5
Survivin
Mcl-1
XIAP

[FIG. 12]

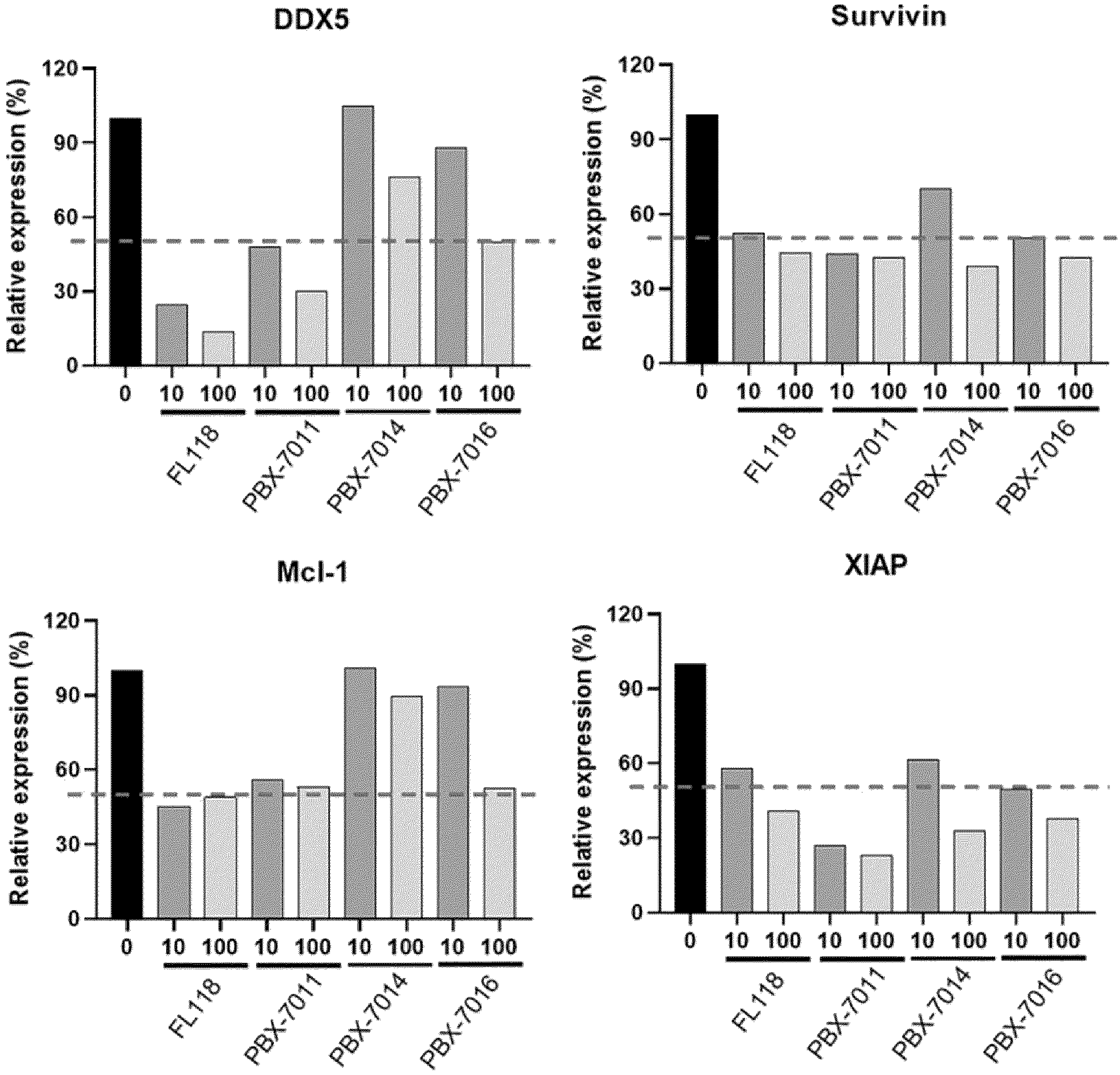
DDX5
Survivin
Mcl-1
XIAP
Relative expression (%)
120
90
60
30
0
0
10 100 10 100 10 100 10 100
FL118
PBX-7011
PBX-7014
PBX-7016

[FIG. 13]

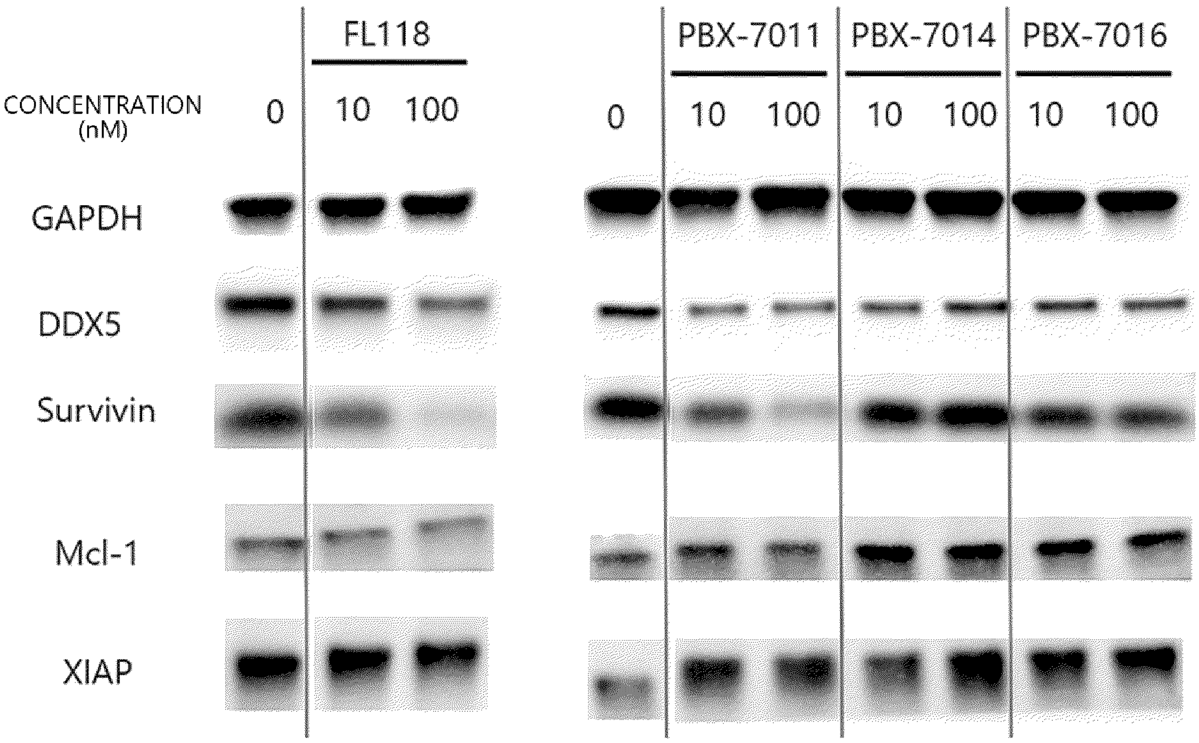
FL118
PBX-7011
PBX-7014
PBX-7016
CONCENTRATION (nM)
0
10
100
0
10
100
10
100
10
100
GAPDH
DDX5
Survivin
Mcl-1
XIAP

[FIG. 14]

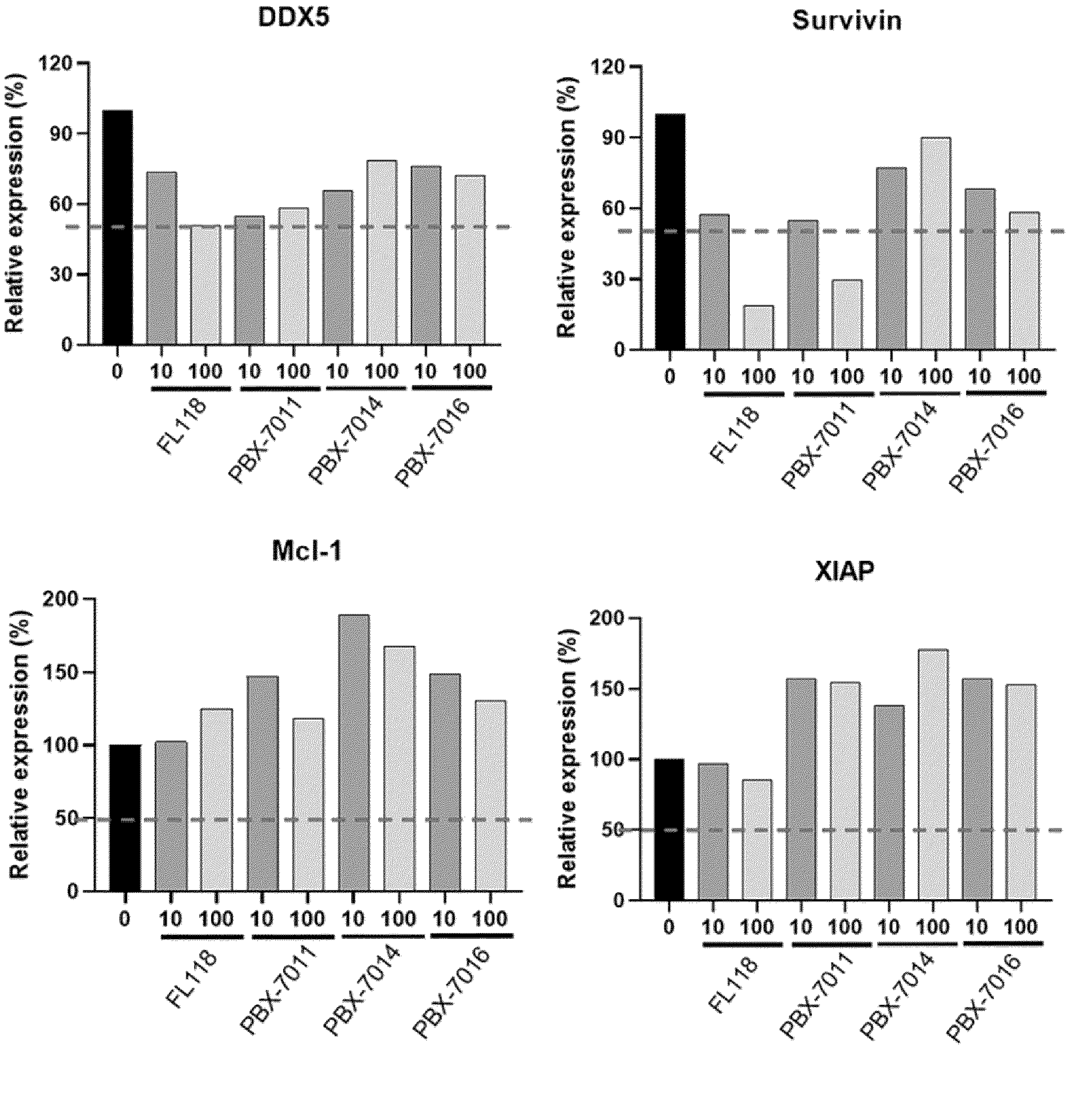
DDX5
Survivin
Mcl-1
XIAP
Relative expression (%)
0
10 100 10 100 10 100 10 100
FL118
PBX-7011
PBX-7014
PBX-7016

[FIG. 15]

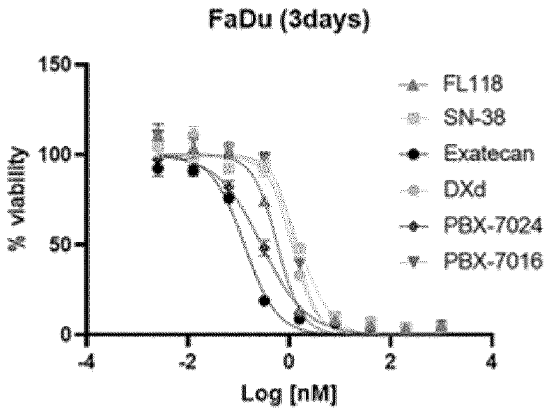


| Drug | $IC_{50}$ (nM) |
|---|---|
| FL118 | 0.5844 |
| SN-38 | 1.555 |
| Exatecan | 0.1298 |
| DXd | 1.130 |
| PBX-7016 | 1.331 |
| PBX-7024 | 0.2841 |

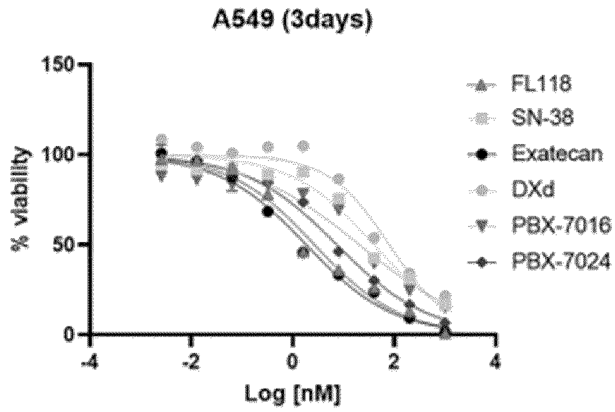


| Drug | $IC_{50}$ (nM) |
|---|---|
| FL118 | 2.670 |
| SN-38 | 39.70 |
| Exatecan | 1.845 |
| DXd | 80.25 |
| PBX-7016 | 20.47 |
| PBX-7024 | 7.387 |

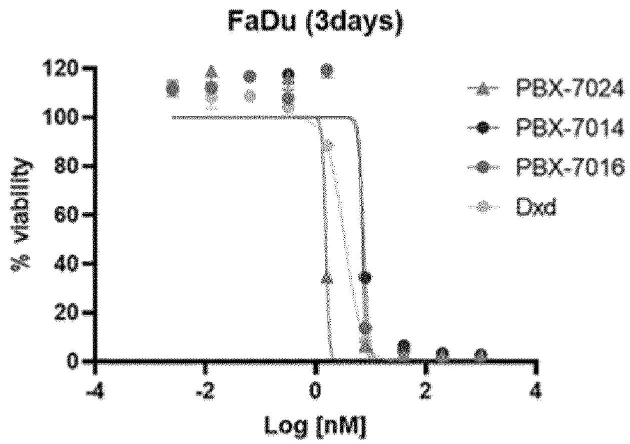


| Drug | $IC_{50}$ (nM) |
|---|---|
| PBX-7024 | 1.528 |
| PBX-7014 | 7.460 |
| PBX-7016 | 7.022 |
| Dxd | 3.369 |

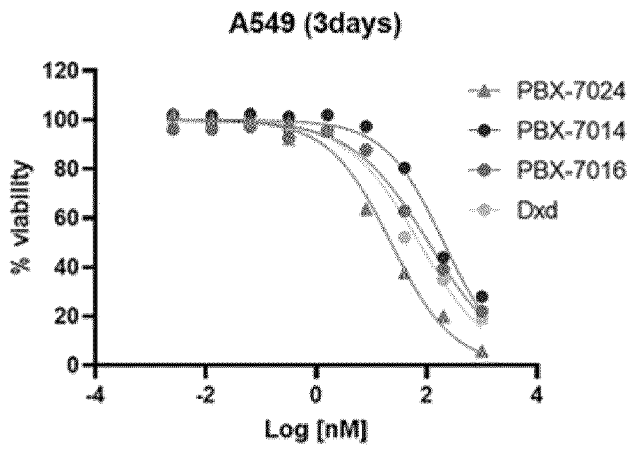


| Drug | $IC_{50}$ (nM) |
|---|---|
| PBX-7024 | 22.51 |
| PBX-7014 | 205.4 |
| PBX-7016 | 109.0 |
| Dxd | 73.24 |

[FIG. 16]

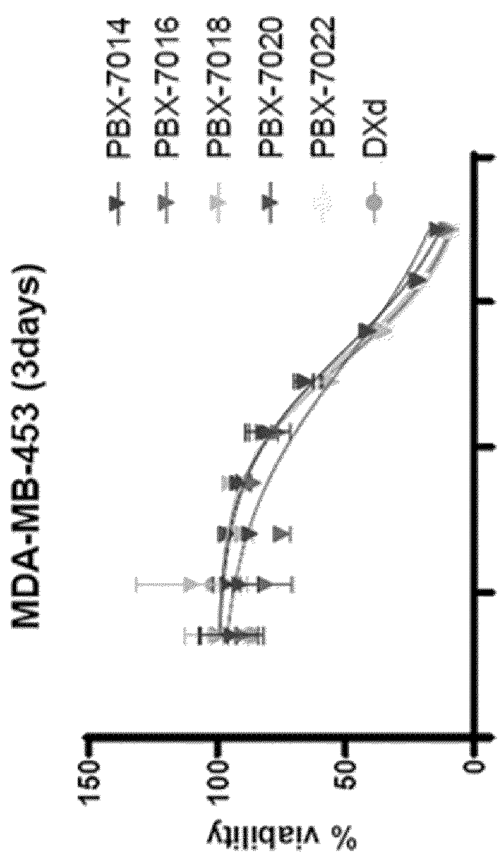


| Drug | $IC_{50}$ (nM) |
|---|---|
| PBX-7014 | 22.35 |
| PBX-7016 | 14.98 |
| PBX-7018 | 14.91 |
| PBX-7020 | 15.78 |
| PBX-7022 | 16.33 |
| DXd | 18.48 |

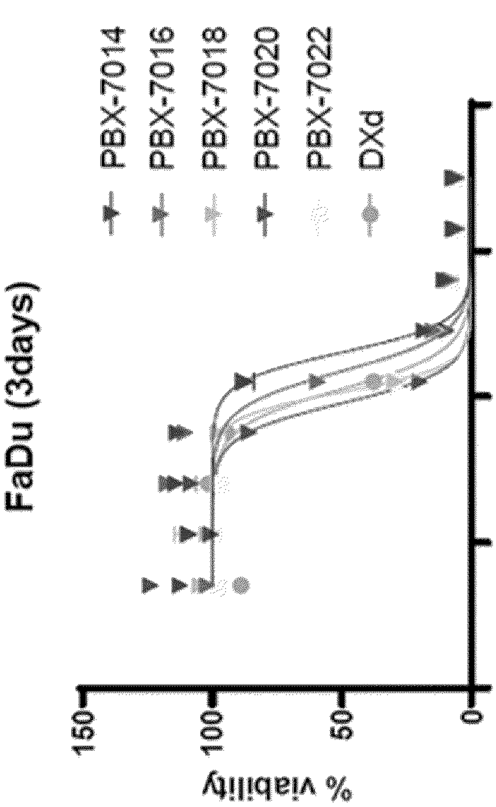


| Drug | $IC_{50}$ (nM) |
|---|---|
| PBX-7014 | 4.037 |
| PBX-7016 | 2.223 |
| PBX-7018 | 1.145 |
| PBX-7020 | 0.8072 |
| PBX-7022 | 1.145 |
| DXd | 1.262 |

[FIG. 17]

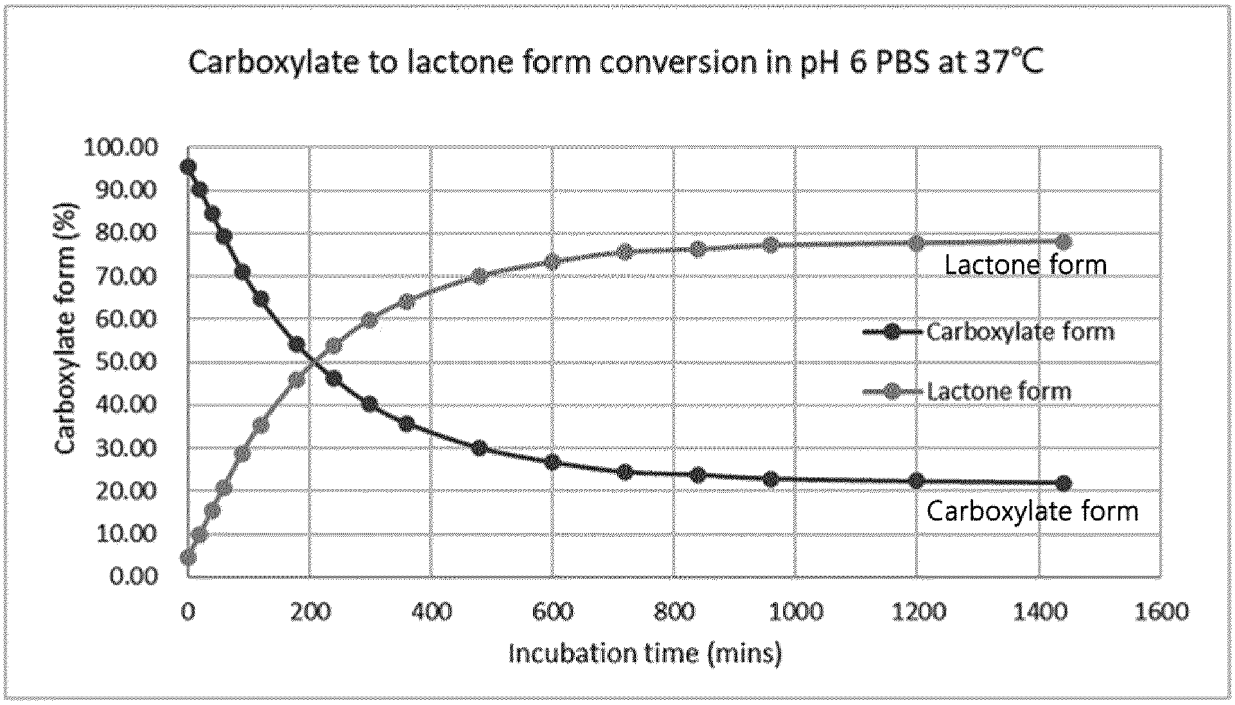
Carboxylate to lactone form conversion in pH 6 PBS at 37°C
Carboxylate form (%)
Incubation time (mins)
Lactone form
Carboxylate form
Carboxylate form
Lactone form
100.00
90.00
80.00
70.00
60.00
50.00
40.00
30.00
20.00
10.00
0.00
0
200
400
600
800
1000
1200
1400
1600

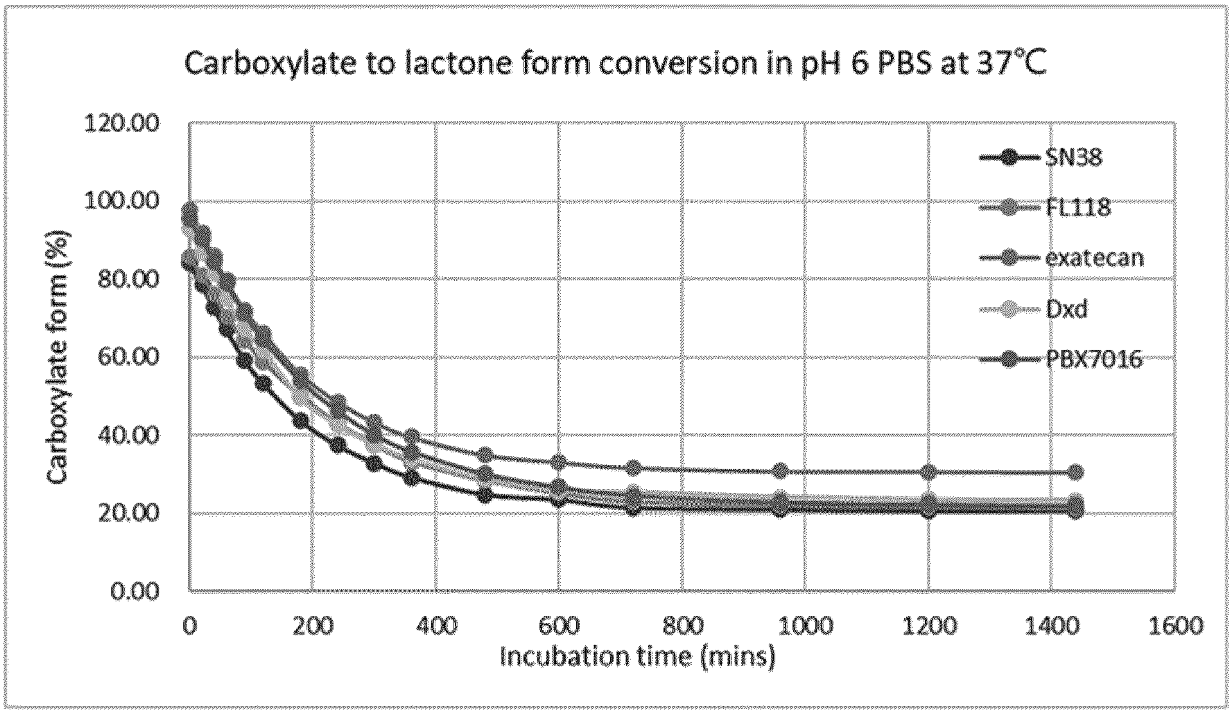
Carboxylate to lactone form conversion in pH 6 PBS at 37°C
Carboxylate form (%)
Incubation time (mins)
SN38
FL118
exatecan
Dxd
PBX7016
120.00
100.00
80.00
60.00
40.00
20.00
0.00
0
200
400
600
800
1000
1200
1400
1600

[FIG. 18]

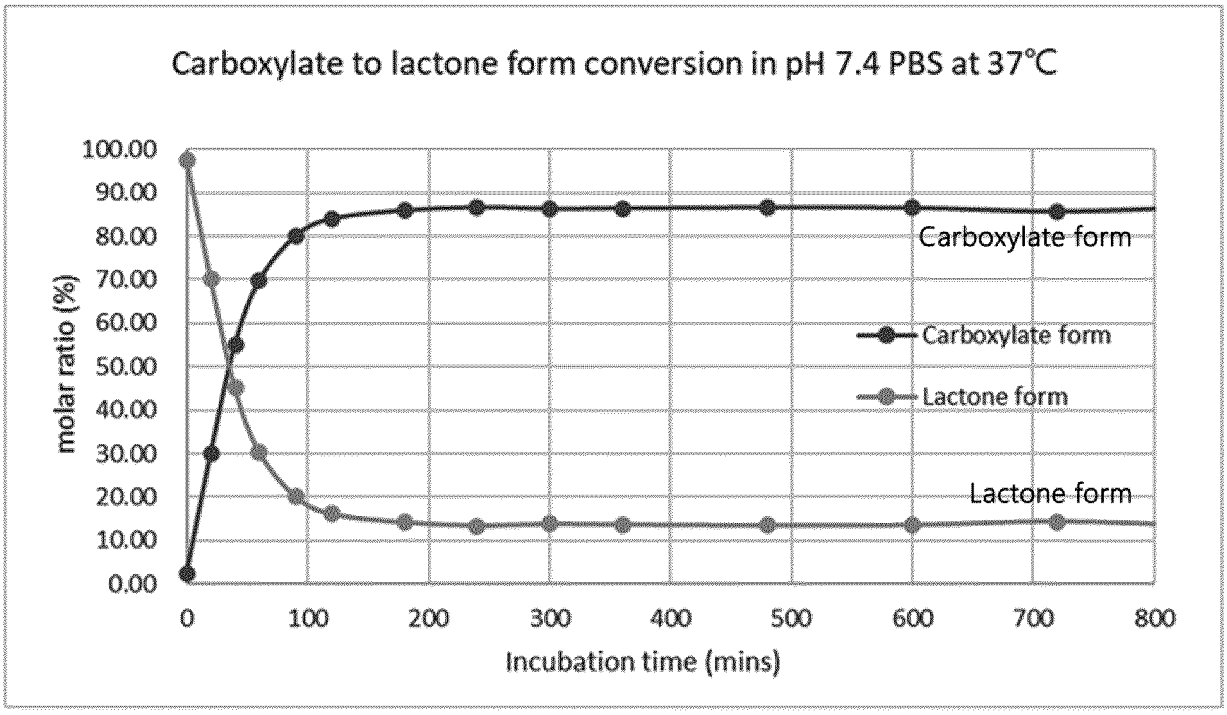
Carboxylate to lactone form conversion in pH 7.4 PBS at 37℃
molar ratio (%)
100.00
90.00
80.00
70.00
60.00
50.00
40.00
30.00
20.00
10.00
0.00
0
100
200
300
400
500
600
700
800
Incubation time (mins)
Carboxylate form
Lactone form

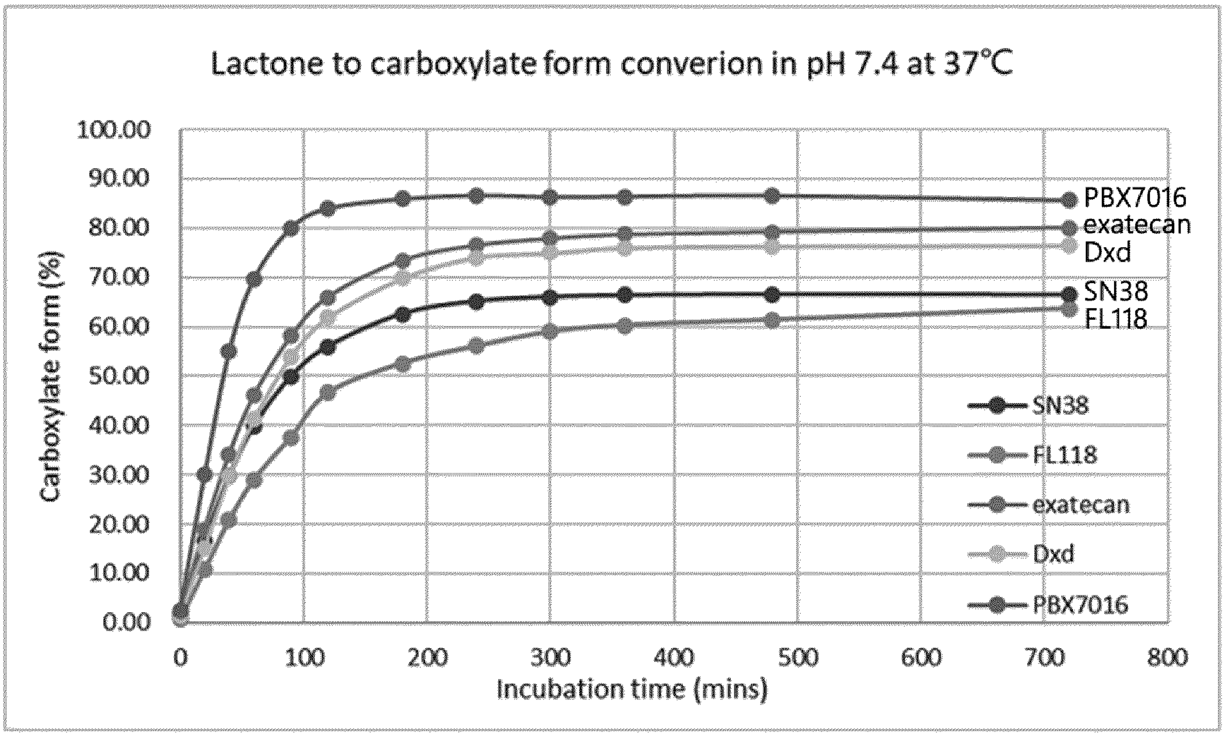
Lactone to carboxylate form converion in pH 7.4 at 37℃
Carboxylate form (%)
100.00
90.00
80.00
70.00
60.00
50.00
40.00
30.00
20.00
10.00
0.00
0
100
200
300
400
500
600
700
800
Incubation time (mins)
PBX7016
exatecan
Dxd
SN38
FL118

[FIG. 19]

## ➢ SEC (Tra-PBX-7014)

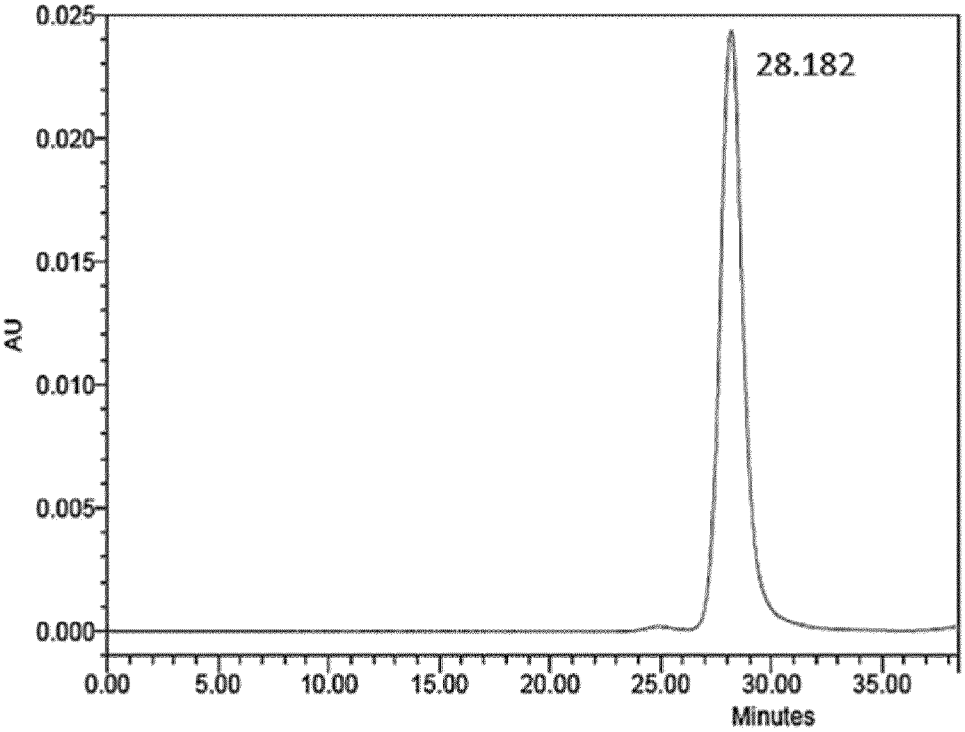


## ➢ SEC (Tra-PBX-7016)

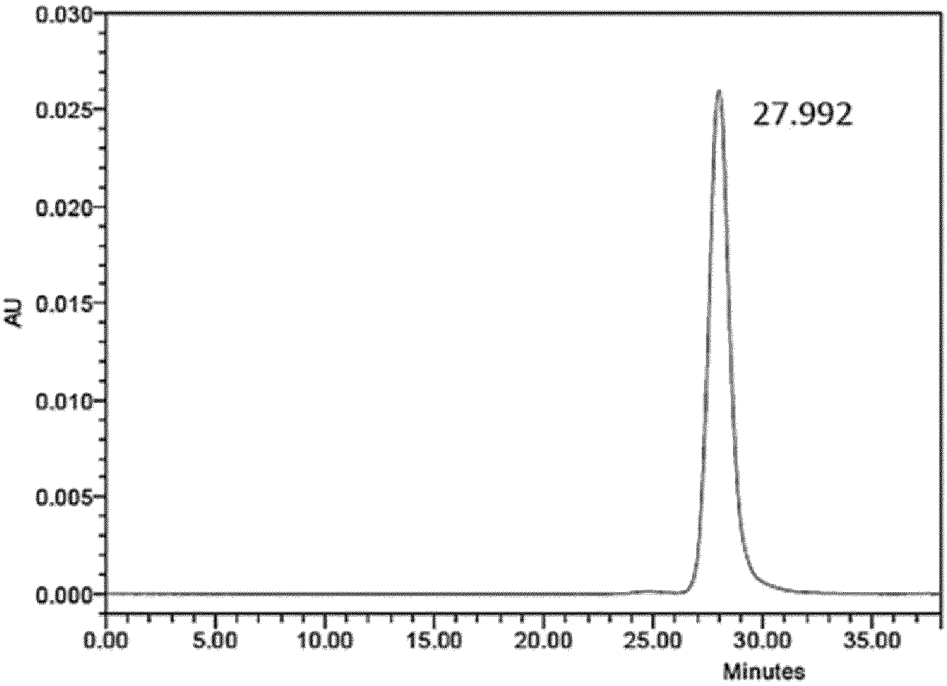

[FIG. 20]

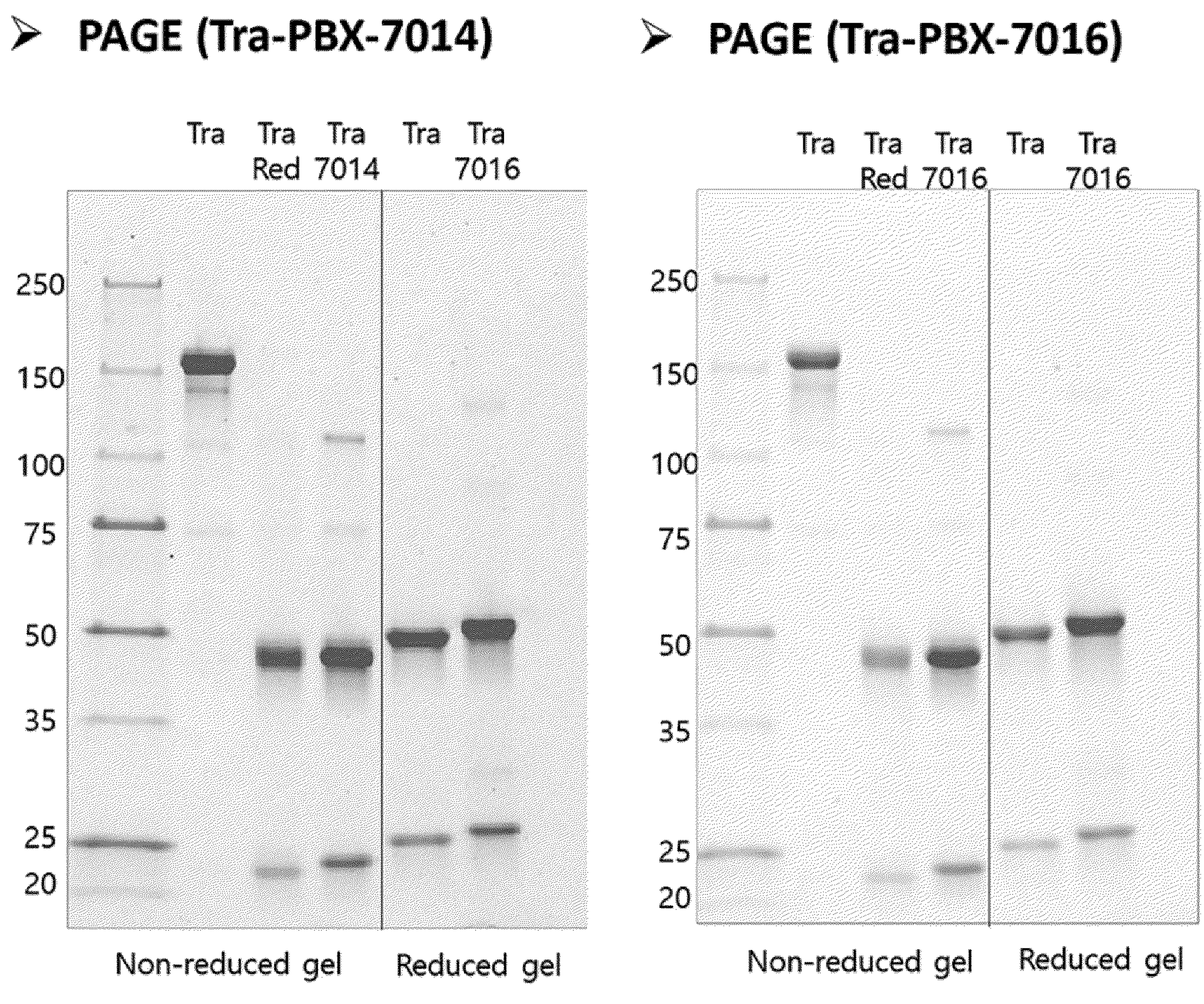
PAGE (Tra-PBX-7014)
Tra
Tra Red
Tra 7014
Tra
Tra 7016
250
150
100
75
50
35
25
20
Non-reduced gel
Reduced gel
PAGE (Tra-PBX-7016)
Tra
Tra Red
Tra 7016
Tra
Tra 7016
250
150
100
75
50
35
25
20
Non-reduced gel
Reduced gel

[FIG. 21]

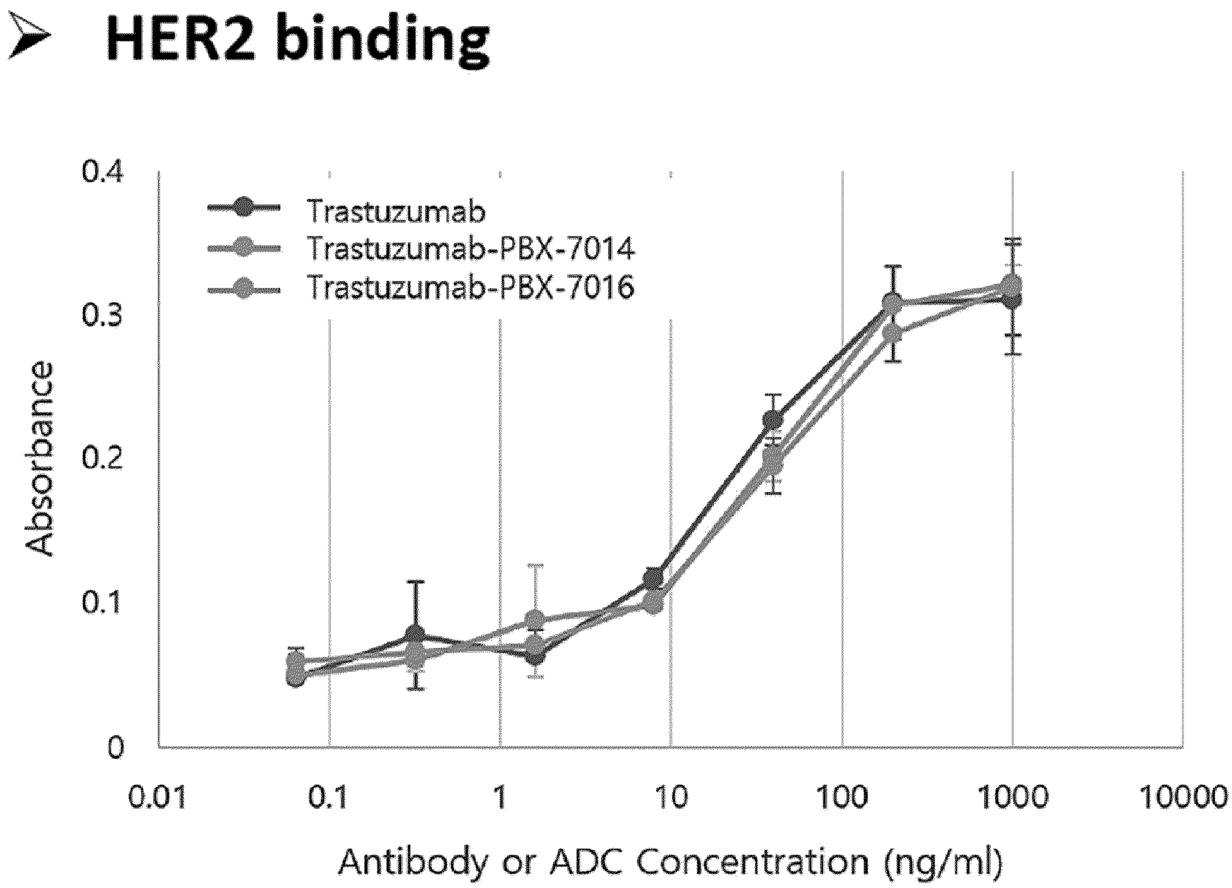
HER2 binding
0.4
0.3
0.2
0.1
0
Absorbance
Trastuzumab
Trastuzumab-PBX-7014
Trastuzumab-PBX-7016
0.01
0.1
1
10
100
1000
10000
Antibody or ADC Concentration (ng/ml)

[FIG. 22]

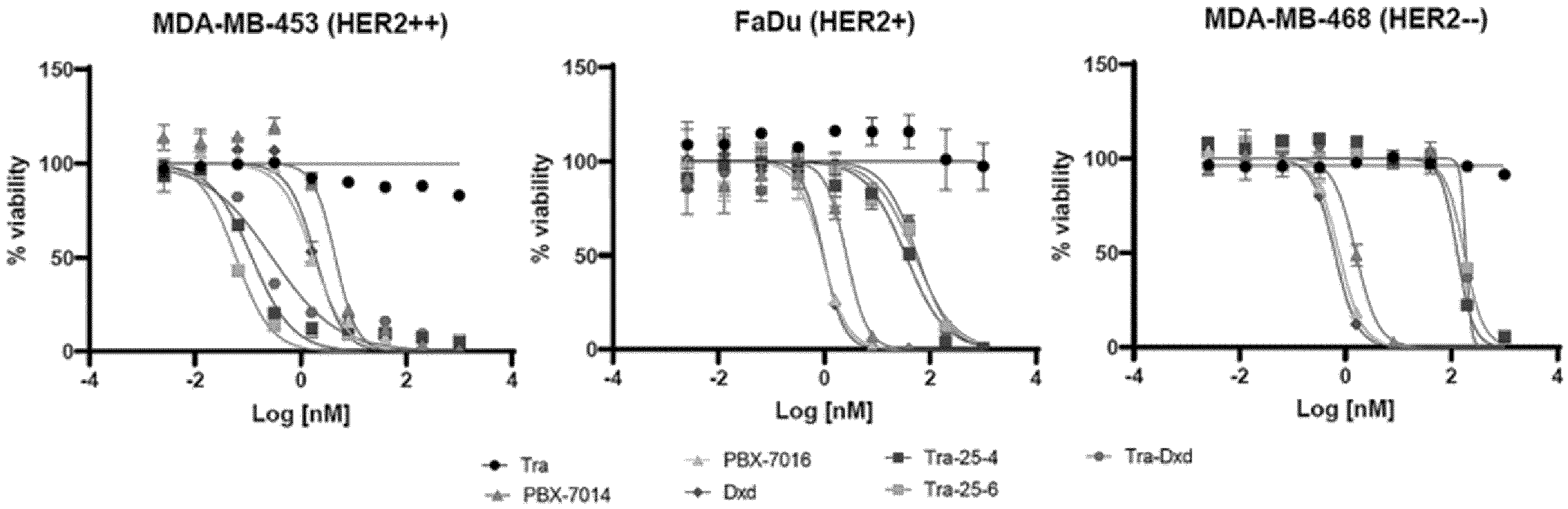


| Drug | | $IC_{50}$ (nM) |
|---|---|---|
| Tra | Ab only | - |
| PBX-7014 | Payload only | 4.539 |
| PBX-7016 | | 1.750 |
| Dxd | | 1.917 |
| Tra-25-4 | ADC | **0.1192** |
| Tra-25-6 | | **0.05973** |
| Tra-Dxd | | **0.2866** |

| Drug | | $IC_{50}$ (nM) |
|---|---|---|
| Tra | Ab only | 1524 |
| PBX-7014 | Payload only | 2.607 |
| PBX-7016 | | 0.9129 |
| Dxd | | 0.9574 |
| Tra-25-4 | ADC | **34.50** |
| Tra-25-6 | | **51.02** |
| Tra-Dxd | | **56.86** |

| Drug | | $IC_{50}$ (nM) |
|---|---|---|
| Tra | Ab only | - |
| PBX-7014 | Payload only | 1.557 |
| PBX-7016 | | 0.7548 |
| Dxd | | 0.6222 |
| Tra-25-4 | ADC | **128.8** |
| Tra-25-6 | | **174.8** |
| Tra-Dxd | | **188.0** |

[FIG. 23]

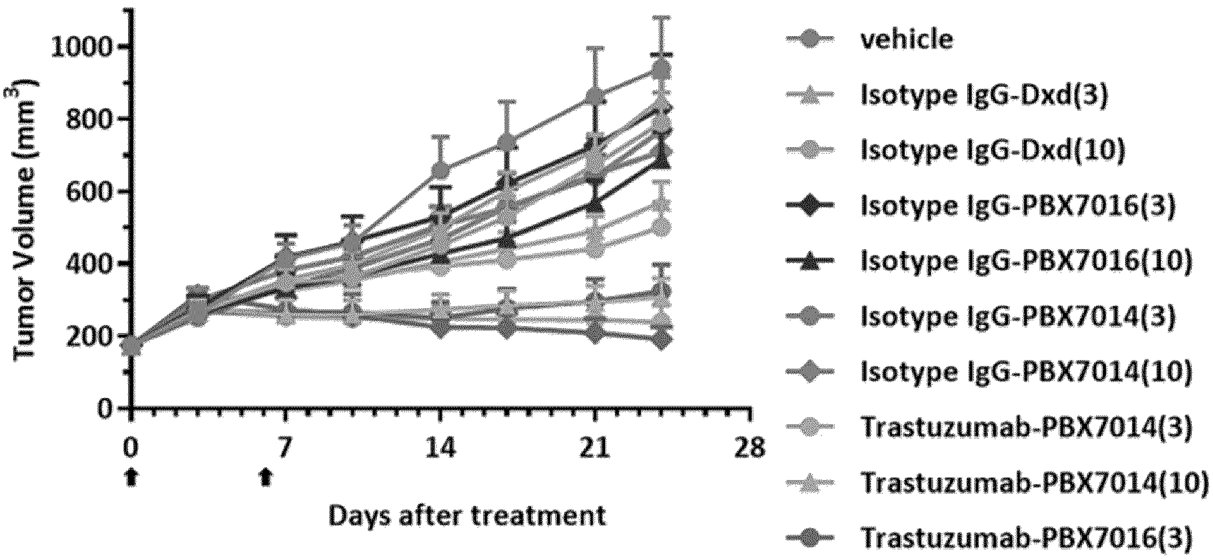


| | Group (mg/kg) | TGI (%) |
|---|---|---|
| 1 | Vehicle | NA |
| 2 | Isotype-Dxd (CTP63-Dxd (3)) | 11.27 |
| 3 | Isotype-Dxd (CTP63-Dxd (10)) | 19.74 |
| 4 | Isotype-PBX7016 (CTP63-PBX7016 (3)) | 14.38 |
| 5 | Isotype-PBX7016 (CTP63-PBX7016 (10)) | 32.95 |
| 6 | Isotype-PBX7014 (CTP63-PBX7014 (3)) | 22.52 |
| 7 | Isotype-PBX7016 (CTP63-PBX7014 (10)) | 30.09 |
| 8 | Trastuzumab-PBX7014 (CTP6-PBX7014 (3)) | 57.42 |
| 9 | Trastuzumab-PBX7014 (CTP6-PBX7014 (10)) | 82.61 |
| 10 | Trastuzumab-PBX7016 (CTP6-PBX7016 (3)) | 80.41 |
| 11 | Trastuzumab-PBX7016 (CTP6-PBX7016 (10)) | 97.80 |
| 12 | Enhertu (DP) (3) | 48.42 |
| 13 | Enhertu (DP) (10) | 91.68 |

[FIG. 24]

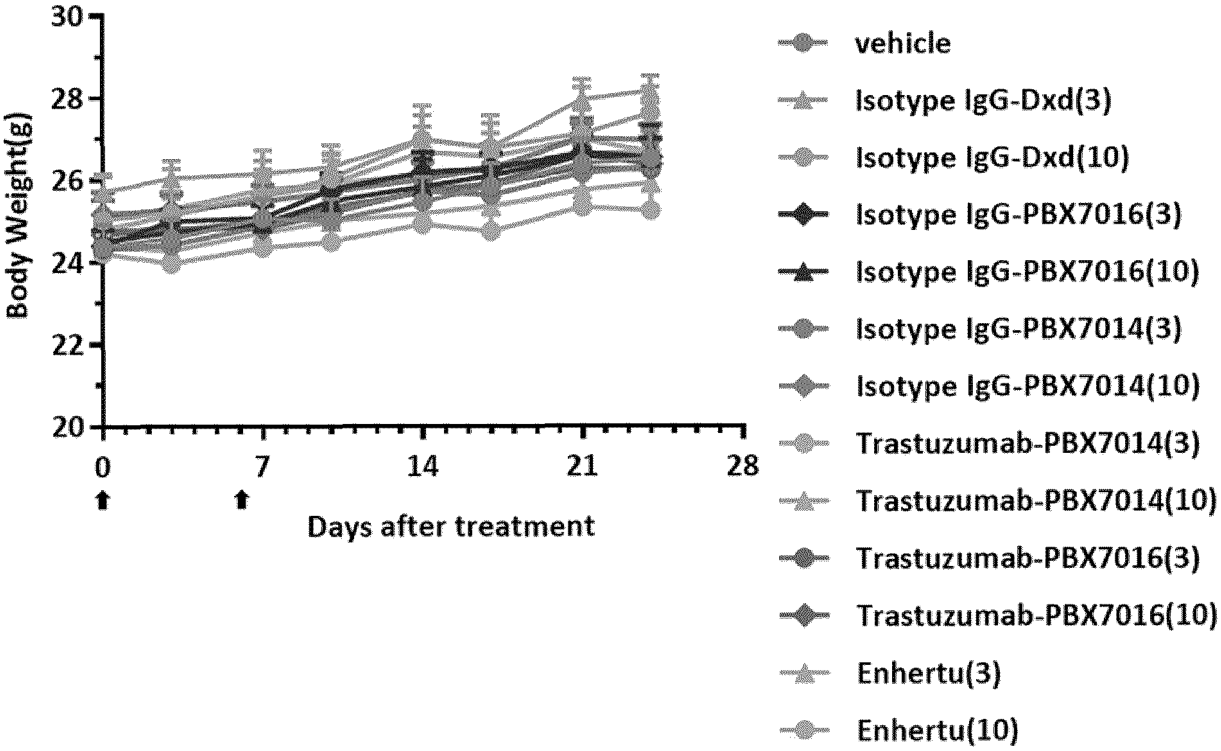
30
28
26
24
22
20
Body Weight(g)
0
7
14
21
28
Days after treatment
vehicle
Isotype IgG-Dxd(3)
Isotype IgG-Dxd(10)
Isotype IgG-PBX7016(3)
Isotype IgG-PBX7016(10)
Isotype IgG-PBX7014(3)
Isotype IgG-PBX7014(10)
Trastuzumab-PBX7014(3)
Trastuzumab-PBX7014(10)
Trastuzumab-PBX7016(3)
Trastuzumab-PBX7016(10)
Enhertu(3)
Enhertu(10)

[FIG. 25]

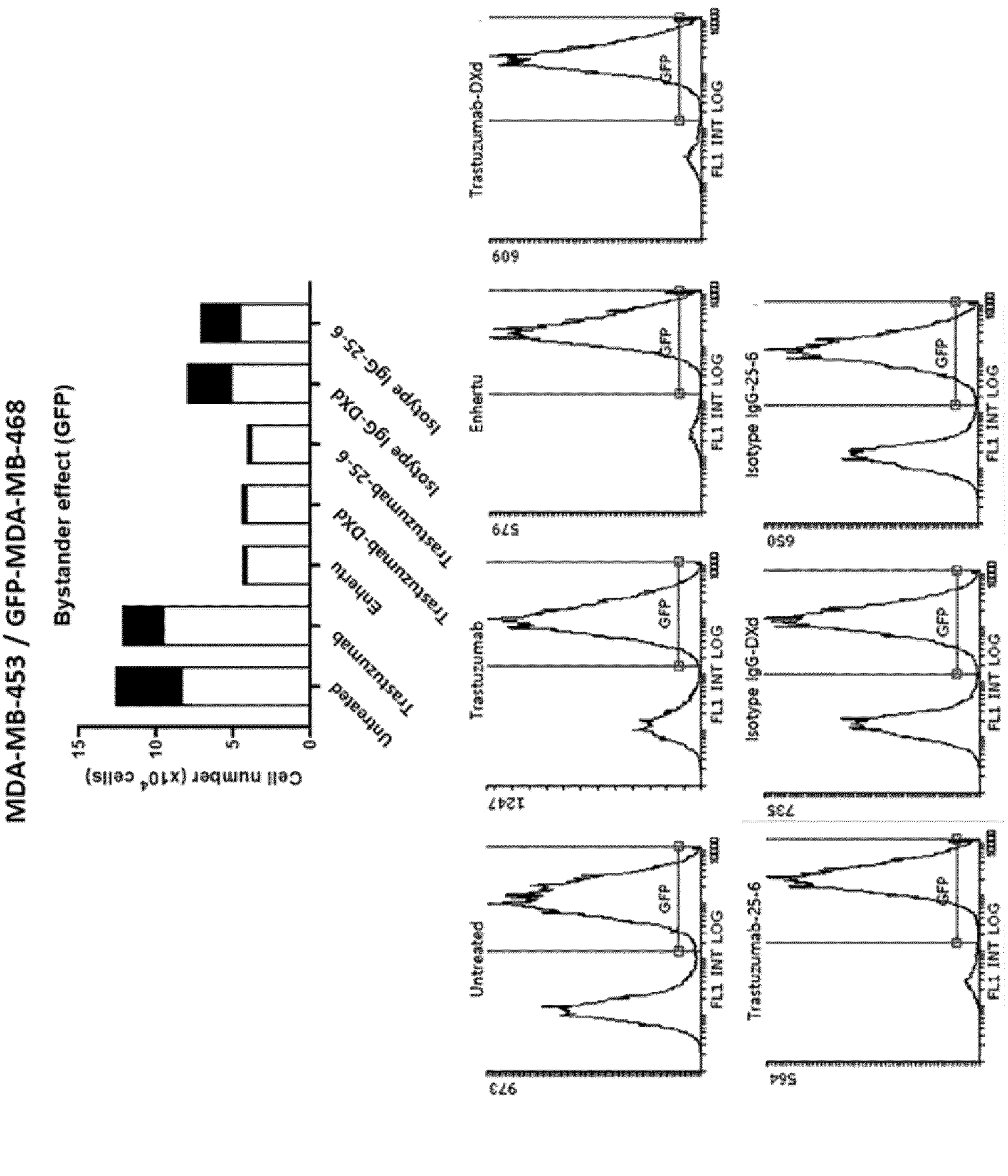
MDA-MB-453 / GFP-MDA-MB-468
Bystander effect (GFP)
Cell number (x10$^4$ cells)
15
10
5
0
Untreated
Trastuzumab
Enhertu
Trastuzumab-DXd
Trastuzumab-25-6
Isotype IgG-DXd
Isotype IgG-25-6
Untreated
Trastuzumab
Enhertu
Trastuzumab-DXd
Trastuzumab-25-6
Isotype IgG-DXd
Isotype IgG-25-6
973
1247
579
609
564
735
650
GFP
FL1 INT LOG

[FIG. 26]

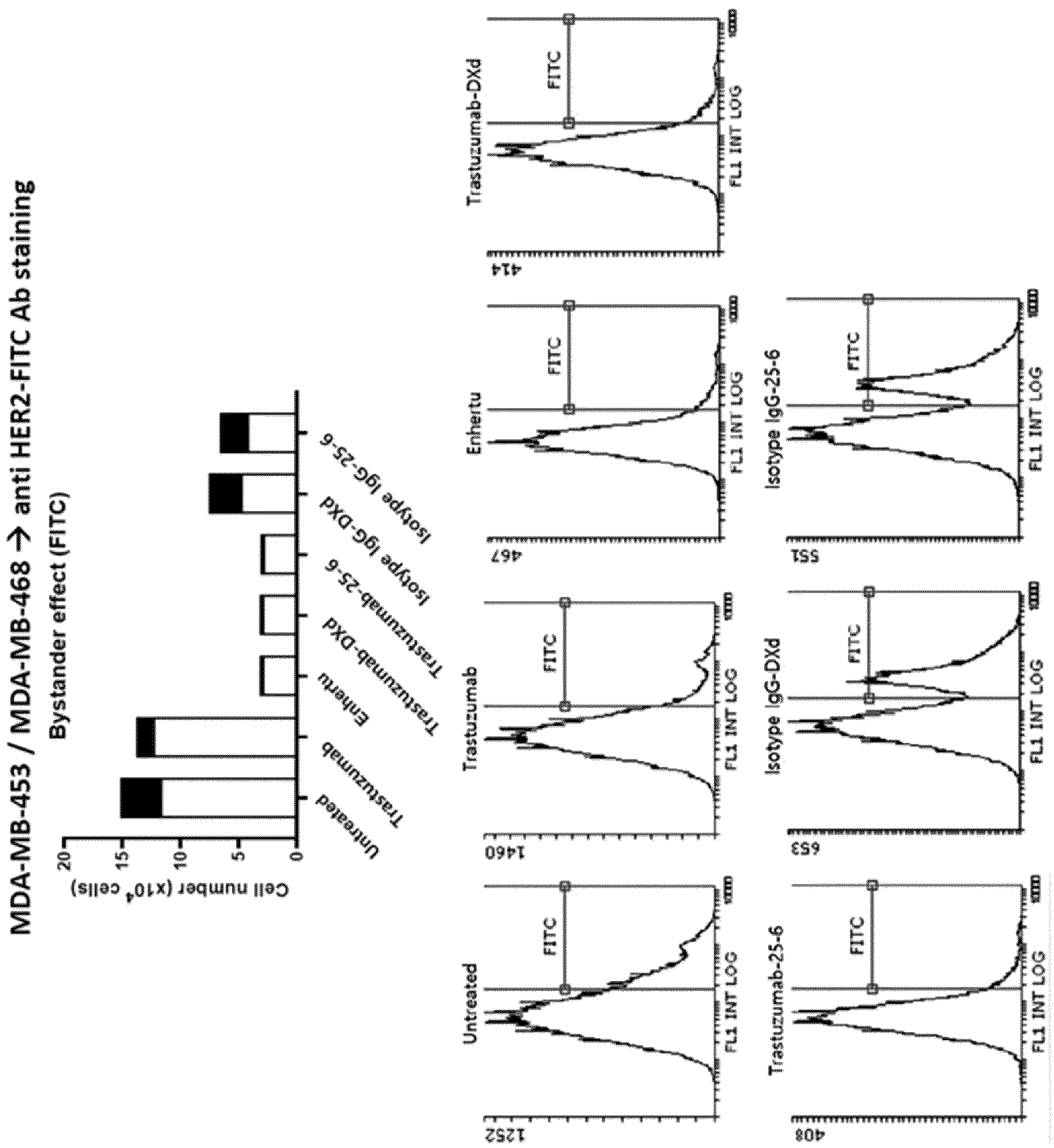
MDA-MB-453 / MDA-MB-468 → anti HER2-FITC Ab staining
Bystander effect (FITC)
Cell number (x10⁴ cells)
0
5
10
15
20
Untreated
Trastuzumab
Enhertu
Trastuzumab-DXd
Trastuzumab-25-6
Isotype IgG-DXd
Isotype IgG-25-6
Untreated
1252
FITC
FL1 INT LOG
Trastuzumab
1460
FITC
FL1 INT LOG
Enhertu
467
FITC
FL1 INT LOG
Trastuzumab-DXd
414
FITC
FL1 INT LOG
Trastuzumab-25-6
408
FITC
FL1 INT LOG
Isotype IgG-DXd
653
FITC
FL1 INT LOG
Isotype IgG-25-6
551
FITC
FL1 INT LOG

[FIG. 27]

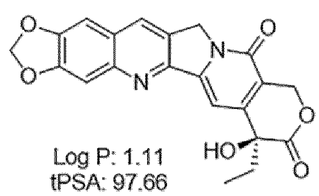

Log P: 1.11
tPSA: 97.66
CLogP: 1.41597

**FL118**

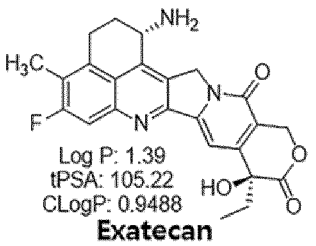

Log P: 1.39
tPSA: 105.22
CLogP: 0.9488

**Exatecan**

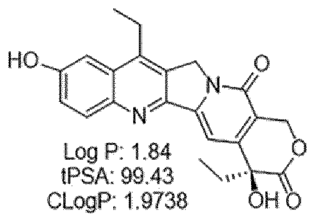

Log P: 1.84
tPSA: 99.43
CLogP: 1.9738

**SN-38**

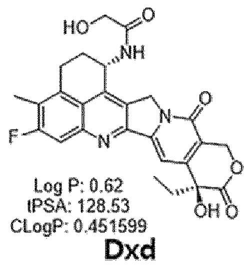

Log P: 0.62
tPSA: 128.53
CLogP: 0.451599

**Dxd**

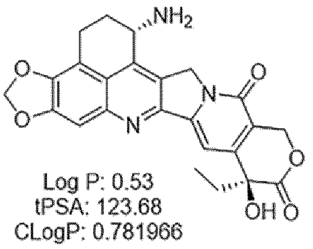

Log P: 0.53
tPSA: 123.68
CLogP: 0.781966

COMPOUND A

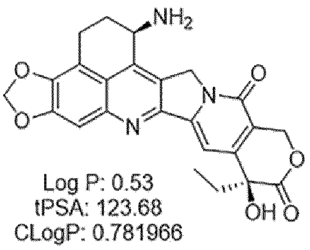

Log P: 0.53
tPSA: 123.68
CLogP: 0.781966

COMPOUND B

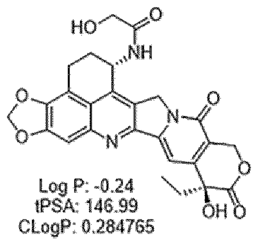

Log P: -0.24
tPSA: 146.99
CLogP: 0.284765

COMPOUND C

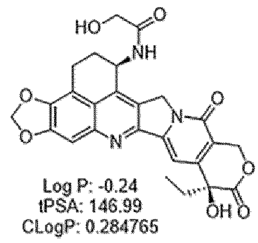

Log P: -0.24
tPSA: 146.99
CLogP: 0.284765

COMPOUND D

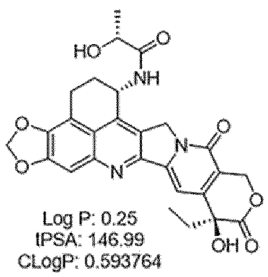

Log P: 0.25
tPSA: 146.99
CLogP: 0.593764

COMPOUND E

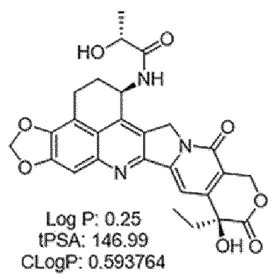

Log P: 0.25
tPSA: 146.99
CLogP: 0.593764

COMPOUND F

[FIG. 28]

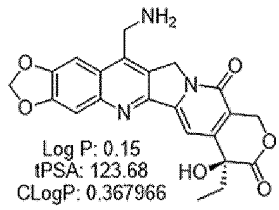


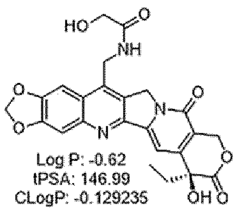


COMPOUND G

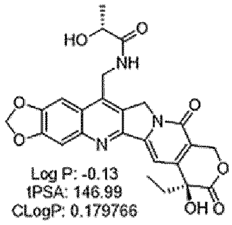


COMPOUND H

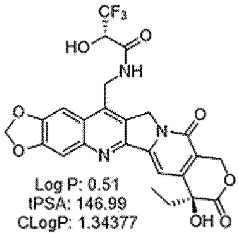


COMPOUND I

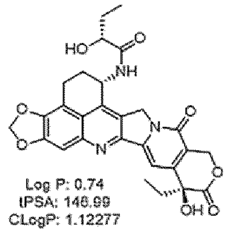


COMPOUND J

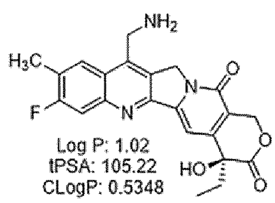


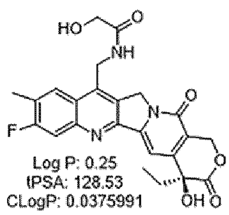


COMPOUND G'

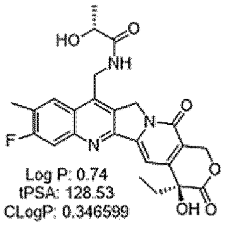


COMPOUND H'

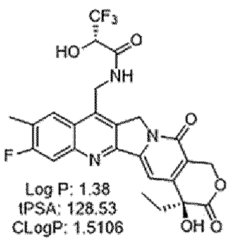


COMPOUND I'

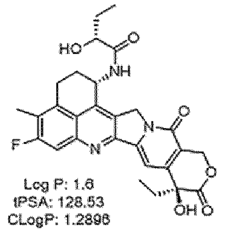


COMPOUND J'

[FIG. 29]

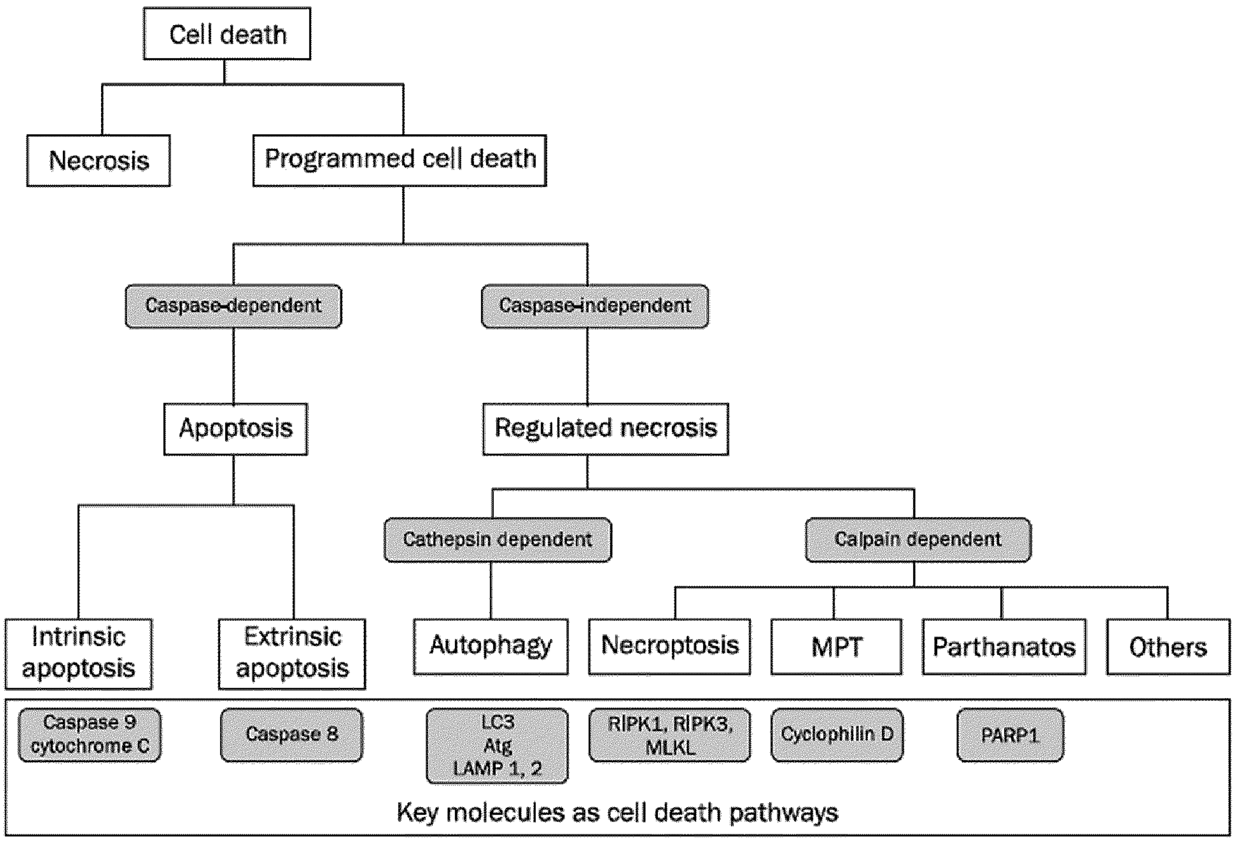
Cell death
Necrosis
Programmed cell death
Caspase-dependent
Caspase-independent
Apoptosis
Regulated necrosis
Cathepsin dependent
Calpain dependent
Intrinsic apoptosis
Extrinsic apoptosis
Autophagy
Necroptosis
MPT
Parthanatos
Others
Caspase 9 cytochrome C
Caspase 8
LC3 Atg LAMP 1, 2
RIPK1, RIPK3, MLKL
Cyclophilin D
PARP1
Key molecules as cell death pathways

**INTERNATIONAL SEARCH REPORT**

International application No. **PCT/KR2023/005380**

**A. CLASSIFICATION OF SUBJECT MATTER**

**C07D 491/22**(2006.01)i; **A61K 31/4745**(2006.01)i; **A61P 35/00**(2006.01)i; **A61K 47/65**(2017.01)i; **A61K 47/68**(2017.01)i; **G01N 33/574**(2006.01)i

According to International Patent Classification (IPC) or to both national classification and IPC

**B. FIELDS SEARCHED**

Minimum documentation searched (classification system followed by classification symbols)

C07D 491/22(2006.01); A61K 31/395(2006.01); A61K 31/535(2006.01); A61K 47/10(2006.01); A61K 47/64(2017.01); A61K 47/68(2017.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal), STN (Registry, Caplus) & keywords: 캄토테신 유도체(camptothecin derivative), 프로드럭(prodrug), DDX5 단백질(DDX5 protein), E3 리가아제(E3 ligase), 항암(anti-cancer)

**C. DOCUMENTS CONSIDERED TO BE RELEVANT**

| Category* | Citation of document, with indication, where appropriate, of the relevant passages | Relevant to claim No. |
|---|---|---|
| A | KR 10-2021-0053871 A (SEAGEN INC.) 12 May 2021 (2021-05-12)<br>See claim 1; and paragraphs [0031], [0032], [0100], [0137], [0257], [0519], [0523], [0538], [0547], [0557], [0559], [0569] and [0775]. | 1-34 |
| A | ZHAO, X. et al. Camptothecin induced DDX5 degradation increased the camptothecin resistance of osteosarcoma. Experimental cell research. 2020, vol. 394, article no. 112148, pp. 1-10.<br>See entire document. | 1-34 |
| A | KR 10-2021-0006362 A (SEATTLE GENETICS, INC.) 18 January 2021 (2021-01-18)<br>See entire document. | 1-34 |
| A | US 5496830 A (SHAPIRO, T. A. et al.) 05 March 1996 (1996-03-05)<br>See entire document. | 1-34 |
| A | LIU, Y. et al. E3 ubiquitin ligase in anticancer drugdsla resistance: recent advances future potential. Frontiers in pharmacology. 2021, vol. 12, article no. 645864, pp. 1-14.<br>See entire document. | 1-34 |

☑ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **20 July 2023** | **20 July 2023** |
| Name and mailing address of the ISA/KR<br>**Korean Intellectual Property Office**<br>**Government Complex-Daejeon Building 4, 189 Cheongsa-ro, Seo-gu, Daejeon 35208**<br>Facsimile No. **+82-42-481-8578** | Authorized officer<br><br>Telephone No. |

Form PCT/ISA/210 (second sheet) (July 2022)

**INTERNATIONAL SEARCH REPORT**

International application No.
**PCT/KR2023/005380**

**C. DOCUMENTS CONSIDERED TO BE RELEVANT**

| Category* | Citation of document, with indication, where appropriate, of the relevant passages | Relevant to claim No. |
|---|---|---|
| PX | WO 2022-170971 A1 (MEDILINK THERAPEUTICS (SUZHOU) CO., LTD. ) 18 August 2022. See pages 4, 24, 50, 62, 124, 171-174, 181 and 379. | 1-34 |

Form PCT/ISA/210 (second sheet) (July 2022)

**INTERNATIONAL SEARCH REPORT**
**Information on patent family members**

International application No.
**PCT/KR2023/005380**

| Patent document cited in search report | Publication date (day/month/year) | Patent family member(s) | Publication date (day/month/year) |
|---|---|---|---|
| KR 10-2021-0053871 A | 12 May 2021 | AR 114919 A1 | 28 October 2020 |
| | | AU 2019-282767 A1 | 07 January 2021 |
| | | BR 112020024915 A2 | 09 March 2021 |
| | | CA 3101866 A1 | 12 December 2019 |
| | | CN 112512592 A | 16 March 2021 |
| | | EP 3801633 A1 | 14 April 2021 |
| | | JP 2021-527040 A | 11 October 2021 |
| | | MX 2020013169 A | 29 March 2021 |
| | | SG 11202011915 A | 30 December 2020 |
| | | TW 202015740 A | 01 May 2020 |
| | | US 2023-0036256 A1 | 02 February 2023 |
| | | WO 2019-236954 A1 | 12 December 2019 |
| KR 10-2021-0006362 A | 18 January 2021 | AR 114473 A1 | 09 September 2020 |
| | | AU 2019-247434 A1 | 08 October 2020 |
| | | BR 112020020466 A2 | 12 January 2021 |
| | | CA 3094313 A1 | 10 October 2019 |
| | | CN 111936169 A | 13 November 2020 |
| | | EA 202092410 A1 | 09 February 2021 |
| | | EP 3773736 A1 | 17 February 2021 |
| | | JP 2021-521111 A | 26 August 2021 |
| | | MA 52669 A | 17 February 2021 |
| | | MX 2020010458 A | 29 January 2021 |
| | | SG 11202009527 A | 29 October 2020 |
| | | TW 202010498 A | 16 March 2020 |
| | | US 2019-0343828 A1 | 14 November 2019 |
| | | US 2022-0193069 A1 | 23 June 2022 |
| | | WO 2019-195665 A1 | 10 October 2019 |
| US 5496830 A | 05 March 1996 | WO 96-08249 A1 | 21 March 1996 |
| WO 2022-170971 A1 | 18 August 2022 | CN 115279417 A | 01 November 2022 |
| | | TW 202241521 A | 01 November 2022 |

Form PCT/ISA/210 (patent family annex) (July 2022)